# EUROPEAN PATENT SPECIFICATION

(11) **EP 1 582 530 B1**
(45) Date of publication and mention of the grant of the patent: **27.12.2006**
(21) Application number: 04008050.9
(22) Date of filing: 02.04.2004
(51) Int. Cl.: C07K 14/705

(54) **Diagnositc use of polymorphisms in the gene for BTNL2 associated with sarcoidosis**
Diagnositische Verwendung von Polymorphismen im BTNL2 assoziert mit Sarcoidosis
Utilisation diagnostic de polymorphisme BTNL2 associée à la sarcoidosis

(43) Date of publication of application: 05.10.2005
(73) Proprietor: Universitätsklinikum Schleswig-Holstein, 24105 Kiel (DE)
(72) Inventor: Hampe, Jochen, 24116 Kiel (DE); Rosenstiel, Philip, 24116 Kiel (DE); Schreiber, Stefan, 24105 Kiel (DE); Schümann, Manfred, 23562 Lübeck (DE); Valentolyte, Ruta, 24116 Kiel (DE)
(74) Representative: Biehl, Christian

(56) References cited:
- STAMMERS MELANIE ET AL: "BTL-II: A polymorphic locus with homology to the butyrophilin gene family, located at the border of the major histocompatibility complex class II and class III regions in human and mouse" IMMUNOGENETICS, vol. 51, no. 4-5, April 2000 (2000-04), pages 373-382, XP002291986 ISSN: 0093-7711 & DATABASE EMBL BTNL2; butyrophylin 18 November 2001 (2001-11-18), WILLIAMS: "Human DNA sequence from clone RP1-172K2" retrieved from EBI Database accession no. Z84814
- DATABASE EMBL Genomic DNA 8 May 2001 (2001-05-08), MUNGALL: "Genomic DNA from H. sapiens chromosome 6" XP002291989 retrieved from EBI Database accession no. AL591281
- ABDALLAH A ET AL: "Inhibitor kappa B-alpha (IkappaB-alpha) promoter polymorphisms in UK and Dutch sarcoidosis." GENES AND IMMUNITY, vol. 4, no. 6, September 2003 (2003-09), pages 450-454, XP009035129 ISSN: 1466-4879
- SATO HIROE ET AL: "HLA-DQB1*0201: A marker for good prognosis in British and Dutch patients with sarcoidosis" AMERICAN JOURNAL OF RESPIRATORY CELL AND MOLECULAR BIOLOGY, vol. 27, no. 4, October 2002 (2002-10), pages 406-412, XP002291987 ISSN: 1044-1549
- SCHUERMANN M ET AL: "CARD15 gene mutations in sarcoidosis." EUROPEAN RESPIRATORY JOURNAL, vol. 22, no. 5, November 2003 (2003-11), pages 748-754, XP009035144 ISSN: 0903-1936

### SEQUENCE LISTING

<110> Universitätsklinikum Schleswig-Holstein
<120> DIAGNOSTIC USE OF POLYMORPHISMS IN THE GENE CODING FOR BTNL2 ASSOCIATED WITH SARCOIDOSIS
<130> pva4009
<160> 458
<170> PatentIn version 3.1
<210> 1
   <211> 22
   <212> DNA
   <213> Artificial Sequence
<220>
   <223> Forward Primer
<400> 1
   tccttaacca acctcatcct gg 22
<210> 2
   <211> 24
   <212> DNA
   <213> Artificial Sequence
<220>
   <223> Reverse Primer
<400> 2
   tggtaactcg acagctgatt cttg 24
<210> 3
   <211> 19
   <212> DNA
   <213> Artificial Sequence
<220>
   <223> Forward Primer
<400> 3
   cggacgtgtg gttcacagc 19
<210> 4
   <211> 22
   <212> DNA
   <213> Artificial Sequence
<220>
   <223> Reverse Primer
<400> 4
   ctccgtgtac aatgtgtccg tg 22
<210> 5
   <211> 17
   <212> DNA
   <213> Artificial Sequence
<220>
   <223> Forward Primer
<400> 5
   gggctgtcca cgtgcac 17
<210> 6
   <211> 28
   <212> DNA
   <213> Artificial Sequence
<220>
   <223> Reverse Primer
<400> 6
   gcagagttga accagtatat aacctttc 28
<210> 7
   <211> 20
   <212> DNA
   <213> Artificial Sequence
<220>
   <223> Forward Primer
<400> 7
   ccacaagaaa caagggcagc 20
<210> 8
   <211> 23
   <212> DNA
   <213> Artificial Sequence
<220>
   <223> Reverse Primer
<400> 8
   gaacatgtcc ggtctaagac caa 23
<210> 9
   <211> 23
   <212> DNA
   <213> Artificial Sequence
<220>
   <223> Forward Primer
<400> 9
   tggagagaac ctggaattaa ggc 23
<210> 10
   <211> 33
   <212> DNA
   <213> Artificial Sequence
<220>
   <223> Reverse Primer
<400> 10
   ggactatatc taagtcttac ttcaatagct gga 33
<210> 11
   <211> 17
   <212> DNA
   <213> Artificial Sequence
<220>
   <223> Forward Primer
<400> 11
   ccatctgggc cttggca 17
<210> 12
   <211> 27
   <212> DNA
   <213> Artificial Sequence
<220>
   <223> Reverse Primer
<400> 12
   ggtaatggtc tggttgttgt agttgaa 27
<210> 13
   <211> 18
   <212> DNA
   <213> Artificial Sequence
<220>
   <223> Forward Primer
<400> 13
   gttctcctcg cggtgctg 18
<210> 14
   <211> 19
   <212> DNA
   <213> Artificial Sequence
<220>
   <223> Reverse Primer
<400> 14
   cccaccctct gccctgtac 19
<210> 15
   <211> 18
   <212> DNA
   <213> Artificial Sequence
<220>
   <223> Forward Primer
<400> 15
   cgcaggtcct cgttcagg 18
<210> 16
   <211> 19
   <212> DNA
   <213> Artificial Sequence
<220>
   <223> Reverse Primer
<400> 16
   agtggatgat tggctgcga 19
<210> 17
   <211> 21
   <212> DNA
   <213> Artificial Sequence
<220>
   <223> Forward Primer
<400> 17
   aggagacctc agagctccca g 21
<210> 18
   <211> 22
   <212> DNA
   <213> Artificial Sequence
<220>
   <223> Reverse Primer
<400> 18
   aagtgaccaa tctcaagcca gc 22
<210> 19
   <211> 20
   <212> DNA
   <213> Artificial Sequence
<220>
   <223> Forward Primer
<400> 19
   ctcacaccct gcagtggatg 20
<210> 20
   <211> 21
   <212> DNA
   <213> Artificial Sequence
<220>
   <223> Reverse Primer
<400> 20
   agggtgagat aatccttgcc g 21
<210> 21
   <211> 23
   <212> DNA
   <213> Artificial Sequence
<220>
   <223> Forward Primer
<400> 21
   gagtgcagcg tctattctca tcc 23
<210> 22
   <211> 20
   <212> DNA
   <213> Artificial Sequence
<220>
   <223> Reverse Primer
<400> 22
   ttggctcctg gagattgagg 20
<210> 23
   <211> 16
   <212> DNA
   <213> Artificial Sequence
<220>
   <223> Forward Primer
<400> 23
   cgtctcgggc accctg 16
<210> 24
   <211> 27
   <212> DNA
   <213> Artificial Sequence
<220>
   <223> Reverse Primer
<400> 24
   agacacacag actagaggta ggtgtca 27
<210> 25
   <211> 19
   <212> DNA
   <213> Artificial Sequence
<220>
   <223> Forward Primer
<400> 25
   ggtggaccaa agagcctgc 19
<210> 26
   <211> 19
   <212> DNA
   <213> Artificial Sequence
<220>
   <223> Reverse Primer
<400> 26
   catccagttc cgccaacac 19
<210> 27
   <211> 19
   <212> DNA
   <213> Artificial Sequence
<220>
   <223> Forward Primer
<400> 27
   ggagtattgg gaccgggag 19
<210> 28
   <211> 20
   <212> DNA
   <213> Artificial Sequence
<220>
   <223> Reverse Primer
<400> 28
   cgctctggtt gtagtagccg 20
<210> 29
   <211> 19
   <212> DNA
   <213> Artificial Sequence
<220>
   <223> Forward Primer
<400> 29
   ctggcctggc tgtcctagc 19
<210> 30
   <211> 21
   <212> DNA
   <213> Artificial Sequence
<220>
   <223> Reverse Primer
<400> 30
   cttcccagta atgaggcagg g 21
<210> 31
   <211> 19
   <212> DNA
   <213> Artificial Sequence
<220>
   <223> Forward Primer
<400> 31 19
   cccagagccc cacagtctt 19
<210> 32
   <211> 22
   <212> DNA
   <213> Artificial Sequence
<220>
   <223> Reverse Primer
<400> 32
   agaaaccctg actgcacaga tc 22
<210> 33
   <211> 19
   <212> DNA
   <213> Artificial Sequence
<220>
   <223> Forward Primer
<400> 33 19
   ctttgagcca cgacaccca 19
<210> 34
   <211> 18
   <212> DNA
   <213> Artificial Sequence
<220>
   <223> Reverse Primer
<400> 34
   ggctcaccag agggcaca 18
<210> 35
   <211> 18
   <212> DNA
   <213> Artificial Sequence
<220>
   <223> Forward Primer
<400> 35
   gcagaaacgc agggcaaa 18
<210> 36
   <211> 23
   <212> DNA
   <213> Artificial Sequence
<220>
   <223> Reverse Primer
<400> 36
   tgtcaagtcc tcggtctctg tgt 23
<210> 37
   <211> 20
   <212> DNA
   <213> Artificial Sequence
<220>
   <223> Forward Primer
<400> 37
   cttgggtgga aaggtgatgg 20
<210> 38
   <211> 22
   <212> DNA
   <213> Artificial Sequence
<220>
   <223> Reverse Primer
<400> 38
   ggttttggga gaggaagagc tc 22
<210> 39
   <211> 21
   <212> DNA
   <213> Artificial Sequence
<220>
   <223> Forward Primer
<400> 39
   ctggctctgc cctttcttct c 21
<210> 40
   <211> 19
   <212> DNA
   <213> Artificial Sequence
<220>
   <223> Reverse Primer
<400> 40
   cggtgatgtt gccctctga 19
<210> 41
   <211> 19
   <212> DNA
   <213> Artificial Sequence
<220>
   <223> Forward Primer
<400> 41
   cgggaacatg cccagaagt 19
<210> 42
   <211> 17
   <212> DNA
   <213> Artificial Sequence
<220>
   <223> Reverse Primer
<400> 42
   ggctggagcc ctcagca 17
<210> 43
   <211> 20
   <212> DNA
   <213> Artificial Sequence
<220>
   <223> Forward Primer
<400> 43
   cccgtcagca ccccaagatg 20
<210> 44
   <211> 24
   <212> DNA
   <213> Artificial Sequence
<220>
   <223> Reverse Primer
<400> 44
   tgggaggtca ggtggatgtt tacc 24
<210> 45
   <211> 21
   <212> DNA
   <213> Artificial Sequence
<220>
   <223> Forward Primer
<400> 45
   gatgggactc accaggtgag g 21
<210> 46
   <211> 21
   <212> DNA
   <213> Artificial Sequence
<220>
   <223> Reverse Primer
<400> 46
   ccagaggtct cctgtaaccc a 21
<210> 47
   <211> 20
   <212> DNA
   <213> Artificial Sequence
<220>
   <223> Forward Primer
<400> 47
   tccagggcta tgaaagtcga 20
<210> 48
   <211> 20
   <212> DNA
   <213> Artificial Sequence
<220>
   <223> Reverse Primer
<400> 48
   tgctggtttc agtcttggct 20
<210> 49
   <211> 26
   <212> DNA
   <213> Artificial Sequence
<220>
   <223> Forward Primer
<400> 49
   cctgcatcct gtctggaagt tagaag 26
<210> 50
   <211> 24
   <212> DNA
   <213> Artificial Sequence
<220>
   <223> Reverse Primer
<400> 50
   tgggccactg actgatttgt gtgt 24
<210> 51
   <211> 18
   <212> DNA
   <213> Artificial Sequence
<220>
   <223> Forward Primer
<400> 51
   cagtggccca gaagaccc 18
<210> 52
   <211> 21
   <212> DNA
   <213> Artificial Sequence
<220>
   <223> Reverse Primer
<400> 52
   agcatcaagg atacccctca c 21
<210> 53
   <211> 19
   <212> DNA
   <213> Artificial Sequence
<220>
   <223> Forward Primer
<400> 53
   ctggagccgg gagtagtgc 19
<210> 54
   <211> 23
   <212> DNA
   <213> Artificial Sequence
<220>
   <223> Reverse Primer
<400> 54
   cgagcctaag gagttaagca tcc 23
<210> 55
   <211> 20
   <212> DNA
   <213> Artificial Sequence
<220>
   <223> Forward Primer
<400> 55
   ggtggagtgg gagcagtcag 20
<210> 56
   <211> 18
   <212> DNA
   <213> Artificial Sequence
<220>
   <223> Reverse Primer
<400> 56
   tcacggccgg aagatgag 18
<210> 57
   <211> 20
   <212> DNA
   <213> Artificial Sequence
<220>
   <223> Forward Primer
<400> 57
   aacgagctcc tttcgtggct 20
<210> 58
   <211> 20
   <212> DNA
   <213> Artificial Sequence
<220>
   <223> Reverse Primer
<400> 58
   cacataccct gttccgcagg 20
<210> 59
   <211> 23
   <212> DNA
   <213> Artificial Sequence
<220>
   <223> Forward Primer
<400> 59
   caaagtcctt gagtcccaac agt 23
<210> 60
   <211> 18
   <212> DNA
   <213> Artificial Sequence
<220>
   <223> Reverse Primer
<400> 60
   aaccccatca tcagcgga 18
<210> 61
   <211> 20
   <212> DNA
   <213> Artificial Sequence
<220>
   <223> Forward Primer
<400> 61
   tgagcgttgc cattatcacc 20
<210> 62
   <211> 22
   <212> DNA
   <213> Artificial Sequence
<220>
   <223> Reverse Primer
<400> 62
   tcctctctca tcaccatcac gt 22
<210> 63
   <211> 19
   <212> DNA
   <213> Artificial Sequence
<220>
   <223> Forward Primer
<400> 63
   ccagggtctt ctccaaggc 19
<210> 64
   <211> 19
   <212> DNA
   <213> Artificial Sequence
<220>
   <223> Reverse Primer
<400> 64
   gccgtgagtt cagaggcag 19
<210> 65
   <211> 18
   <212> DNA
   <213> Artificial Sequence
<220>
   <223> Forward Primer
<400> 65
   aggcccctgg gacagtgt 18
<210> 66
   <211> 17
   <212> DNA
   <213> Artificial Sequence
<220>
   <223> Reverse Primer
<400> 66
   ccgacagccg gaaggaa 17
<210> 67
   <211> 28
   <212> DNA
   <213> Artificial Sequence
<220>
   <223> Forward Primer
<400> 67
   agaacttctt agatgagagt gcaacttc 28
<210> 68
   <211> 26
   <212> DNA
   <213> Artificial Sequence
<220>
   <223> Reverse Primer
<400> 68
   gggaaagaaa atgtctatgg caaagg 26
<210> 69
   <211> 32
   <212> DNA
   <213> Artificial Sequence
<220>
   <223> Forward Primer
<400> 69
   ccatagacat tttctttccc tgatgatttt tt 32
<210> 70
   <211> 24
   <212> DNA
   <213> Artificial Sequence
<220>
   <223> Reverse Primer
<400> 70
   cttgagacac agattctgga acct 24
<210> 71
   <211> 26
   <212> DNA
   <213> Artificial Sequence
<220>
   <223> Forward Primer
<400> 71
   gtaggagctt aagacactgt gtactg 26
<210> 72
   <211> 23
   <212> DNA
   <213> Artificial Sequence
<220>
   <223> Reverse Primer
<400> 72
   gccaaggcat ctgaactaag aga 23
<210> 73
   <211> 21
   <212> DNA
   <213> Artificial Sequence
<220>
   <223> Forward Primer
<400> 73
   gcagctcatc ccaacctcat c 21
<210> 74
   <211> 21
   <212> DNA
   <213> Artificial Sequence
<220>
   <223> Reverse Primer
<400> 74
   gcagctcatc ccaacctcat c 21
<210> 75
   <211> 25
   <212> DNA
   <213> Artificial Sequence
<220>
   <223> Forward Primer
<400> 75
   gttcagagtg ctgttcatga gtgat 25
<210> 76
   <211> 19
   <212> DNA
   <213> Artificial Sequence
<220>
   <223> Reverse Primer
<400> 76
   caggatggca gacgtcaga 19
<210> 77
   <211> 41
   <212> DNA
   <213> Artificial Sequence
<220>
   <223> Forward Primer
<400> 77
   actgtaaaga aagagaatcc attctgataa ttaatcaata t 41
<210> 78
   <211> 25
   <212> DNA
   <213> Artificial Sequence
<220>
   <223> Reverse Primer
<400> 78
   gctgttttgt gtgctctaga aagtg 25
<210> 79
   <211> 21
   <212> DNA
   <213> Artificial Sequence
<220>
   <223> Forward Primer
<400> 79
   gcgaggagaa aatcgcaact t 21
<210> 80
   <211> 27
   <212> DNA
   <213> Artificial Sequence
<220>
   <223> Reverse Primer
<400> 80
   tttccaaacc tgaaggaaca taaggaa 27
<210> 81
   <211> 19
   <212> DNA
   <213> Artificial Sequence
<220>
   <223> Forward Primer
<400> 81
   gagtcagggc ctgggaaga 19
<210> 82
   <211> 19
   <212> DNA
   <213> Artificial Sequence
<220>
   <223> Reverse Primer
<400> 82
   ggaggggcaa gaagatgga 19
<210> 83
   <211> 22
   <212> DNA
   <213> Artificial Sequence
<220>
   <223> Forward Primer
<400> 83
   acccttagca atgtctgcac gt 22
<210> 84
   <211> 22
   <212> DNA
   <213> Artificial Sequence
<220>
   <223> Reverse Primer
<400> 84
   ggttcttccc cactgatcac tg 22
<210> 85
   <211> 22
   <212> DNA
   <213> Artificial Sequence
<220>
   <223> Forward Primer
<400> 85
   gttcttcccc actgatcact gt 22
<210> 86
   <211> 17
   <212> DNA
   <213> Artificial Sequence
<220>
   <223> Reverse Primer
<400> 86
   tgagtcaggg cctggga 17
<210> 87
   <211> 22
   <212> DNA
   <213> Artificial Sequence
<220>
   <223> Forward Primer
<400> 87
   agtaccgctg cctttttgaa aa 22
<210> 88
   <211> 23
   <212> DNA
   <213> Artificial Sequence
<220>
   <223> Reverse Primer
<400> 88
   gtggcaggag caggtattga ata 23
<210> 89
   <211> 22
   <212> DNA
   <213> Artificial Sequence
<220>
   <223> Forward Primer
<400> 89
   agactgaccc tgcagatact ca 22
<210> 90
   <211> 23
   <212> DNA
   <213> Artificial Sequence
<220>
   <223> Reverse Primer
<400> 90
   ttttcaaaaa ggcagcggta ctg 23
<210> 91
   <211> 23
   <212> DNA
   <213> Artificial Sequence
<220>
   <223> Forward Primer
<400> 91
   ggagagcaga tggcagagta cag 23
<210> 92
   <211> 19
   <212> DNA
   <213> Artificial Sequence
<220>
   <223> Reverse Primer
<400> 92
   cctcgtcaat ggcgtcact 19
<210> 93
   <211> 21
   <212> DNA
   <213> Artificial Sequence
<220>
   <223> Forward Primer
<400> 93
   ttgcagctgt gttacctgac a 21
<210> 94
   <211> 22
   <212> DNA
   <213> Artificial Sequence
<220>
   <223> Reverse Primer
<400> 94
   cagagatgat gccatgcttc ct 22
<210> 95
   <211> 21
   <212> DNA
   <213> Artificial Sequence
<220>
   <223> Forward Primer
<400> 95
   tctgagcatc gcatccaaga t 21
<210> 96
   <211> 21
   <212> DNA
   <213> Artificial Sequence
<220>
   <223> Reverse Primer
<400> 96
   agactctgca gaggcgttcc t 21
<210> 97
   <211> 20
   <212> DNA
   <213> Artificial Sequence
<220>
   <223> Forward Primer
<400> 97 20
   ccagagcccc aggtgtattg 20
<210> 98
   <211> 20
   <212> DNA
   <213> Artificial Sequence
<220>
   <223> Reverse Primer
<400> 98
   tggaccaagc aggacacaga 20
<210> 99
   <211> 20
   <212> DNA
   <213> Artificial Sequence
<220>
   <223> Forward Primer
<400> 99 20
   cagagcccca ggtgtattgg 20
<210> 100
   <211> 18
   <212> DNA
   <213> Artificial Sequence
<220>
   <223> Reverse Primer
<400> 100
   acacggccag cagcttct 18
<210> 101
   <211> 25
   <212> DNA
   <213> Artificial Sequence
<220>
   <223> Forward Primer
<400> 101
   gactgagatg cagatggagg agtac 25
<210> 102
   <211> 23
   <212> DNA
   <213> Artificial Sequence
<220>
   <223> Reverse Primer
<400> 102
   ctttgcaatg ccattctcta tcc 23
<210> 103
   <211> 20
   <212> DNA
   <213> Artificial Sequence
<220>
   <223> Forward Primer
<400> 103
   tgccagctac tccccaagag 20
<210> 104
   <211> 18
   <212> DNA
   <213> Artificial Sequence
<220>
   <223> Reverse Primer
<400> 104
   gggctctgag cggtacca 18
<210> 105
   <211> 19
   <212> DNA
   <213> Artificial Sequence
<220>
   <223> Forward Primer
<400> 105
   ggagccaagc ccatttcac 19
<210> 106
   <211> 25
   <212> DNA
   <213> Artificial Sequence
<220>
   <223> Reverse Primer
<400> 106
   cagtcttagc agagaatcca catca 25
<210> 107
   <211> 26
   <212> DNA
   <213> Artificial Sequence
<220>
   <223> Forward Primer
<400> 107
   aatgtgcttc aggtatatcc ctgtct 26
<210> 108
   <211> 30
   <212> DNA
   <213> Artificial Sequence
<220>
   <223> Reverse Primer
<400> 108
   agtaaagttc ttaaacaaac aggacaacaa 30
<210> 109
   <211> 26
   <212> DNA
   <213> Artificial Sequence
<220>
   <223> Forward Primer
<400> 109
   aatgtgcttc aggtatatcc ctgtct 26
<210> 110
   <211> 30
   <212> DNA
   <213> Artificial Sequence
<220>
   <223> Reverse Primer
<400> 110
   agtaaagttc ttaaacaaac aggacaacaa 30
<210> 111
   <211> 26
   <212> DNA
   <213> Artificial Sequence
<220>
   <223> Forward Primer
<400> 111
   aatgtgcttc aggtatatcc ctgtct 26
<210> 112
   <211> 27
   <212> DNA
   <213> Artificial Sequence
<220>
   <223> Reverse Primer
<400> 112
   ccgttcattg gataaagaag taaagtt 27
<210> 113
   <211> 26
   <212> DNA
   <213> Artificial Sequence
<220>
   <223> Forward Primer
<400> 113
   ttatccaatg aacggagtat cttgtg 26
<210> 114
   <211> 21
   <212> DNA
   <213> Artificial Sequence
<220>
   <223> Reverse Primer
<400> 114
   atgacgcatc tgttgctagg g 21
<210> 115
   <211> 22
   <212> DNA
   <213> Artificial Sequence
<220>
   <223> Forward Primer
<400> 115
   cctcactccc gagctctact ga 22
<210> 116
   <211> 24
   <212> DNA
   <213> Artificial Sequence
<220>
   <223> Reverse Primer
<400> 116
   ccacttatgg ccattttctt cttg 24
<210> 117
   <211> 18
   <212> DNA
   <213> Artificial Sequence
<220>
   <223> Forward Primer
<400> 117
   tgggcctgac tgtgggtc 18
<210> 118
   <211> 23
   <212> DNA
   <213> Artificial Sequence
<220>
   <223> Reverse Primer
<400> 118
   accacaccta actcacctcc atg 23
<210> 119
   <211> 22
   <212> DNA
   <213> Artificial Sequence
<220>
   <223> Forward Primer
<400> 119
   aatgggtaag gccagtcttc ag 22
<210> 120
   <211> 25
   <212> DNA
   <213> Artificial Sequence
<220>
   <223> Reverse Primer
<400> 120
   tgcaggattt acatatcaac atcgt 25
<210> 121
   <211> 25
   <212> DNA
   <213> Artificial Sequence
<220>
   <223> Forward Primer
<400> 121
   ggtggtttca agaatcagtc agact 25
<210> 122
   <211> 25
   <212> DNA
   <213> Artificial Sequence
<220>
   <223> Reverse Primer
<400> 122
   ggccttaccc attctgttag acata 25
<210> 123
   <211> 22
   <212> DNA
   <213> Artificial Sequence
<220>
   <223> Forward Primer
<400> 123
   catagaggtt cctgaggtca ca 22
<210> 124
   <211> 22
   <212> DNA
   <213> Artificial Sequence
<220>
   <223> Reverse Primer
<400> 124
   tggtctcaga aacaccttct gt 22
<210> 125
   <211> 19
   <212> DNA
   <213> Artificial Sequence
<220>
   <223> Forward Primer
<400> 125
   ggtggtttca gttgggcag 19
<210> 126
   <211> 22
   <212> DNA
   <213> Artificial Sequence
<220>
   <223> Reverse Primer
<400> 126
   ctccatttcc tggatgaagt ca 22
<210> 127
   <211> 18
   <212> DNA
   <213> Artificial Sequence
<220>
   <223> Forward Primer
<400> 127
   gcccagccca atggagac 18
<210> 128
   <211> 24
   <212> DNA
   <213> Artificial Sequence
<220>
   <223> Reverse Primer
<400> 128
   cctaattcca tccatctgcc atac 24
<210> 129
   <211> 21
   <212> DNA
   <213> Artificial Sequence
<220>
   <223> Forward Primer
<400> 129
   aaaaggcctg gaagctgagt c 21
<210> 130
   <211> 20
   <212> DNA
   <213> Artificial Sequence
<220>
   <223> Reverse Primer
<400> 130
   gagtttggca agcccaacac 20
<210> 131
   <211> 24
   <212> DNA
   <213> Artificial Sequence
<220>
   <223> Forward Primer
<400> 131
   gatgccagat gaaccactag aacc 24
<210> 132
   <211> 24
   <212> DNA
   <213> Artificial Sequence
<220>
   <223> Reverse Primer
<400> 132 24
   cctcagagga gctctcactg ctac 24
<210> 133
   <211> 22
   <212> DNA
   <213> Artificial Sequence
<220>
   <223> Forward Primer
<400> 133
   tgtgctgact ttgagtggga tc 22
<210> 134
   <211> 23
   <212> DNA
   <213> Artificial Sequence
<220>
   <223> Reverse Primer
<400> 134
   aacaccacca aagcatttag tgc 23
<210> 135
   <211> 23
   <212> DNA
   <213> Artificial Sequence
<220>
   <223> Forward Primer
<400> 135
   ctccttctct tccactgatg tgc 23
<210> 136
   <211> 19
   <212> DNA
   <213> Artificial Sequence
<220>
   <223> Reverse Primer
<400> 136
   gcacgtgaag acctgtggc 19
<210> 137
   <211> 21
   <212> DNA
   <213> Artificial Sequence
<220>
   <223> Forward Primer
<400> 137
   agtgcgggag gtgaggaata g 21
<210> 138
   <211> 24
   <212> DNA
   <213> Artificial Sequence
<220>
   <223> Reverse Primer
<400> 138
   ccccaaatac acactcattg tgtc 24
<210> 139
   <211> 25
   <212> DNA
   <213> Artificial Sequence
<220>
   <223> Forward Primer
<400> 139
   cctcaggctt aggtgtgtat tagca 25
<210> 140
   <211> 23
   <212> DNA
   <213> Artificial Sequence
<220>
   <223> Reverse Primer
<400> 140
   tcgtgcctgt agaatctact gcc 23
<210> 141
   <211> 19
   <212> DNA
   <213> Artificial Sequence
<220>
   <223> Forward Primer
<400> 141
   gcatttgctg cttgggatg 19
<210> 142
   <211> 19
   <212> DNA
   <213> Artificial Sequence
<220>
   <223> Reverse Primer
<400> 142
   agcaagctcc agggtgacc 19
<210> 143
   <211> 21
   <212> DNA
   <213> Artificial Sequence
<220>
   <223> Forward Primer
<400> 143
   ccctccatca gactggcact c 21
<210> 144
   <211> 19
   <212> DNA
   <213> Artificial Sequence
<220>
   <223> Reverse Primer
<400> 144
   cggtcacaca gccacatgc 19
<210> 145
   <211> 20
   <212> DNA
   <213> Artificial Sequence
<220>
   <223> Forward Primer
<400> 145
   ccccaagacc cagagagacc 20
<210> 146
   <211> 22
   <212> DNA
   <213> Artificial Sequence
<220>
   <223> Reverse Primer
<400> 146
   agggatacag ctccatgctg aa 22
<210> 147
   <211> 24
   <212> DNA
   <213> Artificial Sequence
<220>
   <223> Forward Primer
<400> 147
   ttgaagtcct gtatcacagg catg 24
<210> 148
   <211> 29
   <212> DNA
   <213> Artificial Sequence
<220>
   <223> Reverse Primer
<400> 148
   accaacagtg ttaagaccat gttactagg 29
<210> 149
   <211> 23
   <212> DNA
   <213> Artificial Sequence
<220>
   <223> Forward Primer
<400> 149
   ttccatcatg accaagctgt agc 23
<210> 150
   <211> 21
   <212> DNA
   <213> Artificial Sequence
<220>
   <223> Reverse Primer
<400> 150
   cagagggaga taaggcatgg c 21
<210> 151
   <211> 27
   <212> DNA
   <213> Artificial Sequence
<220>
   <223> Forward Primer
<400> 151
   tgttggtatg ttggagaatt atgtgaa 27
<210> 152
   <211> 25
   <212> DNA
   <213> Artificial Sequence
<220>
   <223> Reverse Primer
<400> 152
   aaacaagtaa attcactgcc tgcac 25
<210> 153
   <211> 26
   <212> DNA
   <213> Artificial Sequence
<220>
   <223> Forward Primer
<400> 153
   aaaaaagcct gttgtcttag gtatgc 26
<210> 154
   <211> 22
   <212> DNA
   <213> Artificial Sequence
<220>
   <223> Reverse Primer
<400> 154
   agaggttaat gctaggttgc gc 22
<210> 155
   <211> 22
   <212> DNA
   <213> Artificial Sequence
<220>
   <223> Forward Primer
<400> 155
   caggtgattg gtctgttgga cg 22
<210> 156
   <211> 23
   <212> DNA
   <213> Artificial Sequence
<220>
   <223> Reverse Primer
<400> 156
   cccaaaaaga ggtgcatgat agg 23
<210> 157
   <211> 30
   <212> DNA
   <213> Artificial Sequence
<220>
   <223> Forward Primer
<400> 157
   ggagaagagg gctaatatat cctagagtag 30
<210> 158
   <211> 23
   <212> DNA
   <213> Artificial Sequence
<220>
   <223> Reverse Primer
<400> 158
   ggccgctgta attctcttat ctg 23
<210> 159
   <211> 22
   <212> DNA
   <213> Artificial Sequence
<220>
   <223> Forward Primer
<400> 159
   gaagtcatca gctttgtgcc ac 22
<210> 160
   <211> 21
   <212> DNA
   <213> Artificial Sequence
<220>
   <223> Reverse Primer
<400> 160
   accatggagg aaatctctgc a 21
<210> 161
   <211> 21
   <212> DNA
   <213> Artificial Sequence
<220>
   <223> Forward Primer
<400> 161
   ctgtgaggac tggcagatgt g 21
<210> 162
   <211> 20
   <212> DNA
   <213> Artificial Sequence
<220>
   <223> Reverse Primer
<400> 162
   gcctttgcct gtcaagtgtg 20
<210> 163
   <211> 20
   <212> DNA
   <213> Artificial Sequence
<220>
   <223> Forward Primer
<400> 163
   cagcttcttc catctgctca 20
<210> 164
   <211> 21
   <212> DNA
   <213> Artificial Sequence
<220>
   <223> Reverse Primer
<400> 164
   acagtcgata ttgggtagcc a 21
<210> 165
   <211> 20
   <212> DNA
   <213> Artificial Sequence
<220>
   <223> Forward Primer
<400> 165
   ctaggcctcc aacccaggac 20
<210> 166
   <211> 24
   <212> DNA
   <213> Artificial Sequence
<220>
   <223> Reverse Primer
<400> 166
   cccatggctt atttacagct agga 24
<210> 167
   <211> 24
   <212> DNA
   <213> Artificial Sequence
<220>
   <223> Forward Primer
<400> 167
   ttcagaactg ggctgatatc acag 24
<210> 168
   <211> 20
   <212> DNA
   <213> Artificial Sequence
<220>
   <223> Reverse Primer
<400> 168
   aatgaggcca tggtggtgac 20
<210> 169
   <211> 21
   <212> DNA
   <213> Artificial Sequence
<220>
   <223> Forward Primer
<400> 169
   agaagaagag acgcaggacc g 21
<210> 170
   <211> 23
   <212> DNA
   <213> Artificial Sequence
<220>
   <223> Reverse Primer
<400> 170
   ccattttcag aactgggatg ata 23
<210> 171
   <211> 22
   <212> DNA
   <213> Artificial Sequence
<220>
   <223> Forward Primer
<400> 171
   ctcctgactc actctggcct tg 22
<210> 172
   <211> 21
   <212> DNA
   <213> Artificial Sequence
<220>
   <223> Reverse Primer
<400> 172
   tccactctga ggacaaccgt g 21
<210> 173
   <211> 26
   <212> DNA
   <213> Artificial Sequence
<220>
   <223> Forward Primer
<400> 173
   catggtggta atgaattgtg attatg 26
<210> 174
   <211> 27
   <212> DNA
   <213> Artificial Sequence
<220>
   <223> Reverse Primer
<400> 174
   atgcttaaga tttatgtcat gagtcca 27
<210> 175
   <211> 19
   <212> DNA
   <213> Artificial Sequence
<220>
   <223> Forward Primer
<400> 175
   aggaggctga ggtcaaggc 19
<210> 176
   <211> 27
   <212> DNA
   <213> Artificial Sequence
<220>
   <223> Reverse Primer
<400> 176
   gccatcatga aacttatcac tactgac 27
<210> 177
   <211> 21
   <212> DNA
   <213> Artificial Sequence
<220>
   <223> Forward Primer
<400> 177
   aggcctctag gaattggacc a 21
<210> 178
   <211> 20
   <212> DNA
   <213> Artificial Sequence
<220>
   <223> Reverse Primer
<400> 178
   gagcaggagg ccaagtcaga 20
<210> 179
   <211> 22
   <212> DNA
   <213> Artificial Sequence
<220>
   <223> Forward Primer
<400> 179
   aacacagagg gagggtaggt ca 22
<210> 180
   <211> 24
   <212> DNA
   <213> Artificial Sequence
<220>
   <223> Reverse Primer
<400> 180
   gttgtgatta taaagccctg gaca 24
<210> 181
   <211> 25
   <212> DNA
   <213> Artificial Sequence
<220>
   <223> Forward Primer
<400> 181
   aaaaggcagg gagttaataa catga 25
<210> 182
   <211> 20
   <212> DNA
   <213> Artificial Sequence
<220>
   <223> Reverse Primer
<400> 182
   tagaggaacg agggcagtgg 20
<210> 183
   <211> 21
   <212> DNA
   <213> Artificial Sequence
<220>
   <223> Forward Primer
<400> 183
   ctgcttgcat gtctgctgat g 21
<210> 184
   <211> 22
   <212> DNA
   <213> Artificial Sequence
<220>
   <223> Reverse Primer
<400> 184
   cgagaagaat ccacccgtag tg 22
<210> 185
   <211> 21
   <212> DNA
   <213> Artificial Sequence
<220>
   <223> Forward Primer
<400> 185
   atgttcctgc atagcactgg c 21
<210> 186
   <211> 19
   <212> DNA
   <213> Artificial Sequence
<220>
   <223> Reverse Primer
<400> 186
   aggagtgagg gcacttggc 19
<210> 187
   <211> 22
   <212> DNA
   <213> Artificial Sequence
<220>
   <223> Forward Primer
<400> 187
   gaacccattc tctttccctg ct 22
<210> 188
   <211> 24
   <212> DNA
   <213> Artificial Sequence
<220>
   <223> Reverse Primer
<400> 188
   agcgtgtagt cacatttcac atcc 24
<210> 189
   <211> 31
   <212> DNA
   <213> Artificial Sequence
<220>
   <223> Forward Primer
<400> 189
   atttacattc aagtatcatt ctcccttaga t 31
<210> 190
   <211> 28
   <212> DNA
   <213> Artificial Sequence
<220>
   <223> Reverse Primer
<400> 190
   gacatcaaca caataataca gagaccaa 28
<210> 191
   <211> 22
   <212> DNA
   <213> Artificial Sequence
<220>
   <223> Forward Primer
<400> 191
   tcaattgatg gctgatgagg ag 22
<210> 192
   <211> 22
   <212> DNA
   <213> Artificial Sequence
<220>
   <223> Reverse Primer
<400> 192
   agcagaataa aatggtgcca gg 22
<210> 193
   <211> 19
   <212> DNA
   <213> Artificial Sequence
<220>
   <223> Forward Primer
<400> 193
   gggcattgtg gtgtgatgg 19
<210> 194
   <211> 28
   <212> DNA
   <213> Artificial Sequence
<220>
   <223> Reverse Primer
<400> 194
   gcaaagtact agtgagtgta gacatgca 28
<210> 195
   <211> 18
   <212> DNA
   <213> Artificial Sequence
<220>
   <223> Forward Primer
<400> 195
   ccctaacgga ggctctgg 18
<210> 196
   <211> 20
   <212> DNA
   <213> Artificial Sequence
<220>
   <223> Reverse Primer
<400> 196
   ttccccacat cttccagcac 20
<210> 197
   <211> 20
   <212> DNA
   <213> Artificial Sequence
<220>
   <223> Forward Primer
<400> 197
   cccagtgagt gaatcaggca 20
<210> 198
   <211> 17
   <212> DNA
   <213> Artificial Sequence
<220>
   <223> Reverse Primer
<400> 198 17
   accccagagc caccgag 17
<210> 199
   <211> 23
   <212> DNA
   <213> Artificial Sequence
<220>
   <223> Forward Primer
<400> 199
   gaaggacagt gtgagagcca ctg 23
<210> 200
   <211> 26
   <212> DNA
   <213> Artificial Sequence
<220>
   <223> Reverse Primer
<400> 200
   aaagagccac aaattgctaa caacta 26
<210> 201
   <211> 29
   <212> DNA
   <213> Artificial Sequence
<220>
   <223> FAM Probe
<400> 201
   agaagagcag caaccaaagg ctaacgtga 29
<210> 202
   <211> 31
   <212> DNA
   <213> Artificial Sequence
<220>
   <223> TET Probe
<400> 202
   agaagagcag caaacaaagg ctaacgtgag a 31
<210> 203
   <211> 26
   <212> DNA
   <213> Artificial Sequence
<220>
   <223> FAM Probe
<400> 203
   ctgtagctgg cgaggaaggc aggagt 26
<210> 204
   <211> 26
   <212> DNA
   <213> Artificial Sequence
<220>
   <223> TET Probe
<400> 204
   ctgtagctgg cgaggaagcc aggagt 26
<210> 205
   <211> 34
   <212> DNA
   <213> Artificial Sequence
<220>
   <223> FAM Probe
<400> 205
   aaggggcaac atacacaatg tgtttaccaa aatt 34
<210> 206
   <211> 34
   <212> DNA
   <213> Artificial Sequence
<220>
   <223> TET Probe
<400> 206
   aaggggcaac atacacattg tgtttaccaa aatt 34
<210> 207
   <211> 25
   <212> DNA
   <213> Artificial Sequence
<220>
   <223> FAM Probe
<400> 207
   tcttggagcc cagcgaaaga acctg 25
<210> 208
   <211> 27
   <212> DNA
   <213> Artificial Sequence
<220>
   <223> TET Probe
<400> 208
   atcttggagc ccagcaaaag aacctgg 27
<210> 209
   <211> 35
   <212> DNA
   <213> Artificial Sequence
<220>
   <223> FAM Probe
<400> 209
   attgggagaa tatttctgaa tggagtatga gggag 35
<210> 210
   <211> 40
   <212> DNA
   <213> Artificial Sequence
<220>
   <223> TET Probe
<400> 210
   tatctagatt gggaaaatat ttctgaatgg agtatgaggg 40
<210> 211
   <211> 22
   <212> DNA
   <213> Artificial Sequence
<220>
   <223> FAM Probe
<400> 211
   aggaggcggc cgttctggtg tg 22
<210> 212
   <211> 24
   <212> DNA
   <213> Artificial Sequence
<220>
   <223> TET Probe
<400> 212
   tggaggaggc agccgttctg gtgt 24
<210> 213
   <211> 26
   <212> DNA
   <213> Artificial Sequence
<220>
   <223> FAM Probe
<400> 213
   cactcttggc ctcgtcttcc tctgcc 26
<210> 214
   <211> 28
   <212> DNA
   <213> Artificial Sequence
<220>
   <223> TET Probe
<400> 214
   tcactcttgg cctcatcttc ctctgcct 28
<210> 215
   <211> 20
   <212> DNA
   <213> Artificial Sequence
<220>
   <223> FAM Probe
<400> 215
   aggaggcgtc cgtcggaccc 20
<210> 216
   <211> 20
   <212> DNA
   <213> Artificial Sequence
<220>
   <223> TET Probe
<400> 216
   aggaggcgac cgtcggaccc 20
<210> 217
   <211> 39
   <212> DNA
   <213> Artificial Sequence
<220>
   <223> FAM Probe
<400> 217
   aaatggaata agaaataatc atatcctttc ttcccaccc 39
<210> 218
   <211> 38
   <212> DNA
   <213> Artificial Sequence
<220>
   <223> TET Probe
<400> 218
   aaatggaaca agaaataatc atatcctttc ttcccacc 38
<210> 219
   <211> 20
   <212> DNA
   <213> Artificial Sequence
<220>
   <223> FAM Probe
<400> 219
   cccgacaggc gcttcctccg 20
<210> 220
   <211> 19
   <212> DNA
   <213> Artificial Sequence
<220>
   <223> TET Probe
<400> 220
   cccgacgggc gcttcctcc 19
<210> 221
   <211> 30
   <212> DNA
   <213> Artificial Sequence
<220>
   <223> FAM Probe
<400> 221
   cattctgggc tcttgcctgt gaaatctttc 30
<210> 222
   <211> 31
   <212> DNA
   <213> Artificial Sequence
<220>
   <223> TET Probe
<400> 222
   ccattctggg ctctttcctg tgaaatcttt c 31
<210> 223
   <211> 23
   <212> DNA
   <213> Artificial Sequence
<220>
   <223> FAM Probe
<400> 223
   cacgggatga tggcactgct gct 23
<210> 224
   <211> 23
   <212> DNA
   <213> Artificial Sequence
<220>
   <223> TET Probe
<400> 224
   cacgggatgt tggcactgct get 23
<210> 225
   <211> 22
   <212> DNA
   <213> Artificial Sequence
<220>
   <223> FAM Probe
<400> 225
   acctggggaa gtggggacca cg 22
<210> 226
   <211> 22
   <212> DNA
   <213> Artificial Sequence
<220>
   <223> TET Probe
<400> 226
   acctggggaa atggggacca cg 22
<210> 227
   <211> 22
   <212> DNA
   <213> Artificial Sequence
<220>
   <223> FAM Probe
<400> 227
   ccaggcacag gctgaccgag tg 22
<210> 228
   <211> 23
   <212> DNA
   <213> Artificial Sequence
<220>
   <223> TET Probe
<400> 228
   cgccaggcac agactgaccg agt 23
<210> 229
   <211> 25
   <212> DNA
   <213> Artificial Sequence
<220>
   <223> FAM Probe
<400> 229
   agctgtggtc gctgctgtga tgtga 25
<210> 230
   <211> 29
   <212> DNA
   <213> Artificial Sequence
<220>
   <223> TET Probe
<900> 230
   agctgtggtc gctactgtga tgtgaggag 29
<210> 231
   <211> 14
   <212> DNA
   <213> Artificial Sequence
<220>
   <223> FAM Probe
<400> 231
   atccccggac agca 14
<210> 232
   <211> 15
   <212> DNA
   <213> Artificial Sequence
<220>
   <223> VIC Probe
<400> 232
   catcccagga cagca 15
<210> 233
   <211> 26
   <212> DNA
   <213> Artificial Sequence
<220>
   <223> FAM Probe
<400> 233
   accaggattt gccaaggaga ggagca 26
<210> 234
   <211> 25
   <212> DNA
   <213> Artificial Sequence
<220>
   <223> VIC Probe
<400> 234
   accaggattt gccgaggaga ggagc 25
<210> 235
   <211> 14
   <212> DNA
   <213> Artificial Sequence
<220>
   <223> FAM Probe
<400> 235
   caggacattt tctg 14
<210> 236
   <211> 15
   <212> DNA
   <213> Artificial Sequence
<220>
   <223> TET Probe
<400> 236
   aggacatctt ctgcc 15
<210> 237
   <211> 29
   <212> DNA
   <213> Artificial Sequence
<220>
   <223> FAM Probe
<400> 237
   tctgtgagat ccatgaagac agcagcacc 29
<210> 238
   <211> 28
   <212> DNA
   <213> Artificial Sequence
<220>
   <223> TET Probe
<400> 238
   ctgtgagatg catgaagaca gcagcacc 28
<210> 239
   <211> 15
   <212> DNA
   <213> Artificial Sequence
<220>
   <223> FAM Probe
<400> 239
   tgcagatgac attca 15
<210> 240
   <211> 14
   <212> DNA
   <213> Artificial Sequence
<220>
   <223> TET Probe
<400> 240
   tgcaggtgac attc 14
<210> 241
   <211> 24
   <212> DNA
   <213> Artificial Sequence
<220>
   <223> FAM Probe
<400> 241
   tcgacaggat ccctctgctt ctcg 24
<210> 242
   <211> 23
   <212> DNA
   <213> Artificial Sequence
<220>
   <223> TET Probe
<400> 242
   tcgacgggat ccctctgctt ctc 23
<210> 243
   <211> 24
   <212> DNA
   <213> Artificial Sequence
<220>
   <223> FAM Probe
<400> 243
   tgcccacagc aacctcaaac ctgc 24
<210> 244
   <211> 24
   <212> DNA
   <213> Artificial Sequence
<220>
   <223> TET Probe
<400> 244
   ttgcccacag caccctcaaa cctg 24
<210> 245
   <211> 35
   <212> DNA
   <213> Artificial Sequence
<220>
   <223> FAM Probe
<400> 245
   agcaaaggag aagctgagaa gatgaaggaa aagtc 35
<210> 246
   <211> 33
   <212> DNA
   <213> Artificial Sequence
<220>
   <223> TET Probe
<400> 246
   agcaaaggag aagctgagaa gacgaaggaa aag 33
<210> 247
   <211> 27
   <212> DNA
   <213> Artificial Sequence
<220>
   <223> FAM Probe
<400> 247
   agtatgggga ccccccctta atgaaga 27
<210> 248
   <211> 24
   <212> DNA
   <213> Artificial Sequence
<220>
   <223> TET Probe
<400> 248
   tatggggacc cccccttaac gaag 24
<210> 249
   <211> 21
   <212> DNA
   <213> Artificial Sequence
<220>
   <223> FAM Probe
<400> 249
   aaccccgtcc tcatgcccct c 21
<210> 250
   <211> 21
   <212> DNA
   <213> Artificial Sequence
<220>
   <223> TET Probe
<400> 250
   aaccccgtcc ccatgcccct c 21
<210> 251
   <211> 23
   <212> DNA
   <213> Artificial Sequence
<220>
   <223> FAM Probe
<400> 251
   aatcagagca gggaggatgg gga 23
<210> 252
   <211> 21
   <212> DNA
   <213> Artificial Sequence
<220>
   <223> TET Probe
<400> 252
   aatcggagca gggaggatgg g 21
<210> 253
   <211> 29
   <212> DNA
   <213> Artificial Sequence
<220>
   <223> FAM Probe
<400> 253
   tttatactag gtcgtgctag gggcagcgt 29
<210> 254
   <211> 27
   <212> DNA
   <213> Artificial Sequence
<220>
   <223> TET Probe
<400> 254
   tttatactag gtcgcgctag gggcagc 27
<210> 255
   <211> 26
   <212> DNA
   <213> Artificial Sequence
<220>
   <223> FAM Probe
<400> 255
   ccatagatga cgtccagggg atcgat 26
<210> 256
   <211> 27
   <212> DNA
   <213> Artificial Sequence
<220>
   <223> TET Probe
<400> 256
   ccatagatga catccagggg atcgatg 27
<210> 257
   <211> 35
   <212> DNA
   <213> Artificial Sequence
<220>
   <223> FAM Probe
<400> 257
   cataagagaa aggaattgga gcagatgtgt aaccc 35
<210> 258
   <211> 36
   <212> DNA
   <213> Artificial Sequence
<220>
   <223> TET Probe
<400> 258
   tcataagaga aaggaattga agcagatgtg taaccc 36
<210> 259
   <211> 17
   <212> DNA
   <213> Artificial Sequence
<220>
   <223> FAM Probe
<400> 259
   cccgaagccg ccaggcc 17
<210> 260
   <211> 18
   <212> DNA
   <213> Artificial Sequence
<220>
   <223> TET Probe
<400> 260
   ccccgaagcc cccaggcc 18
<210> 261
   <211> 27
   <212> DNA
   <213> Artificial Sequence
<220>
   <223> FAM Probe
<400> 261
   tcccgggata tgactgaggt gatcagc 27
<210> 262
   <211> 26
   <212> DNA
   <213> Artificial Sequence
<220>
   <223> TET Probe
<400> 262
   cccgggatat gactgacgtg atcagc 26
<210> 263
   <211> 32
   <212> DNA
   <213> Artificial Sequence
<220>
   <223> FAM Probe
<400> 263
   tggcctcatt tccacataca gagtttgaga ac 32
<210> 264
   <211> 28
   <212> DNA
   <213> Artificial Sequence
<220>
   <223> TET Probe
<400> 264
   tggcctcatt tccacacaca gagtttga 28
<210> 265
   <211> 13
   <212> DNA
   <213> Artificial Sequence
<220>
   <223> FAM Probe
<400> 265
   ccaacggctc cct 13
<210> 266
   <211> 14
   <212> DNA
   <213> Artificial Sequence
<220>
   <223> TET Probe
<400> 266
   ccaacagctc cctc 14
<210> 267
   <211> 16
   <212> DNA
   <213> Artificial Sequence
<220>
   <223> FAM Probe
<400> 267
   tgagcgtagt cgattc 16
<210> 268
   <211> 16
   <212> DNA
   <213> Artificial Sequence
<220>
   <223> TET Probe
<400> 268
   tgagcgtagt agattc 16
<210> 269
   <211> 18
   <212> DNA
   <213> Artificial Sequence
<220>
   <223> FAM Probe
<400> 269
   caaacacatt tttctttc 18
<210> 270
   <211> 18
   <212> DNA
   <213> Artificial Sequence
<220>
   <223> TET Probe
<400> 270
   caaacacatg tttctttc 18
<210> 271
   <211> 16
   <212> DNA
   <213> Artificial Sequence
<220>
   <223> FAM Probe
<400> 271
   agggctattg cttatt 16
<210> 272
   <211> 17
   <212> DNA
   <213> Artificial Sequence
<220>
   <223> TET Probe
<400> 272
   cagggctatt acttatt 17
<210> 273
   <211> 17
   <212> DNA
   <213> Artificial Sequence
<220>
   <223> FAM Probe
<400> 273
   ttcttcaacg gagaatg 17
<210> 274
   <211> 17
   <212> DNA
   <213> Artificial Sequence
<220>
   <223> TET Probe
<400> 274
   ttcttcaaca gagaatg 17
<210> 275
   <211> 13
   <212> DNA
   <213> Artificial Sequence
<220>
   <223> FAM Probe
<400> 275
   cgcatgggac cgc 13
<210> 276
   <211> 14
   <212> DNA
   <213> Artificial Sequence
<220>
   <223> TET Probe
<400> 276
   ccgcatagga ccgc 14
<210> 277
   <211> 23
   <212> DNA
   <213> Artificial Sequence
<220>
   <223> FAM Probe
<400> 277
   ttccacttta aatttcttac gtc 23
<210> 278
   <211> 24
   <212> DNA
   <213> Artificial Sequence
<220>
   <223> TET Probe
<400> 278
   cttccacttt aaatttctta tgtc 24
<210> 279
   <211> 19
   <212> DNA
   <213> Artificial Sequence
<220>
   <223> FAM Probe
<400> 279
   tttctctctc atgttggtg 19
<210> 280
   <211> 19
   <212> DNA
   <213> Artificial Sequence
<220>
   <223> TET Probe
<400> 280
   tttctctctc aggttggtg 19
<210> 281
   <211> 15
   <212> DNA
   <213> Artificial Sequence
<220>
   <223> FAM Probe
<400> 281
   tgtccctcca ctgca 15
<210> 282
   <211> 15
   <212> DNA
   <213> Artificial Sequence
<220>
   <223> TET Probe
<400> 282
   atgtccctcc atggc 15
<210> 283
   <211> 16
   <212> DNA
   <213> Artificial Sequence
<220>
   <223> FAM Probe
<400> 283
   atccgaagag catatc 16
<210> 284
   <211> 17
   <212> DNA
   <213> Artificial Sequence
<220>
   <223> FAM Probe
<400> 284
   catctgaaga gcatatc 17
<210> 285
   <211> 17
   <212> DNA
   <213> Artificial Sequence
<220>
   <223> VIC Probe
<400> 285
   catctgaaga gcacatc 17
<210> 286
   <211> 16
   <212> DNA
   <213> Artificial Sequence
<220>
   <223> VIC Probe
<400> 286
   atccgaagag cacatc 16
<210> 287
   <211> 17
   <212> DNA
   <213> Artificial Sequence
<220>
   <223> FAM Probe
<400> 287
   tggagaaatg cagctga 17
<210> 288
   <211> 18
   <212> DNA
   <213> Artificial Sequence
<220>
   <223> TET Probe
<400> 288
   atggagaaat gcagccga 18
<210> 289
   <211> 17
   <212> DNA
   <213> Artificial Sequence
<220>
   <223> FAM Probe
<400> 289
   aaggtggtag gtaagaa 17
<210> 290
   <211> 17
   <212> DNA
   <213> Artificial Sequence
<220>
   <223> TET Probe
<400> 290
   aggtggtaag taagaat 17
<210> 291
   <211> 14
   <212> DNA
   <213> Artificial Sequence
<220>
   <223> FAM Probe
<400> 291
   tcgtccaaag gtct 14
<210> 292
   <211> 14
   <212> DNA
   <213> Artificial Sequence
<220>
   <223> TET Probe
<400> 292
   tcgtccgaag gtct 14
<210> 293
   <211> 14
   <212> DNA
   <213> Artificial Sequence
<220>
   <223> FAM Probe
<400> 293
   accagcacag tcct 14
<210> 294
   <211> 15
   <212> DNA
   <213> Artificial Sequence
<220>
   <223> TET Probe
<400> 294
   accagtacag tcctc 15
<210> 295
   <211> 17
   <212> DNA
   <213> Artificial Sequence
<220>
   <223> FAM Probe
<400> 295
   atcagatggt cacctat 17
<210> 296
   <211> 18
   <212> DNA
   <213> Artificial Sequence
<220>
   <223> TET Probe
<400> 296
   catcagatgg acacctat 18
<210> 297
   <211> 15
   <212> DNA
   <213> Artificial Sequence
<220>
   <223> FAM Probe
<400> 297
   tggcttccac ataga 15
<210> 298
   <211> 14
   <212> DNA
   <213> Artificial Sequence
<220>
   <223> TET Probe
<400> 298
   cttccgcata gaac 14
<210> 299
   <211> 29
   <212> DNA
   <213> Artificial Sequence
<220>
   <223> FAM Probe
<400> 299
   catcgcatcc aagataaaga tggcctgtt 29
<210> 300
   <211> 26
   <212> DNA
   <213> Artificial Sequence
<220>
   <223> TET Probe
<400> 300
   catcgcatcc aagatgaaga tggcct 26
<210> 301
   <211> 13
   <212> DNA
   <213> Artificial Sequence
<220>
   <223> FAM Probe
<400> 301
   ctccccggat gtc 13
<210> 302
   <211> 14
   <212> DNA
   <213> Artificial Sequence
<220>
   <223> TET Probe
<400> 302
   ctccctggat gtct 14
<210> 303
   <211> 16
   <212> DNA
   <213> Artificial Sequence
<220>
   <223> FAM Probe
<400> 303
   agaggctggg tagagt 16
<210> 304
   <211> 16
   <212> DNA
   <213> Artificial Sequence
<220>
   <223> TET Probe
<400> 304
   agaggcaggg tagagt 16
<210> 305
   <211> 16
   <212> DNA
   <213> Artificial Sequence
<220>
   <223> FAM Probe
<400> 305
   aatgcatgtg gaggtg 16
<210> 306
   <211> 15
   <212> DNA
   <213> Artificial Sequence
<220>
   <223> TET Probe
<400> 306
   aatgcacgtg gaggt 15
<210> 307
   <211> 14
   <212> DNA
   <213> Artificial Sequence
<220>
   <223> FAM Probe
<400> 307
   acccctgaac agtc 14
<210> 308
   <211> 13
   <212> DNA
   <213> Artificial Sequence
<220>
   <223> TET Probe
<400> 308
   cccctgcaca gtc 13
<210> 309
   <211> 14
   <212> DNA
   <213> Artificial Sequence
<220>
   <223> FAM Probe
<400> 309
   tgtccgtgat tgac 14
<210> 310
   <211> 14
   <212> DNA
   <213> Artificial Sequence
<220>
   <223> TET Probe
<400> 310
   tgtccgtcat tgac 14
<210> 311
   <211> 20
   <212> DNA
   <213> Artificial Sequence
<220>
   <223> FAM Probe
<400> 311
   atttaagact gttagtcaat 20
<210> 312
   <211> 18
   <212> DNA
   <213> Artificial Sequence
<220>
   <223> TET Probe
<400> 312
   ttaagactgt tggtcaat 18
<210> 313
   <211> 16
   <212> DNA
   <213> Artificial Sequence
<220>
   <223> FAM Probe
<400> 313
   caacaacaac aacaaa 16
<210> 314
   <211> 15
   <212> DNA
   <213> Artificial Sequence
<220>
   <223> TET Probe
<400> 314
   acaacgacaa caaat 15
<210> 315
   <211> 13
   <212> DNA
   <213> Artificial Sequence
<220>
   <223> FAM Probe
<400> 315
   cctggactct ttg 13
<210> 316
   <211> 14
   <212> DNA
   <213> Artificial Sequence
<220>
   <223> TET Probe
<400> 316
   ctggaccctt tgca 14
<210> 317
   <211> 14
   <212> DNA
   <213> Artificial Sequence
<220>
   <223> FAM Probe
<400> 317
   ctcccaacag agcg 14
<210> 318
   <211> 14
   <212> DNA
   <213> Artificial Sequence
<220>
   <223> TET Probe
<400> 318
   tcccaaaaga gcgc 14
<210> 319
   <211> 26
   <212> DNA
   <213> Artificial Sequence
<220>
   <223> FAM Probe
<400> 319 tcatcatcaa gggattgcgc aaaagc 26
<210> 320
   <211> 26
   <212> DNA
   <213> Artificial Sequence
<220>
   <223> TET Probe
<400> 320
   tcatcatcaa gggagtgcgc aaaagc 26
<210> 321
   <211> 16
   <212> DNA
   <213> Artificial Sequence
<220>
   <223> FAM Probe
<400> 321
   tgtccaatct ctttgc 16
<210> 322
   <211> 15
   <212> DNA
   <213> Artificial Sequence
<220>
   <223> TET Probe
<400> 322
   tgtccaacct ctttg 15
<210> 323
   <211> 16
   <212> DNA
   <213> Artificial Sequence
<220>
   <223> FAM Probe
<400> 323
   cggtaataca ataggc 16
<210> 324
   <211> 17
   <212> DNA
   <213> Artificial Sequence
<220>
   <223> TET Probe
<400> 324
   ctcggtaata aaatagg 17
<210> 325
   <211> 21
   <212> DNA
   <213> Artificial Sequence
<220>
   <223> FAM Probe
<400> 325
   ctgggtcagc ccaacaccct c 21
<210> 326
   <211> 22
   <212> DNA
   <213> Artificial Sequence
<220>
   <223> TET Probe
<400> 326
   ctgggtcagc ccaacatcct ca 22
<210> 327
   <211> 31
   <212> DNA
   <213> Artificial Sequence
<220>
   <223> FAM Probe
<400> 327
   tgtgaggaac aacattactt atgggctgca g 31
<210> 328
   <211> 27
   <212> DNA
   <213> Artificial Sequence
<220>
   <223> TET Probe
<400> 328
   tgaggaacaa cattgcttat gggctgc 27
<210> 329
   <211> 26
   <212> DNA
   <213> Artificial Sequence
<220>
   <223> FAM Probe
<400> 329
   tgtgtggtag agcacattgg ggctcc 26
<210> 330
   <211> 26
   <212> DNA
   <213> Artificial Sequence
<220>
   <223> TET Probe
<400> 330
   tgtgtggtag agcacactgg ggctcc 26
<210> 331
   <211> 25
   <212> DNA
   <213> Artificial Sequence
<220>
   <223> FAM Probe
<400> 331
   cttccacagg gatggaatga tgctg 25
<210> 332
   <211> 24
   <212> DNA
   <213> Artificial Sequence
<220>
   <223> TET Probe
<400> 332
   cttccacagg gacggaatga tgct 24
<210> 333
   <211> 25
   <212> DNA
   <213> Artificial Sequence
<220>
   <223> FAM Probe
<400> 333
   cctggcttgg ccaaatcgtc ttcag 25
<210> 334
   <211> 27
   <212> DNA
   <213> Artificial Sequence
<220>
   <223> TET Probe
<400> 334
   ccctggcttg gtcaaatcgt cttcaga 27
<210> 335
   <211> 28
   <212> DNA
   <213> Artificial Sequence
<220>
   <223> FAM Probe
<400> 335
   ccttcccatt caacacacac acacattc 28
<210> 336
   <211> 30
   <212> DNA
   <213> Artificial Sequence
<220>
   <223> TET Probe
<400> 336
   ccttcccatt caacacaaac acacattctt 30
<210> 337
   <211> 25
   <212> DNA
   <213> Artificial Sequence
<220>
   <223> FAM Probe
<400> 337
   cattgcctcc ttttcacacc gatcc 25
<210> 338
   <211> 25
   <212> DNA
   <213> Artificial Sequence
<220>
   <223> TET Probe
<400> 338
   cattgcctcc ttctcacacc gatcc 25
<210> 339
   <211> 26
   <212> DNA
   <213> Artificial Sequence
<220>
   <223> FAM Probe
<400> 339
   cctcaccact gtgggagtgt ccatgt 26
<210> 340
   <211> 26
   <212> DNA
   <213> Artificial Sequence
<220>
   <223> TET Probe
<400> 340
   ctcaccactg tgcgagtgtc catgtc 26
<210> 341
   <211> 33
   <212> DNA
   <213> Artificial Sequence
<220>
   <223> FAM Probe
<400> 341
   ctcttccagg tactgtgtct ttatggagtc cag 33
<210> 342
   <211> 28
   <212> DNA
   <213> Artificial Sequence
<220>
   <223> TET Probe
<400> 342
   tccaggtact gcgtctttat ggagtcca 28
<210> 343
   <211> 30
   <212> DNA
   <213> Artificial Sequence
<220>
   <223> FAM Probe
<400> 343
   cttctggctg cctagagttc aaccctttca 30
<210> 344
   <211> 28
   <212> DNA
   <213> Artificial Sequence
<220>
   <223> TET Probe
<400> 344
   tctggctgcc tacagttcaa ccctttca 28
<210> 345
   <211> 22
   <212> DNA
   <213> Artificial Sequence
<220>
   <223> FAM Probe
<400> 345
   cagcaggaga ggcttgccat gg 22
<210> 346
   <211> 22
   <212> DNA
   <213> Artificial Sequence
<220>
   <223> TET Probe
<400> 346
   cagcaggaga gccttgccat gg 22
<210> 347
   <211> 24
   <212> DNA
   <213> Artificial Sequence
<220>
   <223> FAM Probe
<400> 347
   caagcccaga tgacgagggc tctg 24
<210> 348
   <211> 24
   <212> DNA
   <213> Artificial Sequence
<220>
   <223> TET Probe
<400> 348
   caagcccaga tgaccagggc tctg 24
<210> 349
   <211> 26
   <212> DNA
   <213> Artificial Sequence
<220>
   <223> FAM Probe
<400> 349
   actcgacagt gagatgtacg cgctgc 26
<210> 350
   <211> 23
   <212> DNA
   <213> Artificial Sequence
<220>
   <223> TET Probe
<400> 350
   tcggcagtga gatgtacgcg ctg 23
<210> 351
   <211> 37
   <212> DNA
   <213> Artificial Sequence
<220>
   <223> FAM Probe
<400> 351
   actctggtaa gcactgctac aaagtgcatc tttagaa 37
<210> 352
   <211> 39
   <212> DNA
   <213> Artificial Sequence
<220>
   <223> TET Probe
<400> 352
   cgtactctgg taagcactgc tacaaagtga atctttaga 39
<210> 353
   <211> 35
   <212> DNA
   <213> Artificial Sequence
<220>
   <223> FAM Probe
<400> 353
   tagaaaaccc ctacgtgcta tatatgtgtg tgcgt 35
<210> 354
   <211> 38
   <212> DNA
   <213> Artificial Sequence
<220>
   <223> TET Probe
<400> 354
   tagaaaaccc ctacatgcta tatatgtgtg tgcgtatg 38
<210> 355
   <211> 21
   <212> DNA
   <213> Artificial Sequence
<220>
   <223> FAM Probe
<400> 355
   ctgcagccag gtcagcccga a 21
<210> 356
   <211> 20
   <212> DNA
   <213> Artificial Sequence
<220>
   <223> TET Probe
<400> 356
   ctgcagccac gtcagcccga 20
<210> 357
   <211> 34
   <212> DNA
   <213> Artificial Sequence
<220>
   <223> FAM Probe
<400> 357
   tgagtaaatt ttttgcattt gccttcctgg tcta 34
<210> 358
   <211> 30
   <212> DNA
   <213> Artificial Sequence
<220>
   <223> TET Probe
<400> 358
   tgagtaaatt ttttgcgttt gccttcctgg 30
<210> 359
   <211> 36
   <212> DNA
   <213> Artificial Sequence
<220>
   <223> FAM Probe
<400> 359
   ttccttcctg tctatggtac tgatagctgt cgactt 36
<210> 360
   <211> 33
   <212> DNA
   <213> Artificial Sequence
<220>
   <223> TET Probe
<400> 360
   tccttcctgt ctctggtact gatagctgtc gac 33
<210> 361
   <211> 27
   <212> DNA
   <213> Artificial Sequence
<220>
   <223> FAM Probe
<400> 361
   aaggcctttt cacgggaact ctccaaa 27
<210> 362
   <211> 32
   <212> DNA
   <213> Artificial Sequence
<220>
   <223> TET Probe
<400> 362
   aaggcctttt catgggaact ctccaaatat tc 32
<210> 363
   <211> 26
   <212> DNA
   <213> Artificial Sequence
<220>
   <223> FAM Probe
<400> 363
   ccaggcaagc actccacaaa gtcctt 26
<210> 364
   <211> 24
   <212> DNA
   <213> Artificial Sequence
<220>
   <223> TET Probe
<400> 364
   caggcaagca ccccacaaag tcct 24
<210> 365
   <211> 36 <212> DNA
   <213> Artificial Sequence
<220>
   <223> FAM Probe
<400> 365
   atgttgatat ctgaatcaat gctatcttac cacatt 36
<210> 366
   <211> 40
   <212> DNA
   <213> Artificial Sequence
<220>
   <223> TET Probe
<400> 366
   tgttgatatc tgaatcaata ctatcttacc acattctaca 40
<210> 367
   <211> 31
   <212> DNA
   <213> Artificial Sequence
<220>
   <223> FAM Probe
<400> 367
   ctcactttgg atgcagagga aatgagactt c 31
<210> 368
   <211> 31
   <212> DNA
   <213> Artificial Sequence
<220>
   <223> TET Probe
<400> 368
   ctcactttgg atgcaaagga aatgagactt c 31
<210> 369
   <211> 30
   <212> DNA
   <213> Artificial Sequence
<220>
   <223> FAM Probe
<400> 369
   caagggacaa cttgggagaa ttttgagagg 30
<210> 370
   <211> 30
   <212> DNA
   <213> Artificial Sequence
<220>
   <223> TET Probe
<400> 370
   aagggacaac ttgggagaat tctgagagga 30
<210> 371
   <211> 32
   <212> DNA
   <213> Artificial Sequence
<220>
   <223> FAM Probe
<400> 371
   ctctgaactg cctcctctca aaattctccc aa 32
<210> 372
   <211> 31
   <212> DNA
   <213> Artificial Sequence
<220>
   <223> TET Probe
<400> 372
   ctctgaactg cctcctctca gaattctccc a 31
<210> 373
   <211> 21
   <212> DNA
   <213> Artificial Sequence
<220>
   <223> FAM Probe
<400> 373
   accctcatgg ccatggaccg g 21
<210> 374
   <211> 22
   <212> DNA
   <213> Artificial Sequence
<220>
   <223> TET Probe
<400> 374
   catggccatg gaccagggat gg 22
<210> 375
   <211> 29
   <212> DNA
   <213> Artificial Sequence
<220>
   <223> FAM Probe
<400> 375
   aggtaggttc gggcgctctt acattgtct 29
<210> 376
   <211> 29
   <212> DNA
   <213> Artificial Sequence
<220>
   <223> TET Probe
<400> 376
   aggttcgagc gctcttacat tgtctccac 29
<210> 377
   <211> 22
   <212> DNA
   <213> Artificial Sequence
<220>
   <223> FAM Probe
<400> 377
   tgagtggtcc gcctgccttt cc 22
<210> 378
   <211> 26
   <212> DNA
   <213> Artificial Sequence
<220>
   <223> TET Probe
<400> 378
   agtgagtggt ccacctgcct ttcctg 26
<210> 379
   <211> 23
   <212> DNA
   <213> Artificial Sequence
<220>
   <223> FAM Probe
<400> 379
   ccctcccggg ttaggctggg tac 23
<210> 380
   <211> 19
   <212> DNA
   <213> Artificial Sequence
<220>
   <223> TET Probe
<400> 380
   cctcccgggt caggctggg 19
<210> 381
   <211> 30
   <212> DNA
   <213> Artificial Sequence
<220>
   <223> FAM Probe
<400> 381
   agcaatggat cctgaagggt taagtccact 30
<210> 382
   <211> 30
   <212> DNA
   <213> Artificial Sequence
<220>
   <223> TET Probe
<400> 382
   agcaatggat cctgaaggga taagtccact 30
<210> 383
   <211> 36
   <212> DNA
   <213> Artificial Sequence
<220>
   <223> FAM Probe
<400> 383
   atctgtaatc accggctatt tctaaagtca gcgtct 36
<210> 384
   <211> 38
   <212> DNA
   <213> Artificial Sequence
<220>
   <223> TET Probe
<400> 384
   atctgtaatc accagctatt tctaaagtca gcgtctca 38
<210> 385
   <211> 33
   <212> DNA
   <213> Artificial Sequence
<220>
   <223> FAM Probe
<400> 385
   cccagaaacc ttagaatcat tttgccaaaa tct 33
<210> 386
   <211> 31
   <212> DNA
   <213> Artificial Sequence
<220>
   <223> TET Probe
<400> 386
   cccagaaacc ttagaatcat ttcgccaaaa t 31
<210> 387
   <211> 30
   <212> DNA
   <213> Artificial Sequence
<220>
   <223> FAM Probe
<400> 387
   ctctcatctg cttgcttgtc tgctgatgaa 30
<210> 388
   <211> 28
   <212> DNA
   <213> Artificial Sequence
<220>
   <223> TET Probe
<400> 388
   tctcatctgc ttgcatgtct gctgatga 28
<210> 389
   <211> 38
   <212> DNA
   <213> Artificial Sequence
<220>
   <223> FAM Probe
<400> 389
   ctccgagctg caactaaatt atctgaggaa aagtatga 38
<210> 390
   <211> 38
   <212> DNA
   <213> Artificial Sequence
<220>
   <223> TET Probe
<400> 390
   ctccgagctg caactaaatt aactgaggaa aagtatga 38
<210> 391
   <211> 39
   <212> DNA
   <213> Artificial Sequence
<220>
   <223> FAM Probe
<400> 391
   aggtaattgt cattattacc acaaaaagga aaactggag 39
<210> 392
   <211> 38
   <212> DNA
   <213> Artificial Sequence
<220>
   <223> TET Probe
<400> 392
   aggtaattgt cattattacc acagaaagga aaactgga 38
<210> 393
   <211> 23
   <212> DNA
   <213> Artificial Sequence
<220>
   <223> FAM Probe
<400> 393
   cacatcggct ggttgggtgt ctg 23
<210> 394
   <211> 22
   <212> DNA
   <213> Artificial Sequence
<220>
   <223> TET Probe
<400> 394
   cacatcggct ggctgggtgt ct 22
<210> 395
   <211> 25
   <212> DNA
   <213> Artificial Sequence
<220>
   <223> FAM Probe
<400> 395
   aggggatgcc actccttgct ggtct 25
<210> 396
   <211> 26
   <212> DNA
   <213> Artificial Sequence
<220>
   <223> TET Probe
<400> 396
   aggggatgcc actccttcct ggtctt 26
<210> 397
   <211> 25
   <212> DNA
   <213> Artificial Sequence
<220>
   <223> FAM Probe
<400> 397
   accaagaccc gaggagaaga agcct 25
<210> 398
   <211> 23
   <212> DNA
   <213> Artificial Sequence
<220>
   <223> TET Probe
<400> 398
   caagacccga gaagaagcct gcg 23
<210> 399
   <211> 27
   <212> DNA
   <213> Artificial Sequence
<220>
   <223> FAM Probe
<400> 399
   atgcgtgtgt cagggatgag ttgaggt 27
<210> 400
   <211> 26
   <212> DNA
   <213> Artificial Sequence
<220>
   <223> TET Probe
<400> 400
   cgtgtgtcag cgatgagttg aggtga 26
<210> 401
   <211> 39
   <212> DNA
   <213> Artificial Sequence
<220>
   <223> FAM Probe
<400> 401
   cccaccaaag aaatcatgta aggtaatttt ttaagtccc 39
<210> 402
   <211> 39
   <212> DNA
   <213> Artificial Sequence
<220>
   <223> TET Probe
<400> 402
   cccaccaaag aaatcatgta agctaatttt ttaagtccc 39
<210> 403
   <211> 20
   <212> DNA
   <213> Artificial Sequence
<220>
   <223> Forward Primer
<400> 403
   cctcacacat gccaattccc 20
<210> 404
   <211> 18
   <212> DNA
   <213> Artificial Sequence
<220>
   <223> Reverse Primer
<400> 404
   ttttgcctcc ccatcgac 18
<210> 405
   <211> 20
   <212> DNA
   <213> Artificial Sequence
<220>
   <223> Forward Primer
<400> 405
   cctcacacat gccaattccc 20
<210> 406
   <211> 18
   <212> DNA
   <213> Artificial Sequence
<220>
   <223> Reverse Primer
<400> 406
   ttttgcctcc ccatcgac 18
<210> 407
   <211> 24
   <212> DNA
   <213> Artificial Sequence
<220>
   <223> Forward Primer
<400> 407
   caaaccacca ctctctcttc attg 24
<210> 408
   <211> 24
   <212> DNA
   <213> Artificial Sequence
<220>
   <223> Reverse Primer
<400> 408
   ctccctcttg tttttctcca ttct 24
<210> 409
   <211> 30
   <212> DNA
   <213> Artificial Sequence
<220>
   <223> Forward Primer
   <400> 409
taaacatttg tggaagaata atgtgtagtt 30
<210> 410
   <211> 30
   <212> DNA
   <213> Artificial Sequence
<220>
   <223> Reverse Primer
<400> 410
   tcaagaagat aaactatatg tggaagacag 30
<210> 411
   <211> 23
   <212> DNA
   <213> Artificial Sequence
<220>
   <223> Forward Primer
<400> 411
   gagctgtgtt tgaagccaat gtc 23
<210> 412
   <211> 22
   <212> DNA
   <213> Artificial Sequence
<220>
   <223> Reverse Primer
<400> 412
   gagcctccct tttcacaagt ca 22
<210> 413
   <211> 24
   <212> DNA
   <213> Artificial Sequence
<220>
   <223> Forward Primer
<400> 413
   cttgtcttcc tgtttggctg ttaa 24
<210> 414
   <211> 23
   <212> DNA
   <213> Artificial Sequence
<220>
   <223> Reverse Primer
<400> 414
   gggaaggtgt agatagggca ctt 23
<210> 415
   <211> 19
   <212> DNA
   <213> Artificial Sequence
<220>
   <223> Forward Primer
<400> 415
   agcaggccaa ctgcttcct 19
<210> 416
   <211> 21
   <212> DNA
   <213> Artificial Sequence
<220>
   <223> Reverse Primer
<400> 416
   actgagcctg gattgcatga t 21
<210> 417
   <211> 20
   <212> DNA
   <213> Artificial Sequence
<220>
   <223> Forward Primer
<400> 417
   cctggccgaa gttattttgt 20
<210> 418
   <211> 20
   <212> DNA
   <213> Artificial Sequence
<220>
   <223> Reverse Primer
<400> 418
   tagaatccct gggtgtcctg 20
<210> 419
   <211> 26
   <212> DNA
   <213> Artificial Sequence
<220>
   <223> Forward Primer
<400> 419
   ggtttctaaa ctccaatgga gctgtt 26
<210> 420
   <211> 25
   <212> DNA
   <213> Artificial Sequence
<220>
   <223> Reverse Primer
<400> 420
   caaatgtcag agaaattgtc cagga 25
<210> 421
   <211> 21
   <212> DNA
   <213> Artificial Sequence
<220>
   <223> Forward Primer
<400> 421
   ggtaagtacg aggtgctggc a 21
<210> 422
   <211> 24
   <212> DNA
   <213> Artificial Sequence
<220>
   <223> Reverse Primer
<400> 422
   ttgctacact cacagtatcc cagg 24
<210> 423
   <211> 23
   <212> DNA
   <213> Artificial Sequence
<220>
   <223> Forward Primer
<400> 423
   gtgacagcat ttttgttgtg cag 23
<210> 424
   <211> 22
   <212> DNA
   <213> Artificial Sequence
<220>
   <223> Reverse Primer
<400> 424
   ggtgtccatc tgatgctcac tg 22
<210> 425
   <211> 20
   <212> DNA
   <213> Artificial Sequence
<220>
   <223> Forward Primer
<400> 425
   cctcagactc ctgaactccg 20
<210> 426
   <211> 19
   <212> DNA
   <213> Artificial Sequence
<220>
   <223> Reverse Primer
<400> 426
   ggtgctatgg tgcagtccc 19
<210> 427
   <211> 21
   <212> DNA
   <213> Artificial Sequence
<220>
   <223> Forward Primer
<400> 427
   cattttgaca ggctggacac c 21
<210> 428
   <211> 24
   <212> DNA
   <213> Artificial Sequence
<220>
   <223> Reverse Primer
<400> 428
   cctcaactgt cacaaagctt acca 24
<210> 429
   <211> 21
   <212> DNA
   <213> Artificial Sequence
<220>
   <223> Forward Primer
<400> 429
   caatggaagc catggagtgt g 21
<210> 430
   <211> 21
   <212> DNA
   <213> Artificial Sequence
<220>
   <223> Reverse Primer
<400> 430
   aacgtggtgc aaccactgat t 21
<210> 431
   <211> 23
   <212> DNA
   <213> Artificial Sequence
<220>
   <223> Forward Primer
<400> 431
   aagccgaact acatcaacgt ttg 23
<210> 432
   <211> 22
   <212> DNA
   <213> Artificial Sequence
<220>
   <223> Reverse Primer
<400> 432
   ctgcaccaga cagattgtag cc 22
<210> 433
   <211> 22
   <212> DNA
   <213> Artificial Sequence
<220>
   <223> Forward Primer
<400> 433
   tgtcctctaa gggcaaaaca gc 22
<210> 434
   <211> 23
   <212> DNA
   <213> Artificial Sequence
<220>
   <223> Reverse Primer
<400> 434
   gtttccccct ttccaagaaa cta 23
<210> 435
   <211> 28
   <212> DNA
   <213> Artificial Sequence
<220>
   <223> Forward Primer
<400> 435
   taggtgtttc cattgattct attctcac 28
<210> 436
   <211> 22
   <212> DNA
   <213> Artificial Sequence
<220>
   <223> Reverse Primer
<400> 436
   tgatagccca tgattcctga gc 22
<210> 437
   <211> 20
   <212> DNA
   <213> Artificial Sequence
<220>
   <223> Primer for rt-PCR and cDNA cloning
<400> 437
   acccttcagt ctaaggggag 20
<210> 438
   <211> 20
   <212> DNA
   <213> Artificial Sequence
<220>
   <223> Primer for rt-PCR and cDNA cloning
<400> 438
   tggtggattt tccaggctac 20
<210> 439
   <211> 20
   <212> DNA
   <213> Artificial Sequence
<220>
   <223> Primer for rt-PCR and cDNA cloning
<400> 439
   ttagagtcat tggccctgct 20
<210> 440
   <211> 20
   <212> DNA
   <213> Artificial Sequence
<220>
   <223> Primer for rt-PCR and cDNA cloning
<400> 440
   cttcagtgcc acatttccct 20
<210> 441
   <211> 20
   <212> DNA
   <213> Artificial Sequence
<220>
   <223> Primer for rt-PCR and cDNA cloning
<400> 441
   cttctcccac tctgacgagg 20
<210> 442
   <211> 20
   <212> DNA
   <213> Artificial Sequence
<220>
   <223> Primer for rt-PCR and cDNA cloning
<400> 442
   cctgcagata ctcagtgcca 20
<210> 443
   <211> 20
   <212> DNA
   <213> Artificial Sequence
<220>
   <223> Primer for rt-PCR and cDNA cloning
<400> 443
   cgaaggtctg gcactgagta 20
<210> 444
   <211> 20
   <212> DNA
   <213> Artificial Sequence
<220>
   <223> Primer for rt-PCR and cDNA cloning
<400> 444
   agtaccgctg cctttttgaa 20
<210> 445
   <211> 20
   <212> DNA
   <213> Artificial Sequence
<220>
   <223> Primer for rt-PCR and cDNA cloning
<400> 445
   ttcaaaaagg cagcggtact 20
<210> 446
   <211> 20
   <212> DNA
   <213> Artificial Sequence
<220>
   <223> Primer for rt-PCR and cDNA cloning
<400> 446
   tcgtctttcc ttccatgtcc 20
<210> 447
   <211> 20
   <212> DNA
   <213> Artificial Sequence
<220>
   <223> Primer for rt-PCR and cDNA cloning
<400> 447
   cggagatgtt tgtgaccctt 20
<210> 448
   <211> 20
   <212> DNA
   <213> Artificial Sequence
<220>
   <223> Primer for rt-PCR and cDNA cloning
<400> 448
   acttgttcca tcagcatccc 20
<210> 449
   <211> 20
   <212> DNA
   <213> Artificial Sequence
<220>
   <223> Primer for rt-PCR and cDNA cloning
<400> 449
   tggatcacac gcttgttctg 20
<210> 450
   <211> 21
   <212> DNA
   <213> Artificial Sequence
<220>
   <223> Primer for rt-PCR and cDNA cloning
<400> 450
   gcccatagag cagatagtca g 21
<210> 451
   <211> 26
   <212> DNA
   <213> Artificial Sequence
<220>
   <223> Primer - full length amplification
<400> 451
   gtctcgagat ggtggatttt ccaggc 26
<210> 452
   <211> 21
   <212> DNA
   <213> Artificial Sequence
<220>
   <223> Primer - full length amplification
<400> 452
   cacccggggc tcctcttcct g 21
<210> 453
   <211> 26
   <212> DNA
   <213> Artificial Sequence
<220>
   <223> Primer for the deletion variant
<400> 453
   gtctcgagat ggtggatttt ccaggc 26
<210> 454
   <211> 21
   <212> DNA
   <213> Artificial Sequence
<220>
   <223> Primer for the deletion variant
<400> 454
   aacccggggt ggggaagaac c 21
<210> 455
   <211> 22374
   <212> DNA
   <213> Homo sapiens
<400> 455
   agcaataaag tagcccacaa gggaccttgg cccagagctc tgatcctgga gagggcagtt 60
   cctttctctg ggtctctgtt ttctcatcag tggaatgagg gatggcacgt ttgatattca 120
   gttttgcttc caactccagc ccgatgagtg ccatgctctg gtggagactt ggcactagtt 180
   gggaatcttt gatgtgtggc aaggtggagg gaagggtgga tagcacccgc tgatgtgtgg 240
   gacggtgggg tctgtgtgag gacctaactg gggggttgca gatgaggcct ccatgaaggc 300
   cttggctttc tggggaaggt gagaattgcc agtgtcatga ttttgtgtgc acgtctgcag 360
   aattcacaac tgaacccagc caaagtccac ttgtccccgg agtttcactg ttgagaggat 420
   tatagctgtt aggaggtgaa tgtgctattc tgattttcag aatgcaatct cattttcaca 480
   gggcctccgg gatttggctc tgccttcaac tggggtgaga tggcttaaat cctccttcac 540
   ccaaagtcct ggaggaggcc tccacactct ctggctctcc cacagttctg tctttgatca 600
   gcacacccca gggctctgac ctgtttctga ccacctgcta tgtggatgaa gatacatctg 660
   gggctaagtg agaagaccca gtagcaccag gtttcgcccc aaaggagaga gggagacacg 720
   tgagtggttt ggattccctg gtgaaggatg gccacgggaa gctggggaca ttgtctcaga 780
   ttacagagta ggacatacgg acagaatcaa tgtcctttag ggaaatgctg ccacctgtta 840
   cactgcctcc cacttacatt tatatcccac tgcgcagtct tcaaagtgcg attgtagccc 900
   atttgaccct cacagtgttc ttgtgaagca ggctgagtga ggaagaagca cgaaattaaa 960
   aacaagaaaa ccaggatcac attttcattt gtcatggatg atgtggggca tgtgtccctg 1020
   agccttgacg tcttcttatg tcacatggtc atgacacagc ttggaaaaca ttaggatgat 1080
   tgtctccacc acacagcaga gaaaatacag gctcaaaaaa gtaaagaaag atgtccaagg 1140
   tcacacacac acacacaaaa acccaattca ctttgtctcc tagaaatctt attttagctt 1200
   tctctataaa aatgtattgt tttaacatat gataaactat agctgttttt ttttttttaa 1260
   tttttttttt tattatactc taagttttag ggtacatgtg cacattgtgc aggttagtta 1320
   catatgtata catgtgccat gctggtgcgc tgcacccact aatgtgtcat ctagcattag 1380
   gtatatctcc caatgctatc cttcccccct cccccgaccc caccacagtc cccagagtgt 1440
   gatattcccc ttcctgtgtc catgtgatct cattgttcaa ttcccaccta tgagtgagaa 1500
   tatgcggtgt ttggtttttt gttcttgcga tagtttactg agaatgatgg tttccaattt 1560
   catccatgtc cctacaaagg atatgaactc atcatttttt atggctgcat agtattccat 1620
   ggtgtatatg tgccacattt tcttaatcca gtctatccct tccttacacc ttatacaaaa 1680
   atcaattcaa gatggattaa agatttaaac gttaaaccta aaaccataaa aaccctagaa 1740
   gaaaacctag gcattaccat tcaggacata ggcgtgggca aggacttcat gtccaaaaca 1800
   ccaaaagcaa tggcaacaaa agacaaaatt gacaaatggg atctaattaa actaaagagc 1860
   ttctgcacag caaaagaaac taccatcaga gtgaacaggc aacctacaac atgggagaaa 1920
   attttcgcaa cctactcatc tgacaaaggg ctaatatcca gaatctacaa tgaactcaaa 1980
   caaatttaca agaaaaaaac aaactatagc tgtttttaat gtctacatag atacataagc 2040
   tttcaacttc gtgaactaag atacataagc tttcaacttc ctgaactaag ttaatttatt 2100
   cttaccagtc agttactatg gctgcttggc attttgagga gcaacatttg aggcttttac 2160
   tgaaaaaagt tctaatgacc catgatacgt agtaatttgc aattttcttt gatatgagtt 2220
   ttagcagtgc aggataggcc ttgaaattca gaattgttca tcatctaatg cctaaacata 2280
   agctaccact tattattaca atttaacact taaactttat ccagaaaatt aaattaaatt 2340
   aaacatgggt ttttgagaat ttgatgatac atttgcatat tagatcatat atcttgacaa 2400
   caccaaaatt tactatattt aataggaaca gcagtttcag aagattcttg cattacatcc 2460
   aaatagtaaa acggaggggt caaaagctta tcattttaaa aggtttttac ttaaagatgg 2520
   caatttaagt ctagttatct attttcaaaa aatgtgccaa agcttttcag catcctgaca 2580
   gcaaaccctg tggcagggca tttcctcggc accaccggcc ttctgggctg gatgactctt 2640
   tgttgtgggg gctgccacgt gcctcataga atgtttagca acacccctgg tttctactca 2700
   atatatgtcc cctcccccag tcgtgacaac tgaaaatgtt cccagacatt gccaaatgtc 2760
   ctctaagggc aaaacagcct ctttgcccat taagaaccct ggtccaaggt ggttatcctc 2820
   cctgatatat tctctcacag tctatctgag cactggagat ggattctccc aacattcttc 2880
   gaaagcttca ttgatttgag caattttttt gagagttcac ttgtagattt tccctcccac 2940
   cgctttgaca tgaaagacct acaggaacgc aagcctctga agtggtctgt agcaagtgtg 3000
   agttcatttt agttcacaag gctgagaaat catgcttggt atggatgttg tggggctttc 3060
   tttactctta cttggtggaa aaaaatgttc ctgcttttca caaaaggagt cagggaggca 3120
   tcagcaacac ccttcagtct aaggggaggt gactggtaag tttcaggtgg caaggtgctc 3180
   atggtctgca ctgtcttcta acacccaacc ccattttcac ataaatatca tactgccctt 3240
   ttcgatctag gtattgtgaa tgggctcata taagtttcct tagagatatc ttatatgaaa 3300
   gtacatattt tttataaagc aaatgggcaa tatttgtgta acaacaatct cctgactcat 3360
   ttgattgtta aatctccatt tcagaatgtt gatttttacc cctgtcccat aaaatcttac 3420
   tagcagtagt ttcttggaaa gggggaaact gtaattgact tgacattgct cctgagctgg 3480
   tttctctact ttgtgtgaag caagttttct ttcaataaaa tacttgacac atcacatata 3540
   ataagtctta gacttctttc actctgaagt ttgtattttt caatggtaaa actagccttc 3600
   tatccttgtc ctggtaagtt ttaaagtttt tagctgtgtg aagtattttt gttcagctgg 3660
   gacagtgggg ttgctgggtt tgccagtttc tgccagttgg gctttttgtt tttttaattc 3720
   attcgtgttt ttaagtctat tctaaaaggc cacttacaga tattttcccg actatctttc 3780
   tactactctt tctttcactt tccacggcat ctctgtgtcc cgggttctct gagcgctagc 3840
   actgaatgtt ggctttctgg ctgcagtgtg gagacgcaga gccttcctgc cctttcctcc 3900
   atgtgtagaa aacagtggta aagaaaggct ttgcaggagg agttgggagg aggacaggaa 3960
   aaaatatttg aatcttgctc tataagtttt gcaagctcac aacagatgaa cctaaggagt 4020
   tttgtttgct ttggtttggt ttggggtttt tttaatatta ttgttccttt acctcaagtg 4080
   ttttttcatt tcagtcacaa gactacctta cacctaaaaa attgcttaaa atattgtctc 4140
   atgaaattag ggttgctgag gcaggaataa tttcataaag ttctattgga aggcaaatgt 4200
   caggactgat gtaggaagac acaccaacga agttgagggt ggtccaaagt ctgttacaag 4260
   ttggaaggct tttgtaagga agttcaggca gaaagaaagg ggctcttcat attggagttg 4320
   tcttttttca ctggagggta caatacagag gttacaatca ttggacacag atgacaatat 4380
   atagactaaa atatttcatg cacaaaacaa tcagtaaaac tttatgattc agaaacaaat 4440
   ccatatcctt ttcgatgtca ggaggttaca tattaaccac tacatcaaat tagctcagaa 4500
   gctacattga agccgaacta catcaacgtt tgggtgtatg agcagatctg gttataggtt 4560
   acagaggtat ccttcaatca gacgttatct tatgtgtagg aaaaggcaag gactattatt 4620
   gcttatcttt taaggagtag agtgactcag gcaagagaga tgggaggccg tgtgctcaat 4680
   cttgttttgt ctttgaagca tctttctgga gaactgcagg tcatcacagc aggggctttg 4740
   tgaaagtatg ctagcaagcc gaaatgagca agcaaggctt cttacatttg ctacattgtc 4800
   tcacaatata acacgttagg taaagggttg gatgtgatca gtaattgcag agaaatggag 4860
   ccaagcccat ttcactgact gtgcaggggt tgatgtggat tctctgctaa gactgcataa 4920
   ccaatggaag ccatggagtg tgccgggtgt gtcacagcct cacttaggca cagcgtttcc 4980
   ttgtctttgt tccagggagg atggtggatt ttccaggcta caatctgtct ggtgcagtcg 5040
   cctccttcct attcatcctg ctgacaatga agcagtcagg taggattccc ttctcccttt 5100
   actgtatagt ctaatgtccc agtgagctag tctgggtcca aaggtcgaga acaacatcta 5160
   agagtgtaag tctggggcca agccacctgt atccaaaaag gaactcctca cttttgagga 5220
   gctcctccac tcccaggagc tcctccactc ctagctgagt cacctttgga aagttacttg 5280
   agcacctcat accttagttc tttcaccttt ttaatgagaa taacagcagt aactacatct 5340
   cagaggccag cacaggcttt aggattaaat gagacattaa aagcacttga atcagtggtt 5400
   gcaccacgtt aaattattaa ttcactctag tactttgggg tcaagaaatg cagctgcttc 5460
   ttatcataaa ttaagctgca attaaaaaaa atacttggaa ataagaaatt agaagaaagg 5520
   gactttggaa atctcactag ctgaattttg acgtcaaaat gttgcacctt gaaaaataat 5580
   tatttaaaaa ttcagtgtca aaaaaaaaaa atcagtgtca gaaggagatg tcagagccct 5640
   aaggaaaatg cagcatggcg aagtgggaaa aaacacgaca cagagcatca gagggcgcag 5700
   gctctgcctc ggcaccgcag agctggtgtg tctgcctcgg tgccgcagag ctggtgtgtc 5760
   tgccttggcg ctgcagagct ggtgtgatgt gatgaagctg ctggcttctc tgtgcttgtg 5820
   ctgcagctcc ctaccctgta acagtcaatc tgatggaatg tcttcagttc tagcttcata 5880
   tttagacttc atcaatgaag aatacaagca gatctccact taggctcttt agccaccttt 5940
   tgagcagcat ctatttgata cccagcagca gctaagtagg gtgaggttct tcctagcagg 6000
   attggatctg cacagagaaa gagcaaatgg ccccggagca tttagaatga gaaagaacat 6060
   atcatacatg acataaaatg atcaaacata ataacataca taactgttaa acatctcttc 6120
   aatttgcaga taacaaggaa gagatcagac atgtcttaga atttgtttgg aagacccaaa 6180
   aatctctaga caaaccagtg tccttaatcc agcacaaaat gtccttgtga aaactgcatt 6240
   gtacatagtc ccagtaattg gcctcctgcc aactccagac gggaaaaaaa caaacaaaaa 6300
   aaaaacaaaa aaaaaaacag tttttatatc taagagaaga ggaatcctga aaggctaaaa 6360
   aaagctaata aatggattta ttatctaaac ttcaggaaaa taaaatgtta ataacaagaa 6420
   taaggtttcc aatttaaaaa gtcaatttac atgagaataa atgactcaag gaataaataa 6480
   tttacttgag aataaaaaca acccaggtat gctggtatta atatcttgaa gatgaagaaa 6540
   ttaggacttc aaatctcaaa atcttaccaa gagtaataga tttgccaccg aatcacatag 6600
   ctggtaggtg acagacagca acctcaccca catttttgaa ctgcatttgt tttcagtaca 6660
   caaaattgac tctgtaacaa acagatgtgc aaatcatcat gctggatttc tctaaaacat 6720
   taaacatata tctctccctc ttctagttgc ctccattttg acaggctgga caccccaaag 6780
   catgcatatg attttctgat tggcatttcc tcgttcctct ttttcactta ttttatagaa 6840
   gactttagag tcattggccc tgctcatcct atcctggccg gggttgggga agatgccctg 6900
   ttaacctgcc agctactccc caagaggacc acaatgcacg tggaggtgag gtggtaccgc 6960
   tcagagccca gcacacctgt gtttgtgcac agggatggag tggaggtgac tgagatgcag 7020
   atggaggagt acagaggctg ggtagagtgg atagagaatg gcattgcaaa gggaaatgtg 7080
   gcactgaaga tacacaacat ccagccctcc gacaatggac aatactggtg ccatttccag 7140
   gatgggaact actgtggaga aacaagcttg ctgctcaaag tagcaggtga atatctgggg 7200
   aaagacacag ggtctcaaga ggcagagata tattaatttg tggtaagctt tgtgacagtt 7260
   gaggaaatcc ttttgaatca tcaaggtaat tcctaatgct tagtcctcta ccctgattga 7320
   tttaaaaata agtggttctg gagtcttcaa gcttagtgtt aaaatatttt cccaaattta 7380
   atttgcacag ttttgactgt tcttgaggat tatttggaat ccatgacatg cagtctttag 7440
   caattttgct gagacacaca tttgtgctac ctaaggctgg tgcgcaagag caaaccaatt 7500
   gctgcatgaa tgagcccagc tgacgtccgg catctgtatc tcagggcatt ggtttttctc 7560
   tacccaatcc catctatgca gaaactgaga cgtgaaaatg ttctttcctt gtgaaatgcc 7620
   ctcagttaga aagctatcaa caagactgaa aaggctaaga atgtcgtgct ttggagattg 7680
   ttatatacta gtgttcaatg aattggggac tcacagatgc ttttggacac accctctgtg 7740
   aatgggaagg ttcttcatat tgctgttgta ttagctaagc aggaaggagt agggaagaag 7800
   gggacaactg aagaatggat ctgtcttatg aaaagtaaac tatcatatga ttccctgaca 7860
   gggctaactt ctccatgctc taaagagaaa atcaagtaaa tccaattact aaggcaaatg 7920
   gggaaaatag ttctctggag atgttaacgt ctccagctga tactgcttcc ttctttactt 7980
   ccctgggagg ctgtttccta ataccttttt ttttcttaac atccattact gttactgtgt 8040
   tcaaggtgat tcaaaatgga tggaggaata catatatttc tgacatgggg ggacagggaa 8100
   cgtactgatg tatttcaata tcaggaacat ttacaaataa gtcacacatt ttctaagggt 8160
   ccaagggaag ggagtggatc tctcttttac cagcaagtga ttaagacaga attcccattt 8220
   atggaaaata tagtcccctt agtttgggtg tgggagtttc acgttaaata gagaaaaaaa 8280
   attcatgatt tcataaactg ttgtaaacta ggatttctca aaaccatctt acccattgaa 8340
   tagaatattt agctacaaat aacgcctcgt ggagattcgc atttcacatg ctcccgttta 8400
   tccacttgct tccgaagatc acagccacca gcaaaagatg caggaagaac aggcccctgg 8460
   ggagtgattt ctgactttgt gttaaatctt caatcaaatt ggagttcaag acatcatcta 8520
   aaataccaga caatgtgccc aatacagata attttaagat gaaaaaggca gagttcatgg 8580
   cacagaggaa ttaatgaata aattacagct catctctgtt tgcagtgcta gaggcgttgc 8640
   aaaatgtcat gatgtccccc ttctccgatt cccctttctg gatctggaga aaagtactgg 8700
   tcagatgcta aaatgtaaag acccagactg gggaacaaat aaagtgtatc tgaactcaca 8760
   tttgaatggg gagactgagg tcgactttct gctcctcaga ctcctgaact ccggccacct 8820
   ccctgtgagc aacgtcactg ggatctcagc ctctgtgatt tctgcctctg tgggctctgg 8880
   gtgtcagagc tgtggcttcc ctctgcaggt ctggggtctg cccctagcat ccacatggag 8940
   ggacctgggg agagtggagt ccagcttgtg tgcactgcaa ggggctggtt cccagagccc 9000
   caggtgtatt gggaagacat ccggggagag aagctgctgg ccgtgtctga gcatcgcatc 9060
   caagataaag atggcctgtt ctatgcggaa gccaccctgg tggtcaggaa cgcctctgca 9120
   gagtctgtgt cctgcttggt ccacaacccc gtcctcactg aggagaaggg gtcggtcatc 9180
   agcctcccag gtcagtgctc tgcctctagg acccacatgc tcagatcagc aggagaggtc 9240
   ccagggactg caccatagca cctcgtattt ttatcaagag acattatcta tcatatagca 9300
   atatgtattg aaagccataa aacatttatg actggaaata tacgcactac attttacttg 9360
   aatagctgtt atggcgcctg cgttattttt gagttctacc tttccttctt ctgcattgat 9420
   tgtttttact cacaagtatt cattgcattg taaagaaaat aaagctatat ttcttcaact 9480
   gaaatgtttt cctatgctgt cttgtgctga ttacttctag aaatctatca tatgaagata 9540
   atcagagatg tagacaacta tttgcataat agaatattaa ttatggcctt atttataaat 9600
   agcagcaaaa acaaccaacc ccccaataca acagtacagt gtggaatctt gtcttactat 9660
   acattatagc attaataaaa agatattttt aaataatttt aatggttgaa aaaaatcctc 9720
   atgatgcact attcaatgat aaacatgaca tctacctata aattgacatt tccaatcaca 9780
   taaaagttac tggccttaaa taacttataa ctgcactaaa actttcagga taccctccac 9840
   atttggatat aagtatacag ttattcatat aaatggatag attaaaaaaa ctttaaagtt 9900
   aatgttacat tttaatttta aatgaagctg attgtatatt acatataatt cgtctctcta 9960
   ctttgtaatt taggtgatac tgtttatgtc catataattt tgtactttct gtgtatctat 10020
   aaaaggaaca tatgttattt tcatgctgat agctaagtat ttatgctgta aatatcagaa 10080
   gaaaccaaca gaatcatggg gagatagaag tgacagcatt tttgttgtgc agagaaaagg 10140
   aaaaaaagct gtatcattgg gtaatctgtg ggaaagaatc agtatttttc tgcatttcat 10200
   ttttttaatg aattgagctt ccctatataa tgtataaatt gctttctttt ttaaaaaata 10260
   gtttctctcc tttcccttac accaaattcc ttaatatgtt attcttgttc tttttccaga 10320
   gaaactccag actgagctgg gtaagtacga ggtgctggca cacacctgtg gagggagcct 10380
   ctgccctccc cagcagaggg aggggagcta ccacgagacc atgtgaccca gaggaaaaac 10440
   tgaggcacga ttttaccagg tcctatgttc attaaatacc agaggagacc agcaaagtaa 10500
   tggcctcatt caaggaggtt gcagctgtgt tacctgacac tttgggggct catggagatt 10560
   ccagtgagca tcagatggac acctatagga agcatggcat catctctgtt ctatgtgttg 10620
   aggggaaagg gagctgcctc agaatgtgtg ggggatgaca gcagacagct ggccggggcc 10680
   tggaggccct ttgaaaacat ttcaagtgtg aacaagggca gcatcattat gacaacctgg 10740
   gttgcaccca gcacctccct gctcaactct gctatggggt cctgcacctg ctcctcaccc 10800
   cagtacaccc ctaagctaat cactattggg agggagctgt tactttctga gtgcaacctg 10860
   ggatactgtg agtgtagcaa tttttttttt tttttttttt gagacggagt cttgctctgt 10920
   cgcccaggct ggagtgcagt ggcgcgatct tggctcactg caagctccgc ctcccaggtt 10980
   caggccattc tcctgcctca gcctcccaag tagctgggac tacaggcaac cgccaccacg 11040
   cccggctaat tttttgtatt tttagtagag acgagatttc accgtgttag ccaggatggt 11100
   ctcgatctcc tgatctcgtg atccccccac ctcagcctcc caaagtgctg ggattacagg 11160
   cgtgagccac cacgccgggc attttttttt tttttttgag acggagtctc gctctgttgc 11220
   ccaagctgga gtgcaatggc gccatcttgg ctcactgcaa cctccatctc ctggattcaa 11280
   gctattctcc tgcctcagcc tcctgagtaa ctgggactac aggcatgcgc caccacaccc 11340
   agctaatttt tttttttttt ttttagtaga gacggggttt caccatgttg gccaggatgg 11400
   tctcgatctc ctgacctcat gatctgccgg ccttggcctc ccaaaggtgc tgggattaca 11460
   ggcatgagcc actgcaccca gcctaatttt tgttttttaa atctcggagt tggactgagg 11520
   gtattatttt gctgcacctg agataagtgg tttaaaacat aaccagccga tggctgagcc 11580
   cctttgagtt tcccctttac ctccttacta gctccctcct ttttatggcc tcttctccca 11640
   gcccaaggca accagcctgc ctccctgcct aggtgcatag aacgtggatc ccttatgtgc 11700
   tctttccatc aattccttgc tctccatcaa gtcacctgct ccaggaatgt gtatccatgt 11760
   gtgtgcttta ggtgagctta agcacacctt tcagcccaca aaagagtttt tgaggaaaat 11820
   cagggtaaca aacacaaccc aatctctacc agagtctact atggcacctt caaccccatt 11880
   aggccataaa cataaaggga agggtccctg gaaggcgcaa agtaactcca caatctgagg 11940
   agagacacac acctgtgtat cttacccttc tgagatcagg aagtaaagcc agtgtttggg 12000
   gcaggttgac acaagtctgt ctctcaagaa ggttttagga gaagagtcac tcctctgagt 12060
   tctctgagac ccactggaga cccagcacac acaatacgac cccatgtcct gttgttccta 12120
   agtcatggta acctccgggg ccacaattaa ccaacacgga aagatggagc ttccctttgc 12180
   ttcctgtcag gtgggagagg agggctctgg gcacggaagc tttgcaggtg agctctaatg 12240
   atgggacacc cagctctcta tggaagtcct aagactgaaa tcactgcttg tagtcttaca 12300
   aataatgttt ttcatttata aaaataatag tttttaaaaa tattaattgt atgccaggca 12360
   cgatgctgaa tatgttatgg gaattatttc attttttctt acaataatta taaagtttaa 12420
   ttgctttatc ctcatattac agctaagaaa caatcttaga gacatacatg gtttgccaaa 12480
   aatcaggaag aaatggtgat gtttaccttc taatctacat ctatctaact taacagtgca 12540
   agtccttaat tacttatgtg tgactttgtc agctcattca tctctatttt tctcatcttt 12600
   aaagtgaaca ggttgaattt aatctctaat gtagtgtcca gttctaagag attagatata 12660
   acttagcata tttgagaaat gagatagtgc attaattgaa tatgctgatt gaaagaggat 12720
   tatgtggtat gatgatgaga ttatcaggtt tcaaggaatc agtgtataat tcagtgtact 12780
   tcacgctcca gaagggaatt aatttttcag tggatattca gaaggcattc agaacataca 12840
   aacaataacc aaaacaaaac aaagcaatat atttcagcac aaaattgtct ggaaggctgt 12900
   gtacaaaacc gtatagaaaa aggaggttaa ctactgcatg ttcacactta taagggggag 12960
   ctgattggtg agaacacatg gatgcatggc agggaacaac acacactgga cacctgttgg 13020
   gggatggggg agggagagca tcaggaagaa cagctaatgg atgctgggct taacacctgg 13080
   gtgatgggat gacctgtgca gtaagccacc atggcacatg tttacctatg taacaaacct 13140
   gcacatcctg cacatgtacc ccggaactta aaattgaaag aaaaaaaaag tgggttaggc 13200
   atagtggctc ccaaggagct tgtattctag tagaacatca attattaaca aacagggctg 13260
   catgattttt ttagcacttt aaactttagt tttacttgga ttctggacaa aaggagtttt 13320
   gtctgtatct gtaatgcatc aaattaattg aatctccata cttacatttt gcttccttca 13380
   gtctccattg aagtcaattt tagctcttaa ttacctatgt caggcagggc aagccagttt 13440
   aagatctaat atgtgcacag gtcacacacg gacagggaat aaaatcctca tatggataaa 13500
   ggagatgagg aaacagtctg ttttattaag taatttaccg taagccatgt ggatgcagaa 13560
   acagccccaa actaatccct agtccatttt ttctgattct tgtcttcaga ctttaataga 13620
   tacataggag aggaaaataa tattgtttta ggtaaaatta caggacaaag caaacaaaat 13680
   gctggaaata actgggagtc aactgttacc tggaatggaa ggtaataatc tgaaaattca 13740
   aattgtgttg acagggactt tcctctctac aaaggtagct ggcagttttc ggccaatgac 13800
   ataatgggat gttaacgcct ttggccagtg actctatgca gagaaccaac aattttatgc 13860
   ttgtgtttct attataaatg atcacacctt atgcaaaacc attcttatca tttaacagaa 13920
   aaggacgaca cttaatttag aatagagaaa aaatggcacg ggcagggcct gatgagaatg 13980
   gaaaatgtaa tatagcttgg gcccaggaaa gccctcagag aacatctttc cccactttgc 14040
   acagtgggag aattagaccc cttttttaca ttcatcattg tttcacaatt tgtactttgc 14100
   cgacttcccc aattattgat cttaaaatgt taattttaat tttcaaaatt atttgaggac 14160
   attcaactgg tgtgtgtcta taactccacc tatgtggagc attggggaaa taaaatatag 14220
   cggctggacc agacttacaa ggtactataa agtccaccta gaggaaagct tcttcaaata 14280
   tcattaccaa aagtccacat gagacacgtt tttaaacccc tccctattac cacgtcagag 14340
   ggtggggatg gctccaacgc cttcaagtct cacaggtctg tgcacctctg ctcacgctgc 14400
   agggtcttcc aggcacttaa tgcaaactat tttgttcaac tctgaaacat tatgattttg 14460
   gtagttgtat agcaaattta cagtttaaga aactgaggca ttaagaagta gtttaagaca 14520
   gattaagaag tagtttaaaa tcatacagaa ttgaagtgtt agaagaaaaa ctttagacag 14580
   actaaattta gcagagtcta actgagaaaa ggatgactca taaacaagcc atccctcaga 14640
   atcagaacgg tttcagagaa ttcaaagcag caatttggtc agacagcatt tatggacaga 14700
   aaatggaaat gaggtacaga aaacagaagt gaggtacaga gacagctcta ttggttgcag 14760
   cctggcgttt gccgatttga acagggtttc aacagttggc tgcctgcaac taacagaagt 14820
   gcagcagctg ctactcagct atttattaca aaagtgtact cctaagttag ccttagctcc 14880
   taagttaggc ttattcataa aataattttg caattaaaac tctcagaata aaatgagcaa 14940
   tttataaatt tggccccagg atgcctctgt acctgagtgc ttatgtaaca aactgcaacc 15000
   taagttaggc tttcagtagt tgacgtacta agttaaattg cagtttgtta cataaggagt 15060
   caggtacaga ggcatctggg gccaaattta taaactgttc actttattct gggtgttttt 15120
   tgtttgtttg tttttaaagt ctcgctttgt cacccagcct ggagtgcagt ggcatgatct 15180
   cagctcactg caacctctgc ctcccgggtt cgagcaattc ttccacctca gcctcctgag 15240
   tagctgggat tacaggcacc caccatcatg cccgactaat ttttgtattt ctgtagagac 15300
   ggggtttcac catgttggcc aggctggtct tgaactcctg acctctggtg atccacccgc 15360
   ttcggcctcc caaagtgctg ggattataga cttgagccac cacacccagc ctatctggga 15420
   gttttaattg caaaattatt ttattattgg tctgtaaact gaaaaaaata caggtagaag 15480
   caggtaggga ttcaattact gttttactca gactgacaca catcatggaa tgattcctcc 15540
   ttaaagaaga gcaacctcta ttctgagtcc atgaaacccc cacctcacct aaggatgacc 15600
   ctgagcctca cagcaaccct gggatgctca tggtttctaa actccaatgg agctgttctt 15660
   agagatgaca ttattggttc cattttgatt ctttcattgt gttttgtcca ctctatttaa 15720
   cagcttcttt aaaagtgaat ggaccttccc agcccatcct cgtcagagtg ggagaagata 15780
   tacagctaac ctgttacctg tcccccaagg cgaatgcaca gagcatggag gtgaggtggg 15840
   accgatccca ccgttaccct gctgtgcatg tgtatatgga tggggaccat gtggctggag 15900
   agcagatggc agagtacaga gggaggactg tactggtgag tgacgccatt gacgagggca 15960
   gactgaccct gcagatactc agtgccagac cttcggacga cgggcagtac cgctgccttt 16020
   ttgaaaaaga tgatgtctac caggaggcca gtttggatct gaaggtggta agtaagaatt 16080
   ctagatagat attttgtatt caatacctgc tcctgccact ttaatatgct agttcctgga 16140
   caatttctct gacatttgaa attttcttaa tgcttattca tcatcaaatt tatatcaaat 16200
   cctccagtgt gtgaaactgt catagttact taaaatccac aaactcaaat ggatgaaatt 16260
   taattatttg ggcatataac ttgtatcttt tccagtatct tcaaaaattg accatatctt 16320
   taaggaaatc tgagtatctt actacatatt ttacaaaagg ctcattgttt cacagtgtgt 16380
   tattagaatt ctgagaaatg caataaaatt ctagaaactg taatcatctg aagactaaac 16440
   tggatccact gtgtaaacca aaattataaa tattgacagt tttggcaggg acaggggaga 16500
   aaagatgaaa tcactccctg cagcatcatt tcccactgtc cgtgtgtgtg agaattgaaa 16560
   cgtccaaggt tagcagatgc cattgagtac atataggccc aagtcttgga tatgctacgt 16620
   gtttctccca cggattttga ttctaaatta ataaacacgt ttagccatgg gggcgcatgt 16680
   tgagaagttt catgagcctc gtattttctg ccattggacg tagatattta taaggtttcg 16740
   atttttgaga atatcctcct actccgtatg ttaatcctgt ccatgtgacc catgccctgg 16800
   ccgaagttat tttgttctca gggacagaat aaatgttgga tttgaacatt taccttacct 16860
   cttaatcatc ccagaatgat ttaggaatac aaagaataaa aataagcaaa caaacaaaat 16920
   aagaaaatgc tccagccagc ctcatcccta agtggagtgc aatccccctt aagcttttct 16980
   cccttcccca agccctccac atccccttct ttcctcccag tgaaattgct gcccactcat 17040
   ccacttgaaa ggaccccagg ctttggttgc tttctctgcc ccaggtctgg gttcttcccc 17100
   actgatcact gtggaggggc aagaagatgg agaaatgcag ccgatgtgct cttcagatgg 17160
   gtggttccca cagccccacg tgccatggag ggacatggaa ggaaagacga taccatcatc 17220
   ttcccaggcc ctgactcaag gcagccacgg gctgttccac gtgcagacat tgctaagggt 17280
   cacaaacatc tccgctgtgg acgtcacttg ttccatcagc atcccctttt tgggcgagga 17340
   gaaaatcgca actttttctc tctcaggttg gtgattcctt atgttccttc aggtttggaa 17400
   aataaatatg aagaccaact cagacttact cattgtattg ctcttcttcc tttgtgattt 17460
   ttatttgctg ctctctgacg aggtctcctg ctatcctctg caaagctggt tttcaggaca 17520
   cccagggatt ctactcctct gagtttctct ctgacaccct caactagcgc cctgcaatta 17580
   tttttatcat tggatacaca ctaaattctc gtaattgacc ctgttttcac tctctctctt 17640
   taaattctct tcttctgatg tgaaaacaat taaattacag aactttgtgt tttccccctc 17700
   atggaggatt ttcttctgga ttagaaactg atttttatta ataccatctt catggttgcg 17760
   gcttgagtaa ttttatcctt tcctcctcaa gtcattccag tgattgtgtg atatagttgg 17820
   aagagagaag ggaaaacttg tctgtgcatg tgtacatttc tgagacgagc aaacatgaaa 17880
   tacttgcatt ctctcctctg catctccaga gcagggagga gcaggccaac tgcttcctgg 17940
   gaggccaaat tctctccctt ctctccagcc tagaagctgc tcagatgaaa agtacaatct 18000
   taatattgtc ccagtttcac agtttcaagg gattcttggg tttccatttt agagtccagg 18060
   atgacatttt tgtggaaaac actgcttgtt tggggattgc ttcttgctgt ggctgtaggc 18120
   ctgcccagga agaggagctg aaaagaggta agtcagggaa acagaaacag cggagcacac 18180
   tgcagtggga accgtactgc gtacttcccc gagcctccct tttcacaagt cacctatggg 18240
   aggcagtcag tgaatatcca gtaactaagg aaacatgaga aacaggaaag cccagggctt 18300
   atcatgcaat ccaggctcag tgatgcatga ggtcagtgtt tccacttgta atagaagcaa 18360
   aatgattaaa agataatgat ttgatttcct gctgcccatt ccccagtgat ctggttaggg 18420
   aagaagaaga aaaaagtgag tagtacagcc atccttgttt tcaaggctga gagctgaggt 18480
   tcactgagat ttgttggcgt gaacaatttc ctatgatctt taaaaatgtg gtgacctact 18540
   tgttggtgat ttctgtgttg ctggtgactt ctcatgtgtt agggaggctg ttttgtgtgc 18600
   tctagaaagt gcttccactt taaatttctt atgtccgtga aacacttgtc ttcctgtttg 18660
   gctgttaata gaatgaaatt atattgatta attatcagaa tggattctct ttctttacag 18720
   tgaatgtgac attggcttca aacacagctc acctgagact gatttcttct gaacagaaca 18780
   agcgtgtgat ccatggacat tcaggcagcc agatatccca cagagatctg actatctgct 18840
   ctatgggcca gagggaactc ctgtcaggga gctggtactg agaggtggag actgggaaca 18900
   gggcgcgatg ggtcccggag gttgccaagc acatgttgca aagaaagagt catctctgtg 18960
   tcacctgaca gtgagtttta ggcaatggat tgcaaaagaa atgagttctg ggccatcccc 19020
   tctcttccaa gccccatccc cttgaaggtg gcccctgaac aagtcagaat ccccctgacc 19080
   gtgcctctga atacctctcc tcttataaca tgattggcaa gtgccctatc tacaccttcc 19140
   ccaaaacttc cttctctggg actctgcacc ccgcttcatt ctttggttcc ctaatgtagg 19200
   ttctctggac attttttctg tacccacaag aacatctcca acgtatcagg aaaataacaa 19260
   tctgggattc tggaaaccag aatgatattc tgagtcctga tcatttctca tttctctaac 19320
   tgcttgaagg gggctgccat gatcattgaa gtactaactc aaggtgtttt aattctactc 19380
   aggaaaatct gctggagact gatcttttca gagacctatc ctgttccagg ttctctgaga 19440
   tccggcctat caatcacaat ctgacatctg ataatcaagg acccttagaa aatcccatgg 19500
   gaaatacaat ttagcctaca ggaagaaatg aaaggaataa tcaaggattt ggtcagtttc 19560
   tttttttttt tttttctttt cttttttttt ttttttttct gagacaaagt ctcactctgt 19620
   cacccaggct ggagtgcagt ggcatgatct cagctgactg caacctctgc ctcccgggtt 19680
   caagcaattc tcttgcctca gtctcatgag tagctgggat tacaggcatg tgccaccatg 19740
   cccagcctat ttttgtattt ttagtagaga tggggtttca ctttgttggc caggctggtc 19800
   tcaaacttct ggcctcaaat gatccactcg cctcggcctc ccaaagtgct ggaattacag 19860
   gcatgagcca ccacacctgg cctgatcagt ttatttaaga tggtgacaat taattgtcca 19920
   cataggtgga actgagaaaa taaaaaatat atatatacag ttaaaaaaaa gtgaaagaaa 19980
   atacagctgc ataaggggga gaaaaatctg atcactaaaa tataatcttg aaaatcttat 20040
   gaagtgttgt aaaatccctc ctccagactt tttgagaaag ttgatacagg aaattgagtc 20100
   aggaaaaaaa ttttagactt tccgatccaa gcgaaaaatt atgtatcatc tatacgaaca 20160
   tgtcaatttt attgttagag caaaataaag tgcacaattt aaaattaata attataaaat 20220
   aaacaaatat aagcagtaca tgtaacacag taaaaatatt ttaaagttat aatgagttca 20280
   agaagataaa ctatatgtgg aagacagaag aatgtcaaca agacttttgg aaagaatgtt 20340
   atgaagtgag aagacgagga cctgctagtg acgctctgct gctctccagg atggcagacg 20400
   tcagaggcat cgcggtccca tgcggatcac tcatgaacag cactctgaac ttgctcttct 20460
   gggccatcat ttttttttct atttttaaac tcataaattt aaacacataa atgttagaaa 20520
   ctacacatta ttcttccaca aatgtttaca cattaatttt aataaacatt tttttcaatt 20580
   gtggtaaaat acataacata aaataaaacc ctttaaccat tttaaagtgt acagttctgt 20640
   ggtattgtat atgtgacatt gctgtgtgac catcatcatc actttctagt tccagaatat 20700
   tttcatcgcc ctaaacgaaa cttcataccc attaagcagt tactacccat ttttcacccc 20760
   ctactcccca ggtaatctac tttctgtccc tatgaacttt tctattctga atattttata 20820
   taaatggaac cataaactat ctgtgtatgt gtatgtgtat gtggcttctt tcccttagcc 20880
   taatattttt agagttcttc catgtcatag catgtatcag tgcttctttc ctttttaaaa 20940
   aaaaaattaa cagacattat ttttaagaac agttttagat ttataacaaa ataaaatgga 21000
   aaatccagag agctcctaga tccctcctct ctccccccta tttctgctat tattaacatc 21060
   ttacattagt gtggcacatt tgttacaacc catgaaagaa tagtgacaca ttattttcaa 21120
   ctaaagtcca cagttttaca ttaggttcac cttttgttta tataatctct ggagtttaac 21180
   aaatgatttc tacatttaca ttgatctatc tttatattta tgaagactta aatttattag 21240
   taaatatact aggtttacct gaaaatattt taacactatt ttgatttaat gtaattaaaa 21300
   ataatttatt gcacaacatc taaaggttaa ctattatttt tcacttactt ttgagtcttc 21360
   cttcctagtg aatgtagctc tttgcttgtt agtctgtgct tgtgaaggcc ctatgaatac 21420
   atatattcac tttccaggga gaaagtctca agaaaattgg ttttatcagt cacattctct 21480
   cactgtatat tagaaaacat actttcctat agcttcccaa attggtgatt aatgtacttt 21540
   ctgagttcca gttccagtta gaaaagccac aggaaggaga ggggttggtg gcgttgagga 21600
   tgatgcccta gtcactggaa gtccctgtgg ctgttgggtg ggtaatcggg ttggtgcctc 21660
   aggatacagt tccctccgct cctggtgtgt agacagctgg gtgtagtcat taaagtgggg 21720
   ccactctcag aataaatgtg cagattggct ggatctcagt ttcagggaga ggacaggagg 21780
   aagccacaga gactgaattg ttaactccct gacattttcc tcctttgtca gtacaattta 21840
   acttccatga ggacagtgat ttttttcctt cttgtctagc cccacaacct gtaaaaatga 21900
   catacattga ttatcaataa acatttgtaa aatgcatgaa ttgtctcata aagtgatatt 21960
   tattgaatat cttacctgtt gaatgtacta tgctaaggcc tagagataca atccttaata 22020
   agacataatt cctgccctca ggtctttctt ttttgcagtg gagtcactct gtttttggaa 22080
   tgtcttatta taccttcata aactttacta aaaatgggag tgccagagta gtccagcaat 22140
   aaagaattaa ttgatgtagc caggtggtga attcactttt ccagaaagga taggctactt 22200
   ggagaccctg cactacttat tgatttactc aaagctacaa atgaaaataa gatggacact 22260
   gttatccctg acaggtctaa agctctgagt ccaatcaacc atccaccttc acgttttcta 22320
   ccatgtccac ctgcagccac tgctccccaa tctctaagct tagaaggaca aaca 22374
<210> 456
   <211> 388
   <212> PRT
   <213> Homo sapiens
<400> 456
   Met Val Asp Phe Pro Gly Tyr Asn Leu Ser Gly Ala Val Ala Ser Phe
   1               5                   10                  15
   Leu Phe Ile Leu Leu Thr Met Lys Gln Ser Glu Asp Phe Arg Val Ile
               20                  25                  30
   Gly Pro Ala His Pro Ile Leu Ala Gly Val Gly Glu Asp Ala Leu Leu
           35                  40                  45
   Thr Cys Gln Leu Leu Pro Lys Arg Thr Thr Met His Val Glu Val Arg
       50                  55                  60
   Trp Tyr Arg Ser Glu Pro Ser Thr Pro Val Phe Val His Arg Asp Gly
   65                  70                  75                  80
   Val Glu Val Thr Glu Met Gln Met Glu Glu Tyr Arg Gly Trp Val Glu
                   85                  90                  95
   Trp Ile Glu Asn Gly Ile Ala Lys Gly Asn Val Ala Leu Lys Ile His
               100                 105                 110
   Asn Ile Gln Pro Ser Asp Asn Gly Gln Tyr Trp Cys His Phe Gln Asp
           115                 120                 125
   Gly Asn Tyr Cys Gly Glu Thr Ser Leu Leu Leu Lys Val Ala Glu Lys
       130                 135                 140
   Leu Gln Thr Glu Leu Ala Ser Leu Lys Val Asn Gly Pro Ser Gln Pro
   145                 150                 155                 160
   Ile Leu Val Arg Val Gly Glu Asp Ile Gln Leu Thr Cys Tyr Leu Ser
                   165                 170                 175
   Pro Lys Ala Asn Ala Gln Ser Met Glu Val Arg Trp Asp Arg Ser His
               180                 185                 190
   Arg Tyr Pro Ala Val His Val Tyr Met Asp Gly Asp His Val Ala Gly
           195                 200                 205
   Glu Gln Met Ala Glu Tyr Arg Gly Arg Thr Val Leu Val Ser Asp Ala
       210                 215                 220
   Ile Asp Glu Gly Arg Leu Thr Leu Gln Ile Leu Ser Ala Arg Pro Ser
   225                 230                 235                 240
   Asp Asp Gly Gln Tyr Arg Cys Leu Phe Glu Lys Asp Asp Val Tyr Gln
                   245                 250                 255
   Glu Ala Ser Leu Asp Leu Lys Val Val Gly Leu Gly Ser Ser Pro Leu
   260 265 270
   Ile Thr Val Glu Gly Gln Glu Asp Gly Glu Met Gln Pro Met Cys Ser
   275 280 285
   Ser Asp Gly Trp Phe Pro Gln Pro His Val Pro Trp Arg Asp Met Glu
   290 295 300
   Gly Lys Thr Ile Pro Ser Ser Ser Gln Ala Leu Thr Gln Gly Ser His
   305 310 315 320
   Gly Leu Phe His Val Gln Thr Leu Leu Arg Val Thr Asn Ile Ser Ala
   325 330 335
   Val Asp Val Thr Cys Ser Ile Ser Ile Pro Phe Leu Gly Glu Glu Lys
   340 345 350
   Ile Ala Thr Phe Ser Leu Ser Glu Ser Arg Met Thr Phe Leu Trp Lys
   355 360 365
   Thr Leu Leu Val Trp Gly Leu Leu Leu Ala Val Ala Val Gly Leu Pro
   370 375 380
   Arg Lys Arg Ser
   385
<210> 457
   <211> 388
   <212> PRT
   <213> Homo sapiens
<400> 457
   Met Val Asp Phe Pro Gly Tyr Asn Leu Ser Gly Ala Val Ala Ser Phe
   1 5 10 15
   Leu Phe Ile Leu Leu Thr Met Lys Gln Ser Glu Asp Phe Arg Val Ile
   20 25 30
   Gly Pro Ala His Pro Ile Leu Ala Gly Val Gly Glu Asp Ala Leu Leu
   35 40 45
   Thr Cys Gln Leu Leu Pro Lys Arg Thr Thr Met His Val Glu Val Arg
   50 55 60
   Trp Tyr Arg Ser Glu Pro Ser Thr Pro Val Phe Val His Arg Asp Gly
   65 70 75 80
   Val Glu Val Thr Glu Met Gln Met Glu Glu Tyr Arg Gly Trp Val Glu
   85 90 95
   Trp Ile Glu Asn Gly Ile Ala Lys Gly Asn Val Ala Leu Lys Ile His
   100 105 110
   Asn Ile Gln Pro Ser Asp Asn Gly Gln Tyr Trp Cys His Phe Gln Asp
   115 120 125
   Gly Asn Tyr Cys Gly Glu Thr Ser Leu Leu Leu Lys Val Ala Glu Lys
   130 135 140
   Leu Gln Thr Glu Leu Ala Ser Leu Lys Val Asn Gly Pro Ser Gln Pro
   145 150 155 160
   Ile Leu Val Arg Val Gly Glu Asp Ile Gln Leu Thr Cys Tyr Leu Ser
   165 170 175
   Pro Lys Ala Asn Ala Gln Ser Met Glu Val Arg Trp Asp Arg Ser His
   180 185 190
   Arg Tyr Pro Ala Val His Val Tyr Met Asp Gly Asp His Val Ala Gly
   195 200 205
   Glu Gln Met Ala Glu Tyr Arg Gly Arg Thr Val Leu Val Ser Asp Ala
   210 215 220
   Ile Asp Glu Gly Arg Leu Thr Leu Gln Ile Leu Ser Ala Arg Pro Ser
   225 230 235 240
   Asp Asp Gly Gln Tyr Arg Cys Leu Phe Glu Lys Asp Asp Val Tyr Gln
   245 250 255
   Glu Ala Ser Leu Asp Leu Lys Val Val Gly Leu Gly Ser Ser Pro Leu
   260 265 270
   Ile Thr Val Glu Gly Gln Glu Asp Gly Glu Met Gln Pro Ile Cys Ser
   275 280 285
   Ser Asp Gly Trp Phe Pro Gln Pro His Val Pro Trp Arg Asp Met Glu
   290 295 300
   Gly Lys Thr Ile Pro Ser Ser Ser Gln Ala Leu Thr Gln Gly Ser His
   305 310 315 320
   Gly Leu Phe His Val Gln Thr Leu Leu Arg Val Thr Asn Ile Ser Ala
   325 330 335
   Val Asp Val Thr Cys Ser Ile Ser Ile Pro Phe Leu Gly Glu Glu Lys
   340 345 350
   Ile Ala Thr Phe Ser Leu Ser Glu Ser Arg Met Thr Phe Leu Trp Lys
   355 360 365
   Thr Leu Leu Val Trp Gly Leu Leu Leu Ala Val Ala Val Gly Leu Pro
   370 375 380
   Arg Lys Arg Ser
   385
<210> 458
   <211> 388
   <212> PRT
   <213> Homo sapiens
<400> 458
   Met Val Asp Phe Pro Gly Tyr Asn Leu Ser Gly Ala Val Ala Ser Phe
   1 5 10 15
   Leu Phe Ile Leu Leu Thr Met Lys Gln Ser Glu Asp Phe Arg Val Ile
   20 25 30
   Gly Pro Ala His Pro Ile Leu Ala Gly Val Gly Glu Asp Ala Leu Leu
   35 40 45
   Thr Cys Gln Leu Leu Pro Lys Arg Thr Thr Met His Val Glu Val Arg
   50 55 60
   Trp Tyr Arg Ser Glu Pro Ser Thr Pro Val Phe Val His Arg Asp Gly
   65 70 75 80
   Val Glu Val Thr Glu Met Gln Met Glu Glu Tyr Arg Gly Trp Val Glu
   85 90 95
   Trp Ile Glu Asn Gly Ile Ala Lys Gly Asn Val Ala Leu Lys Ile His
   100 105 110
   Asn Ile Gln Pro Ser Asp Asn Gly Gln Tyr Trp Cys His Phe Gln Asp
   115 120 125
   Gly Asn Tyr Cys Gly Glu Thr Ser Leu Leu Leu Lys Val Ala Glu Lys
   130 135 140
   Leu Gln Thr Glu Leu Ala Ser Leu Lys Val Asn Gly Pro Ser Gln Pro
   145 150 155 160
   Ile Leu Val Arg Val Gly Glu Asp Ile Gln Leu Thr Cys Tyr Leu Ser
   165 170 175
   Pro Lys Ala Asn Ala Gln Ser Met Glu Val Arg Trp Asp Arg Ser His
   180 185 190
   Arg Tyr Pro Ala Val His Val Tyr Met Asp Gly Asp His Val Ala Gly
   195 200 205
   Glu Gln Met Ala Glu Tyr Arg Gly Arg Thr Val Leu Val Ser Asp Ala
   210 215 220
   Ile Asp Glu Gly Arg Leu Thr Leu Gln Ile Leu Ser Ala Arg Pro Ser
   225 230 235 240
   Asp Asp Gly Gln Tyr Arg Cys Leu Phe Glu Lys Asp Asp Val Tyr Gln
   245 250 255
   Glu Ala Ser Leu Asp Leu Lys Val Val Gly Leu Gly Ser Ser Pro Leu
   260 265 270
   Ile Thr Val Glu Gly Gln Glu Asp Gly Glu Met Gln Leu Met Cys Ser
   275 280 285
   Ser Asp Gly Trp Phe Pro Gln Pro His Val Gln Trp Arg Asp Met Glu
   290 295 300
   Gly Lys Thr Ile Pro Ser Ser Ser Gln Ala Leu Thr Gln Gly Ser His
   305 310 315 320
   Gly Leu Phe His Val Gln Thr Leu Leu Arg Val Thr Asn Ile Ser Ala
   325 330 335
   Val Asp Val Thr Cys Ser Ile Ser Ile Pro Phe Leu Gly Glu Glu Lys
   340 345 350
   Ile Ala Thr Phe Ser Leu Ser Glu Ser Arg Met Thr Phe Leu Trp Lys
   355 360 365
   Thr Leu Leu Val Trp Gly Leu Leu Leu Ala Val Ala Val Gly Leu Pro
       370                 375                 380
   Arg Lys Arg Ser
   385

## Description

### FIELD OF THE INVENTION

The invention relates to a gene which has been associated with sarcoidosis where the gene is found to be mutated. More specifically, the invention relates to the coding sequence of the BTNL2 gene of human subjects.

### BACKGROUND OF THE INVENTION

Sarcoidosis is a multi-systemic immune disorder, characterized by non-caseating granulomas and an exaggerated cellular immune response due to increased inflammatory activity of macrophages and CD4 helper T cells (Newman et al. 1997, Ziegenhagen et al. 2003). Typical sites of disease manifestation include the lung, lymph nodes, eyes and skin. The clinically presentation of the disease varies between smouldering inflammation, with slowly progressing pulmonary fibrosis, and an acute inflammatory syndrome (Löfgren Syndrome). Although spontaneous resolution is observed in more than 50% of cases, the disease can also take a recalcitrant course with ultimate chronic respiratory failure. The prevalence of sarcoidosis ranges from 10-14 per 100,000 in central Europe and among US Caucasians to 64 per 100,000 in Sweden. Familial clustering of the disease has been observed in several populations although estimates of the relative risk to first degree relatives vary between 2.8 and 18 (Rybicki et al. 2001).

In a recent genome screen of 63 sarcoidosis families (Schurmann et al. 2001), the inventors of the present invention identified linkage of the disease to chromosome 6p21. Several HLA markers have since been investigated for disease association (Sato et al. 2002, Foley et al. 2001, Rybicki et al. 2003, Rossman et al. 2003) and, following conflicting initial results, the *DRB1* gene has recently been identified as a risk factor for sarcoidosis (Foley et al. 2001, Rossman et al. 2003). Nevertheless, the population attributable risk of mutations in this gene is only small (~10% for Caucasians), and in view of the strong linkage signal obtained for 6p21 it appears likely that additional risk factors exist in the region. This hypothesis has been investigated before for some candidate genes (Grutters et al. 2002, Abdallah et al. 2003).

### DESCRIPTION OF THE INVENTION

The invention is based on the isolation of a coding sequence of the BTNL2 gene found in human individuals. It is another object of the invention to provide a method wherein BTNL2, or parts thereof, is amplified with one or more oligonucleotide primers. It is another object of the invention to provide a method of identifying individuals who carry no mutation of the BTNL2 coding sequence and therefore have no increased genetic susceptibility to sarcoidosis based on their BTNL2 genes. It is another object of the invention to provide a method of identifying a mutation leading to an increased susceptibility to sarcoidosis.

A person skilled in the art of genetic susceptibility testing will find the present invention useful for:
a) identifying individuals having a BTNL2 gene with no coding mutations, who therefore cannot be said to have an increased susceptibility to sarcoidosis from their BTNL2 genes;
b) avoiding of misinterpretations of polymorphisms found in the BTNL2 gene;
c) determining the presence of a previously unknown mutation in the BTNL2 gene;
d) identifying a mutation which increases the genetic susceptibility to sarcoidosis;
e) probing a human sample of the BTNL2 gene;
f) performing gene therapy;
g) making a functioning protein coded for by the BTNL2 genes for therapy of sarcoidosis.

In view of the strong linkage signal obtained for 6p21 and the associated likelihood of an additional risk factors in this region, the inventors of the present invention performed a comprehensive three-stage SNP fine mapping experiment on 6p21 in order to search for additional disease susceptibility genes. To overcome the preceding misinterpretations it is one aspect of the invention to have recruited a combination of extended families, trios and singleton patients, allowing both family-based (TDT) and population-based (case-control) association analyses to be performed in parallel. The idea of this procedure is that the consistency of TDT and case-control would deliver a pragmatic guide to the trustworthiness of individual association signals.

### BRIEF DESCRIPTION OF THE FIG.S

Fig. 1 shows a graphical representation of the stage I SNP screen on chromosome 6p21.
Fig. 2 shows a graphical representation of the stage II SNP screen of the combined "basic" and "extension" patient samples.
Fig. 3 shows the splicing alternative at SNP rs2076530 in exon 5 of the *BTNL2* gene.
Fig. 4 shows the computationally derived domain architecture of the *BTNL2* gene product.
Fig. 5 shows the computationally derived 3D structure model of the second IgV domain and the following IgC domain of the BTNL2 homodimer.
Fig. 6 shows the expression pattern of the *BTNL2* gene, analysed by nested rt-PCR and subsequent agarose gel electrophoresis of the rt-PCR products.
Fig. 7 shows fluorescence images of allelic BTNL2-GFP fusion protein transiently transfected into HeLa cells.

### DETAILED DESCRIPTION OF THE INVENTION

### Identification of association lead - stage I SNP scan

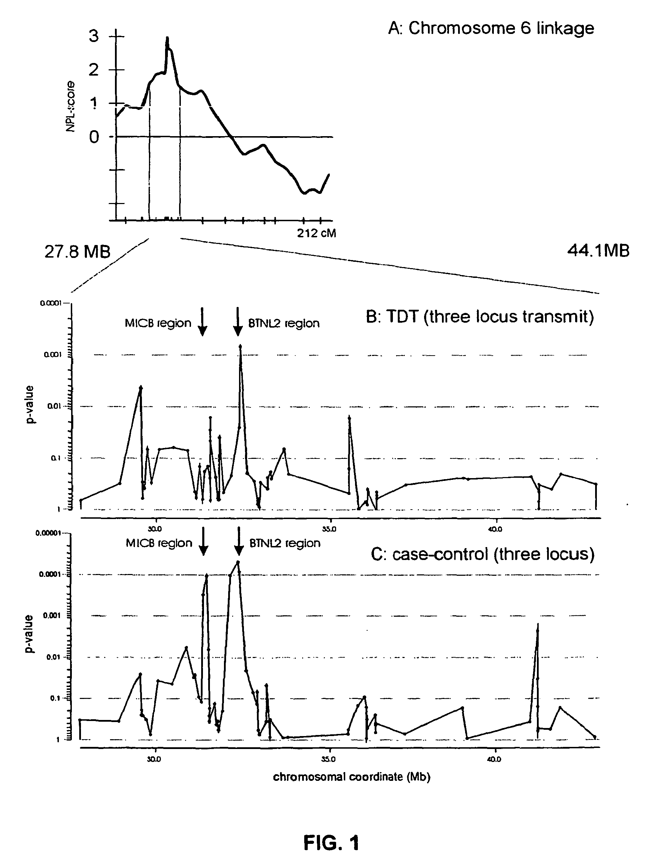

In the initial "stage I" SNP scan, individuals from the so-called "basic" sample were genotyped for 69 SNPs from an interval surrounding the maximum LOD position of the original genome screen (Schurmann et al. 2001), as shown in Fig. 1 (NCBI release 32 map coordinates 27.8 Mb - 44.1 Mb). Defined by a decrease in LOD score by 1.5 units or less, this interval represented an approximate 99% confidence region for linkage. To achieve sufficient power for association lead identification, a p value of 0.001 was adopted as a threshold for significance in both the TDT and the case-control analyses of three-locus SNP haplotypes. Applying this criterion, only the *BTNL2* sub-region gave a consistent association signal. A second peak in the *MICB* sub-region yielded a p value smaller than 0.001 in the case-control comparison, but not in the TDT analysis (p=0.3). This second putative lead region was nevertheless investigated further by genotyping markers rs1063635, rs3134900, rs3130062, rs1041981 and rs1799964 (for details: see Table 4a and Table 4b) in the "extension" sample (Table 1), together with the *BTNL2* lead markers rs2076523, hCV2455668, hCV2455646 and rs7192. Genotype logistic regression analysis with likelihood ratio-based forward inclusion at the p<0.05 level resulted in a model that included only two markers from the *BTNL2* region (rs2076523, rs7192), but none of the *MICB* markers. Furthermore, substantial long range LD was observed between markers in the *BTNL2* region and in the *MICB* region, with D' values ranging up to 0.94. Therefore, all subsequent mapping efforts were confined to the *BTNL2* region.

### Investigation of the BTNL2 gene region - stage II SNP scan

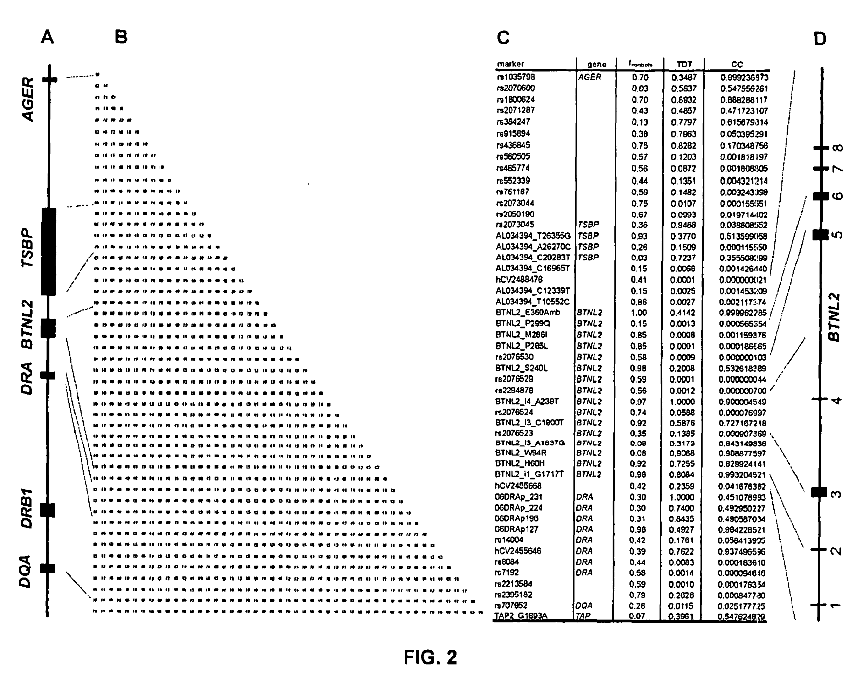

In "stage II", a total of 48 SNPs from the 440 kb association region around the *BTNL2* gene (NCBI release 32 map coordinates 32.177 - 32.616 Mb) were genotyped in the combined "basic" and "extension" samples, as shown in Table 1. SNPs were identified either in the ABI "Assay on Demand" database (www.store.appliedbiosystems.com), in dbSNP (www.ncbi.nlm.nih.gov), or by direct genomic sequencing of 47 sarcoidosis patients. Mutation screening of these individuals covered all exon and adjacent intron sequences of the revised *BTNL2* gene model (see next paragraph). Further SNPs were established in the *TSBP* gene, the *DRA* gene (including the promoter, not hitherto analysed for SNPs), and in some non-coding sequence telomeric of the *BTNL2* gene as shown in Fig. 2 and table 5. Single point TDT and case-control data were interpreted taking the haplotype structure of the region into account (Fig. 2B). Consistent results were obtained by the two tests for a 15 kb region at the 3-prime end of the *BTNL2* gene (markers hCV2488476 to rs2294878). Here, p-values were two to three orders of magnitude smaller than for the other regions (Fig. 2C).

### Functional and structural effects of BTNL2 gene mutations

The gene structure of *BTNL2,* as logged in public sequence databases, was scrutinised by means of cDNA cloning. In the original report (Stammers et al. 2000), no contiguous cDNA amplification was achieved for exons 1-4 and 5-6, raising the possibility that two different genes exist at this locus. We have identified three additional exons (one 5' and two 3') and excluded original exon 3 from the gene model. Overlapping rt-PCR successfully confirmed the presence of a single transcript and, thereby, corroborated the validity of this revised gene model.

The 15kb segment most strongly associated with sarcoidosis contains four functional SNPs (P299Q, M286I, P285L, rs2076530), which together yielded a p value of 1.9*10⁻⁷ in a four-marker haplotype TDT, and of 6.2*10⁻⁷ in the corresponding case-control analysis (for single point results, see Fig. 2C). Most of the association was due to rs2076530, leaving p=0.03 for the inclusion of the other three markers in a case-control haplotype regression model. The strong association between sarcoidosis and rs2076530 was verified in an independent "replication" sample ("stage III", Table 1; TDT χ²=9.7, p=0.0018; case-control χ²=21.0, p=2.7 * 10⁻⁵).

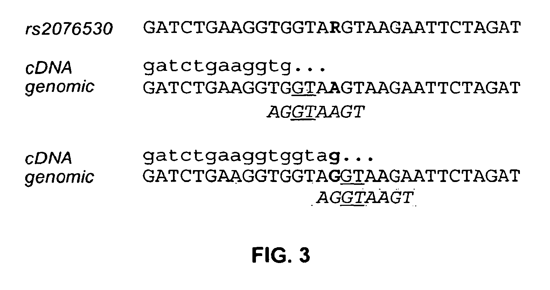

According to the reported gene model (Stammers et al. 2000), rs2076530 would have been predicted to cause an amino acid exchange. But as a further aspect of the invention it was presumed and could be shown indeed that, in reality, the SNP is located at position -1 of a donor splice site as shown in Fig. 3. A four base pair cDNA deletion observed in cloned rt-PCR products from the gene model verification is explicable by the way in which rs2076530 alters the anatomy of the splice site. Guanine is present at position -1 of 77% of donor sites (Krawczak et al. 1992, Long et al. 1999) so that the A to G transition of rs2076530 is likely to result in the recruitment of an alternative splice site located 4 bp upstream as shown in Fig. 3. Genotype-specific splicing was confirmed by rt-PCR of DNA/cDNA pairs from lymphoblastoid cell lines and peripheral blood samples (Table 2). The loss of four bases from the cDNA transcribed from the A allele causes a frameshift and a premature stop in the next exon. In the corresponding protein product, the 118 C-terminal residues in the untruncated protein would be replaced by five differing amino acids.

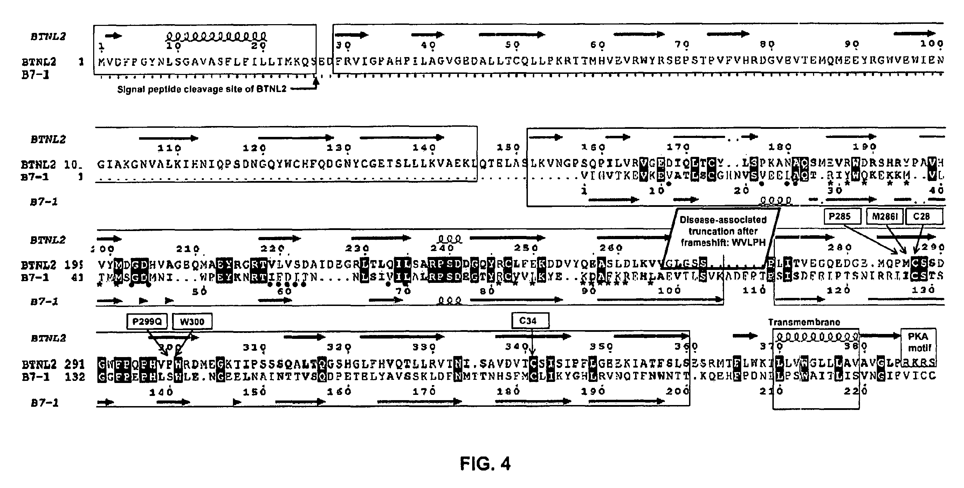

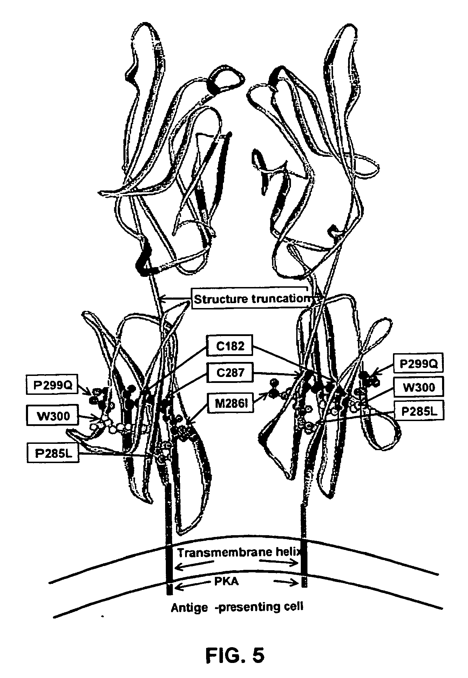

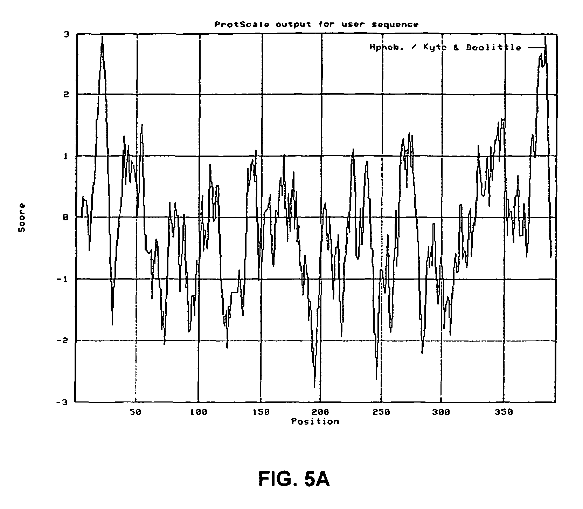

The effects of the disease-associated variants upon the structure of the BTNL2 protein were investigated *in silico.* The *BTN* (butyrophilin-like) genes are members of the immunoglobulin superfamily (IgSF). The general domain architecture of the *BTN* genes comprises a small but variable number of consecutive immunoglobulin-like (Ig-like) ectodomains, with an N-terminal signal peptide and a C-terminal transmembrane helix anchoring the protein to the membrane of antigen-presenting cells (Rhodes et al. 2001, Sharpe et al. 2002). The protein product of the revised *BTNL2* gene as shown in Fig. 4 contains two N-terminal homologous IgV (Ig-like variable) domains with a sequence identity of 46% and one C-terminal IgC (Ig-like constant) domain. The cleavage site of the putative N-terminal signal peptide (Rhodes et al. 2001, Sharpe et al. 2002, Stammers et al. 2000) is predicted between residues 26 and 27, and a hydropathy index plot as shown in Fig. 5A together with the results of transmembrane prediction servers (Albrecht et al. 2003) support a single C-terminal transmembrane helix following the IgC domain. Such a membrane-anchoring helix is also found at the C-termini of IgC domains of MHC antigens and other co-stimulatory receptors. Indeed, a distant homology of butyrophilin-like proteins to MHC antigens and B7-1/-2 (CD80/CD86) co-stimulatory receptors (Ikemizu et al. 2000, Schwartz et al. 2001, Stamper et al. 2001, Zhang et al. 2003) has previously been reported (Rhodes et al. 2001, Stammers et al. 2000). The IgV and IgC domain sequence of the crystal structure of the B7-1 homodimer (Ikemizu et al. 2000, Stamper et al. 2000, Bajorath et al. 2001) is detected, with a significant *E*-value of 3·10⁻⁷, by a BLAST search of PDB using the second IgV and the adjacent IgC domain of BTNL2. We thus modeled the 3D structure of both BTNL2 domains based on a manually curated sequence-structure alignment of BTNL2 to the B7-1 template, sharing an amino acid sequence identity of 24%. The resulting 3D model of BTNL2 as depicted in Fig. 5 shows a truncation of the protein structure at the site of the premature stop introduced by rs2076530, just before the start of the IgC domain and the attached transmembrane helix. Variants P285L, M286I and P299Q are located in β-strands on the protein surface of the IgC domain (Bajorath et al. 2001) and mapped to the residues I126, I127 and S140 of B7-1. Interestingly, all variants change to amino acids whose side chains are physico-chemically more similar to the corresponding wild-type B7-1 residues in its IgC domain. Several residues involved in binding of the IgV domain of B7-1 to its receptor CTLA-4 (Stamper et al. 2000) are conserved in BTNL2 (Fig. 4, Fig. 5). This observation suggests that BTNL2 may have a similar receptor-binding site as B7-1 (see Fig. 5).

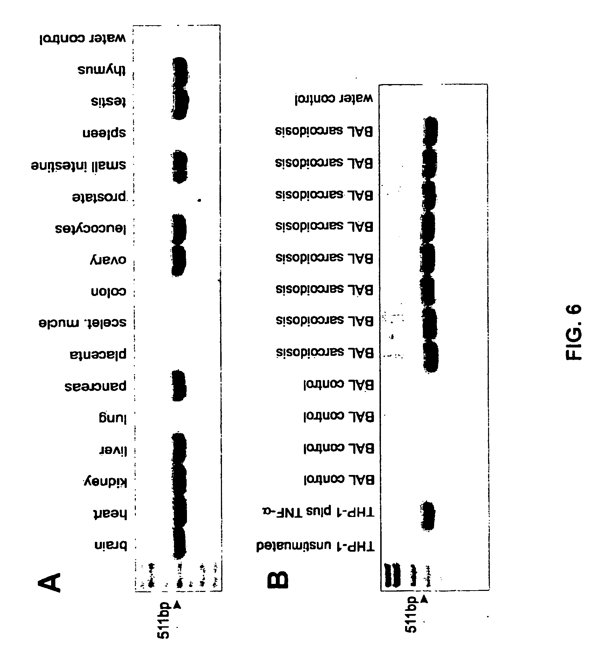

To evaluate the expression of the *BTNL2* gene in relevant target cells, rt-PCR was performed in a tissue panel, in THP 1 cells, and in bronchoalveolar lavage (BAL) cells from patients and controls. Since *BTNL2* expression was generally low, nested rt-PCR had to be employed to detect sufficient amounts of transcript. Fig. 6A shows the observed expression in a variety of tissues, including leucocytes and thymus. Transcripts were also detected in all eight BAL cell samples from sarcoidosis patients, but not in BAL-cells from control individuals or in normal lung cells as shown in Fig. 6B. Expression of the *BTNL* gene could also be induced by TNFα-stimulation in the myelomonocytic cell line THP1 (Fig. 6B).

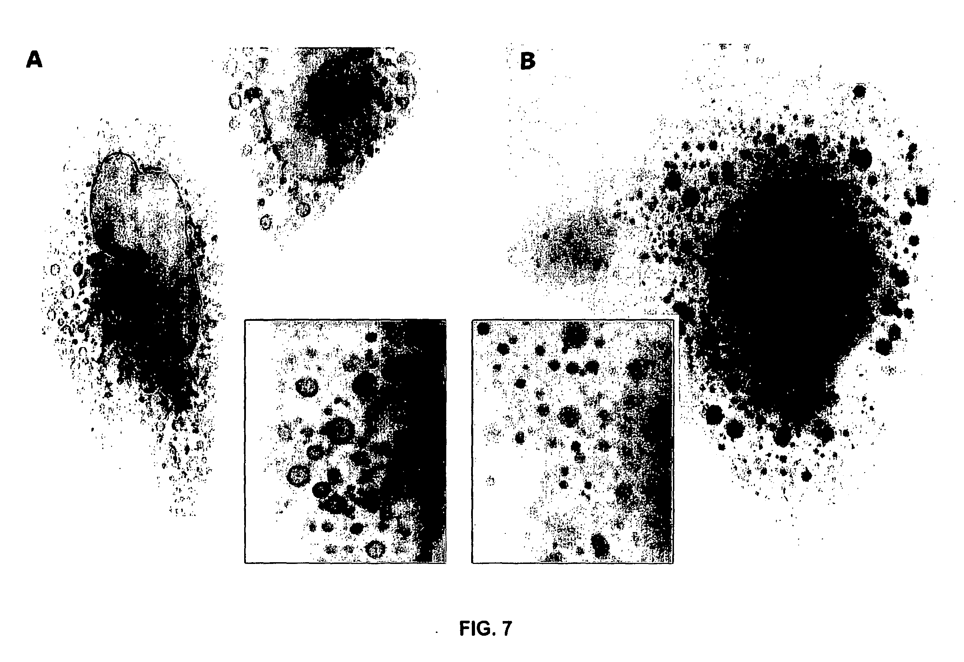

The two alleles of the *BTNL2* transcript (4bp deletion versus full length) were cloned into a mammalian expression vector with C-terminal fusion GFP. In transfection experiments of HeLa and HEK-cells (data not shown), these allelic constructs showed a different subcellular localization. In contrast to the full-length transcript with a clear membrane pattern, the deletion allele of the BTNL2-GFP fusion protein was contained in cytoplasmatic vesicular structures as shown in Fig. 7. The high expression levels in the transfection experiments do not reflect the physiological abundance of the protein in immunoregulatory cells, where a nested PCR was necessary to detect the transcript. Therefore, the vesicle formation and the high abundance of BTNL2 in all membranes represents likely an effect of this overexpression.

### Differentiation of BTNL2 and DRB1 effects

The truncating A allele of rs2076530 represents a strong predisposing factor for sarcoidosis (table 3A). However, the *DRB1* gene in close vicinity (~200 kb) of the *BTNL2* gene has also been implicated in the aetiology of the disease (Foley et al. 2001, Rossman et al. 2003). Group typing of *DRB1* alleles in all three samples of the present study (Hampe et al. 2004) indeed confirmed that amplification group 3 (alleles DRB1*03, *08, *11(not *1122/30), *12, *13, *14, (not*1410/39)) (Hampe et al. 2004) confers an increased disease risk. It was therefore possible that alleles in amplification group 3 acted as a confounder of the association between rs2076530 and sarcoidosis. However, a stratified analysis of the respective *DRB1* and *BTNL2* genotypes (table 3B) was insignificant throughout (Breslow-Day test for homogeneity of odds ratios: χ²=0.12, 1 d.f., p>0.5 for both stratifications). Therefore, *DRB1* amplification group 3 and rs2076530 allele A represent independent risk factors for sarcoidosis. Since no important protective alleles have hitherto been identified within *DRB1* amplification group 3, further subtyping of *DRB1* was regarded unnecessary. Interestingly, the odds ratios obtained for heterozygous (OR=1.55) and homozygous (OR=2.56) carriers of rs2076530 allele A follow a multiplicative model whereas the presence of DRB1 group 3 had an almost dominant effect (ORs=1.90 and 2.02). The population attributable risks of rs2076530 were 34% for genotype AA and 22% for AG, owing to the high frequency of the risk allele.

### DISCUSSION

A three-stage SNP mapping experiment was performed on chromosome 6p21 in order to localise genetic variants that predispose to sarcoidosis. A major disease-associated SNP (rs2076530) was identified in the 3' region of the *BTNL2* gene. This variant is common (minor allele frequency 0.42) and confers its risk completely independently of (known) disease-associated mutations in the *DRB1* gene. The susceptibility allele of rs2076530 is characterised by an odds ratio of 1.55 in heterozygotes, and of 2.56 in homozygotes. Compared to associations seen in other complex diseases like, for instance, that of *CARD15* gene mutations and Crohn disease, rs2076530 is of moderate influence upon the individual disease risk, but has a substantial contribution on the population level (PARs 22% for heterozygotes and 34% for homozygotes). This is especially intriguing in view of the profound impact of the SNP upon BTNL2 protein structure and function. This apparent discrepancy may be explicable in terms of a highly redundant system of co-stimulatory molecules, including the products of the butyrophilin gene cluster, B7-1 and other, as yet unknown members of the same functional class of proteins. The human *BTN* gene cluster has recently been localised on chromosome 6p22 (Rhodes et al. 2001), approximately 6 Mb telomeric of *BTNL2.* For completeness, six markers from the *BTN* cluster were also genotyped in the combined "basic" and "extended" samples of the present study, however without providing any evidence for disease association (data not shown).

One of the principal co-stimulatory pathways for naive T-cells is initiated when CTLA-4 and CD28 engage B7 class molecules in the context of a primary signal, delivered through the TCR (Walunas et al. 1996). The B7-1 protein (*CD80*) is a co-stimulatory molecule with known anti-inflammatory activity mediated through interaction with CTLA-4 (Borriello et al. 1997, Collins et al. 2002). Deletion of the IgC domain in the *CD80* gene had a substantial pro-inflammatory effect in a mouse plasmid vaccination model (Agadjanyan et al. 2003). Based upon amino acid homology and domain structure, BTNL2 was modelled along the B7-1 protein (Fig. 4). It may therefore be hypothesised that a potential T-cell down-regulatory function of BTNL2 would be impaired by the truncating mutation rs2076530 and the concomitant loss of the IgC domain and transmembrane helix, possibly by the lack of membrane localisation (Fig. 7). Inappropriate T-cell activation would also be compatible with the clinical immunology of sarcoidosis, characterized by a dysregulated T-helper cell activation (Zissel et al. 2000).

The importance of co-stimulation and its dysfunction are increasingly recognised for many autoimmune disorders (Ueda et al. 2003). The findings of the inventors of the present invention also emphasise the importance of this pathway since CTLA4, a prototype of a putative BTNL2 receptor, is a risk factor for a wide range of autoimmune disorders, including type I diabetes, autoimmune hypothyroidism and Graves disease (Ueda et al. 2003). The exact functional role of BTNL2 in the co-stimulatory system remains to be elucidated.

However it could be shown that variants of BTNL2 confer susceptibility to sarcoidosis, a disorder that is characterized by a disturbed T-cell function. As the BTNL2 protein serves as a co-stimulatory molecule, the disease susceptibility variants likely influence T-cell activation. Therefore diagnosis on basis of the genetic or the protein variants is useful for the choice of therapy. Especially interference with or enhancement of BTNL2 function provides a handle for therapeutic targeting of T-cell function and the immunoregulatory balance.

Enhancement of the BTNL2 function may be accomplished by administration of the long protein form (SEQ ID NO: 456) either by parenteral (intravenous, subcutaneous) or oral administration. Also the naturally occurring variants as depicted in SEQ ID NO: 457 and SEQ ID NO: 458, which only differ in their amino acids at positions 285, 286 and 299, may be used.

Interference with BTNL2 function may be accomplished by the design of a small molecule or antibody (or antibody fragment) inhibitors of BTNL2 binding to its natural ligands on the T-cell.

Still another therapeutic approach is the performance of gene therapy with a vector transformed with a nucleotide sequence of the wild type BTNL2 sequence (SEQ ID NO: 455), comprising transfecting sarcoidosis affected cells in vivo with an effective amount of vector, allowing the cells to take up the vector, and measuring a reduction in sarcoidosis.

### EXAMPLE

### METHODS

### Sample recruitment

Index patients were contacted between 2000 and 2003 through either specialised hospitals and general practitioners, or through the German sarcoidosis patient organisation (Deutsche Sarkoidose-Vereinigung e. V., http://http://www.sarkoidose.de). All diagnoses were made on the basis of the International Consensus Statement on sarcoidosis (Costabel and Hunninghake 1999, Statement on sarcoidosis 1999). Patients from sarcoidosis families were interviewed by telephone about their individual and familial sarcoidosis history. All patients (families and singletons) completed a questionnaire about the course of their disease. Physicians were contacted to confirm the diagnosis, and the presence of sarcoidosis was additionally verified by biopsy in 78% of cases from the "basic" sample, 90% of patients from the "extension" sample, and in 88% of patients from the "replication" sample. For the remainder, the clinical course and the radiology and laboratory data were regarded as sufficiently consistent with the diagnosis of sarcoidosis (Costabel and Hunninghake 1999, Statement on sarcoidosis 1999). All patients were of German origin. A group of 517 age- and sex-matched healthy German controls were also included in the study. All participants gave written informed consent for participation in the study. The protocols were approved in writing by the institutional ethics and data protection authorities. An overview of the patient samples is given in Table 1.

### Additional details about the preparation of biological material

Bronchoalveolar lavage (BAL) cell samples were taken from eight sarcoidosis patients with overt disease (average age: 44.8 ± 3.6 years), and four controls (average age: 32.7 ± 8.2 years). The diagnosis of chronic sarcoidosis was established as noted in the text (Costabel and Hunninghake 1999). All sarcoidosis patients showed clinical signs for active disease. Four patients, who underwent bronchoscopy for evaluation of chronic coughing and were retrospectively found to be free of inflammatory or malignant disorder, served as additional controls. All patients and controls gave written informed consent to the study. The protocol was approved the institutional review board of the institution. BAL was performed as previously described (Hunninghake et al. 1979). Mean recovery was 62.5% and the average cell number/100ml of BAL cells was 12.9 ± 3.3x106. The cells were centrifuged at 500 x g and washed three times with phosphate buffered saline (PBS) at +4°C. The cell suspensions used in this study contained 85.4 ± 5.3% alveolar macrophages. Total cellular RNA was extracted from BAL cells using TRIzol reagent® (Invitrogen, Paisley, UK) according to the manufacturer's recommendations. To remove contaminating genomic DNA, total RNA samples were treated with deoxyribonuclease I (Boehringer Mannheim, Mannheim, FRG). Reverse transcription (RT) was performed on equal amounts of total RNA per sample, using 1x First Strand Buffer (Invitrogen, Paisley, UK), 2'-deoxynucleotide 5'triphosphate (Invitrogen, Paisley, UK), dithiothreitol, oligo (dT)12-18 primer (Invitrogen, Paisley, UK), RNasin, and Superscript RT (Invitrogen, Paisley, UK). Prior to addition of the RT, the RT mixture was incubated for 5 min at 65°C, followed by an incubation period of 5 min at 37°C. After addition of the enzyme, incubation was continued for another 1 h at 37°C. Forquality control, each RNA sample was subjected to RT-PCR omitting the RT.

### Genotyping and sequencing

SNP genotyping was performed as previously described, using TaqMan technology on an automated platform (Hampe et al. 2002, Hampe et al. 2001). Primer and probe sequences of all assays are included in Table 4a and Table 4b. Sequencing of genomic DNA was performed using Applied Biosystems (Foster City, Ca, USA) BigDye^{™} chemistry according to the supplier's recommendations (for primer sequences, see Table 5). *DRB1* genotyping was performed using group-specific primers in PCRs of 2,5ng of genomic DNA as described (Hampe et al. 2004). All markers were tested for Hardy-Weinberg equilibrium in controls before inclusion in the association statistics.

### cDNA cloning and rt-PCR

In order to verify the published *BTNL2* gene model, conserved exons were searched for in mouse and human genomic sequences (Twinscan gene predictions, genome.ucsc.edu) and related to predicted splice sites (www.fruitfly.org/seq_tools/splice.html). The predicted exons were verified by rt-PCR in a pool of cDNA tissue samples (Human Multiple Tissue cDNA Panels I and II, Clontech) and by subsequent cloning and sequencing of PCR products. Details of the primers are given in tables 4a, 4b, 5 and 6. Bronchoalveolar lavage (BAL) cell cDNA samples were obtained from sarcoidosis patients with overt disease and from controls according to standard clinical and molecular procedures (Hunninghake et al. 1979). For expression analysis, nested PCR was performed with primers TTAGAGTCATTGGCCCTGCT (SEQ ID NO: 439) and GCCCATAGAGCAGATAGTCAG (SEQ ID NO: 450) for the first, and AGTACCGCTGCCTTTTTGAA (SEQ ID NO: 444) and TGGATCACACGCTTGTTCTG (SEQ ID NO:449) for the second round of amplification Table 6).

### Statistical analysis

Single point and extended haplotype transmission disequilibrium tests were performed using the TRANSMIT (Clayton et al. 1999) and GENEHUNTER (Kruglyak et al. 1996) programs. Haplotype frequency estimates among singletons were obtained using an implementation of the EM algorithm (HAPMAX, www.uwcm.ac.uk\uwcm\mg\download) (Krawczak et al. 1988). Significance testing of haplotype frequency differences was also performed with HAPMAX, making use of the fact that twice the log-likelihood ratio between two nested data models approximately follows a χ² distribution with k degrees of freedom, where k is the difference in parameter number between the two models. Significance assessment of associations with or between single locus genotypes was performed using χ² or Fisher's exact test for 2 x 3 contingency tables. Genotypic regression analysis was performed with SPSS, coding individual SNP genotypes as categorical variables.

### In silico protein analysis

Protein domain architectures were retrieved from the Pfam database (www.sanger.ac.uk/Software/Pfam/) and examined via the NCBI conserved domain search website (www.ncbi.nlm.nih.gov:80/Structure/cdd/wrpsb.cgi). To predict the 3D structure of the *BTNL2* gene product, we explored all fold recognition servers available via the meta-server BioInfo.PL. Based upon the prediction results, a sequence-structure alignment of BTNL2 to B7-1 (Fig. 4) was constructed for 3D-modeling server WHAT IF (www.cmbi.kun.nl/gv/servers/WIWWWI/), which returned a full-atom structure model of the second IgV and the following IgC domain of BTNL2.

### Fluorescent fusion proteins

Allelic variants of the *BTNL2* transcript were constructed from clones generated during the gene model verification (see above). Final full-length amplification was performed with primers GTC TCG AGA TGG TGG ATT TTC CAG GC (SEQ ID NO: 451) and CAC CCG GGG CTC CTC TTC CTG (SEQ ID NO: 452), for the untruncated transcript and GTC TCG AGA TGG TGG ATT TTC CAG GC (SEQ ID NO: 453) and AAC CCG GGG TGG GGA AGA ACC (SEQ ID NO: 454) for the 4pb deletion variant. Fragments were cloned into the expression vector pEGFP-N2 (Clontech, Palo Alto, Ca / USA). Hela cells were transfected with 1 µg of the respective constructs according to the manufacturer's protocol (Fugene 6, Roche, Basel / Switzerland). Empty pEGFP-N2 vector served as the positive control. Cells were fixed in 4% PFA in PBS (pH7.6) after 24 hours and mounted onto glass slides in 1:1 glycerol in PBS. Fluorescence was visualized with an epifluorescence microscope (Axiophot, Zeiss, Jena / Germany).

**Table 1: Samples of sarcoidosis patients used for disease association analysis. Upper part: non-overlapping categories of sampling units (families, trios, singleton cases); central part: summary of case/patient numbers. The total number of independent cases equals the sum of the sampling units in the upper part; lower part: illustration of experiments performed in different combinations of patient samples.**

| | | Patient sample | | |
|---|---|---|---|---|
| Sampling unit | basic | extension | replication | Full |
| Affected sib pairs | 70 | 9 | 10 | 89 |
| (ASP) - 2 sibs | | | | |
| ASP - 3 sibs | 8 | -- | -- | 8 |
| Complex families | 11 | 5 | -- | 16 |
| Trios (complete) | 153 | 72 | 203 | 428 |
| Trios (one parent missing) | 10 | 79 | 62 | 151 |
| Single patients | 13 | 65 | 177 | 255 |
| Total number of independent cases | 265 | 230 | 452 | 947 |
| Total number of patients (male/female) | 372 (163/209) | 248 (123/125) | 462 (175/287) | 1082 (461/621) |
| Average age at diagnosis (SD) in years | 34.7 (10.3) | 37.2 (11.4) | 37.3 (11.3) | 36.4 (11.1) |

| Experiment | Initial SNP screen | | | |
|---|---|---|---|---|
| | SNP fine mapping | | Replication | |
| | | | | Differentiation of *DRB1 -BTNL2* effects |

| **Table 2:** Verification of the splicing effects of rs2076530. Matching pairs ofcDNA and DNA | | | | | |
|---|---|---|---|---|---|
| from lymphoblastoid cell lines (upper part) and from the peripheral blood of normal controls | | | | | |
| (lower part) were genotyped and the *BTNL2* splice forms evaluated by rtPCR, cloning and | | | | | |
| sequencing of PCR products. | | | | | |
| source | rs2076530 genotype | no. samples | no. clones | 4 bp present | 4 bp deleted |
| cell lines | GG | 4 | 64 | 64 | 0 |
| | AG | 5 | 98 | 22 | 76 |
| | AA | 3 | 131 | 0 | 131 |
| leucocytes | GG | 1 | 36 | 36 | 0 |
| | AG | 3 | 36 | 9 | 27 |
| | AA | 3 | 35 | 0 | 35 |

| **Table 3A:** Association between *BTNL2* and *DRB1* risk alleles and sarcoidosis (based upon | | | | | | |
|---|---|---|---|---|---|---|
| 947 cases and 517 controls). f_{case}, f_{control}: allele frequency in cases and controls. Genotypes at | | | | | | |
| both loci were in Hardy-Weinberg equilibrium among controls; p (global): error probability of | | | | | | |
| a global χ² test for allelic association; tdt (obs./exp.): ratio of observed vs. expected number of | | | | | | |
| transmissions; p (allele): allele group-specific TDT error probability, as reported by the | | | | | | |
| TRANSMIT program. | | | | | | |
| Locus | allele | f_{case} | f_{control} | p (global) | tdt (obs./exp.) | p (allele) |
| *BTNL2* (rs2076360) | A | 0.69 | 0.58 | | 1303/1239 | 3.1*10⁻⁷ |
| | G | 0.31 | 0.42 | 3.3*10⁻⁹ | 605/669 | 3.1*10⁻⁷ |
| | | | | | | |
| *DRB1* (allele groups) | 1 | 0.067 | 0.112 | | 124/155 | 1.1*10⁻⁵ |
| | 2 | 0.193 | 0.169 | | 344/330 | n.s. |
| | 3 | 0.551 | 0.461 | | 1010/947 | 7.4*10⁻⁷ |
| | 4 | 0.100 | 0.146 | | 186/217 | 0.0001 |
| | 5 | 0.082 | 0.103 | | 145/156 | n.s. |
| | 6 | 0.003 | 0.005 | | 10/4 | n.s. |
| | 7 | 0.004 | 0.004 | 7.1*10⁻⁸ | 7/3 | n.s. |

| **Table 3B:** Differentiation between *BTNL2* and *DRB1* effects. x: *DRB1* alleles other than | | | |
|---|---|---|---|
| alleles in amplification group 3. | | | |
| rs2076530 | *DRB1* | case | control |
| Any | 3-3 | 282 | 125 |
| Any | 3-x | 481 | 227 |
| Any | x-x | 184 | 165 |
| AA | any | 445 | 170 |
| AG | any | 414 | 261 |
| GG | any | 88 | 86 |
| AA | 3-3,3-x | 388 | 137 |
| AA | x-x | 57 | 33 |
| AG,GG | 3-3,3-x | 375 | 215 |
| AG,GG | x-x | 127 | 132 |

**Table 4a: Primers for all Taqman SNP assays used. "Assay on Demand" assays (starting with hCV, https://store.appliedbiosystems.com).**

| Marker | hCV number | Gene | Position | Primers: | | Cohort used |
|---|---|---|---|---|---|---|
| | | | (NCBI) MB | Forward 5'-3' | | |
| | | | | Reverse 5'-3' | | |
| rs2073528 | hCV11255445 | *BTN3A2* | 26,44 | | | Basic/extension |
| rs2072803 | hCV2474923 | *BTN2A2* | 26,46 | | | Basic/extension |
| | hCV2474907 | *BTN3A1* | 26,48 | | | Basic/extension |
| | hCV2474883 | | 26,50 | | | Basic/extension |
| | hCV98Q4528 | | 26,54 | | | Basic/extension |
| rs3736781 | hCV9804725 | *BTN1A1* | 26,57 | | | Basic/extension |
| rs126007 | | | 27,80 | TCCTTAACCAACCTCATCCTGG | (SEQ ID NO: 001) | Basic-screen |
| | | | | TGGTAACTCGACAGCTGATTCTTG | (SEQ ID NO: 002) | |
| rs929042 | | *RFP* | 28,95 | CGGACGTGTGGTTCACAGC | (SEQ ID NO: 003) | Basic-screen |
| | | | | CTCCGTGTACAATGTGTCCGTG | (SEQ ID NO: 004) | |
| rs53161 | | | 29,56 | GGGCTGTCCACGTGCAC | (SEQ ID NO: 005) | Basic-screen |
| | | | | GCAGAGTTGAACCAGTATATAACCTTTC | (SEQ ID NO: 006) | |
| rs404240 | | *UBD* | 29,59 | CCACAAGAAACAAGGGCAGC | (SEQ ID NO: 007) | Basic-screen |
| | | | | GAACATGTCCGGTCTAAGACCAA | (SEQ ID NO: 008) | |
| rs362536 | | *UBD* | 29,59 | TGGAGAGAACCTGGAATTAAGGC | (SEQ ID NO: 009) | Basic-screen |
| | | | | GGACTATATCTAAGTCTTACTTCAATAGCTGGA | (SEQ ID NO: 010) | |
| rs1805057 | | *GABBR1* | 29,64 | CCATCTGGGCCTTGGCA | (SEQ ID NO: 011) | Basic-screen |
| | | | | GGTAATGGTCTGGTTGTTGTAGTTGAA | (SEQ ID NO: 012) | |
| rs3130253 | | *MOG* | 29.73 | GTTCTCCTCGCGGTGCTG | (SEQ ID NO: 013) | Basic-screen |
| | | | | CCCACCCTCTGCCCTGTAC | (SEQ ID NO: 013) | |
| rs1569315 | | *HLA-G* | 29,85 | CGCAGGTCCTCGTTCAGG | (SEQ ID NO: 015) | Basic-screen |
| | | | | AGTGGATGATTGGCTGCGA | (SEQ ID NO: 016) | |
| rs8347 | | *HCG-V* | 30,10 | AGGAGACCTCAGAGCTCCCAG | (SEQ ID NO: 017) | Basic-screen |
| | | | | AAGTGACCAATCTCAAGCCAGC | (SEQ ID NO: 018) | |
| rs1264457 | | *HLA-E* | 30,52 | CTCACACCCTGCAGTGGATG | (SEQ ID NO: 019) | Basic-screen |
| | | | | AGGGTGAGATAATCCTTGCCG | (SEQ ID NO: 020) | |
| rs2074510 | | *GTF2H4* | 30.94 | GAGTGCAGCGTCTATTCTCATCC | (SEQ ID NO: 021) | Basic-screen/extension |
| | | | | TTGGCTCCTGGAGATTGAGG | (SEQ ID NO: 022) | |
| rs3095318 | | *CDSN* | 31,15 | CGTCTCGGGCACCCTG | (SEQ ID NO: 023) | Basic-screen |
| | | | | AGACACACAGACTAGAGGTAGGTGTCA | (SEQ ID NO: 024) | |
| rs2073721 | | *SC1* | 31,19 | GGTGGACCAAAGAGCCTGC | (SEQ ID NO: 025) | Basic-screen |
| | | | | CATCCAGTTCCGCCAACAC | (SEQ ID NO: 026) | |
| rs1050393 | | *HLA-C* | 31,30 | GGAGTATTGGGACCGGGAG | (SEQ ID NO: 027) | Basic-screen |
| | | | | CGCTCTGGTTGTAGTAGCCG | (SEQ ID NO: 028) | |
| rs1051488 | | *HLA-B* | 31,38 | CTGGCCTGGCTGTCCTAGC | (SEQ ID NO: 029) | Basic-screen |
| | | | | CTTCCCAGTAATGAGGCAGGG | (SEQ ID NO: 030) | |
| rs1063630 | | *MICA* | 31,44 | CCCAGAGCCCCACAGTCTT | (SEQ ID NO: 031) | Basic/extension |
| | | | | AGAAACCCTGACTGCACAGATC | (SEQ ID NO: 032) | |
| rs1063635 | | *MICA* | 31,44 | CTTTGAGCCACGACACCCA | (SEQ ID NO: 033) | Basic-screen/extension |
| | | | | GGCTCACCAGAGGGCACA | (SEQ ID NO: 034) | |
| rs3828916 | hCV3273671 | | 31,53 | | | Basic/extension |
| rs3131639 | | *MICB* | 31,53 | GCAGAAACGCAGGGCAAA | (SEQ ID NO: 035) | Basic/extension |
| | | | | TGTCAAGTCCTCGGTCTCTGTGT | (SEQ ID NO: 036) | |
| rs3134900 | | *MICB* | 31,53 | CTTGGGTGGAAAGGTGATGG | (SEQ ID NO: 037) | Basic-screen/extension |
| | | | | GGTTTTGGGAGAGGAAGAGCTC | (SEQ ID NO: 038) | |
| AC006046_ G30156A | | *MICB* | 31,53 | CTGGCTCTGCCCTTTCTTCTC | (SEQ ID NO: 039) | Basic/extension |
| | | | | CGGTGATGTTGCCCTCTGA | (SEQ ID NO: 040) | |
| rs3130062 | | *IKBL* | 31,59 | CGGGAACATGCCCAGAAGT | (SEQ ID NO: 041) | Basic-screen/extension |
| | | | | GGCTGGAGCCCTCAGCA | (SEQ ID NO: 042) | |
| rs1800683 | hCV7514865 | *LTA* | 31,60 | | | Basic/extension |
| rs1041981 | | *LTA* | 31,60 | CCCGTCAGCACCCCAAGATG | (SEQ ID NO: 043) | Basic/extension |
| | | | | TGGGAGGTCAGGTGGATGTTTACC | (SEQ ID NO: 044) | |
| rs1799964 | | *TNFa* | 31,60 | GATGGGACTCACCAGGTGAGG | (SEQ ID NO: 045) | Basic-screen/extension |
| | | | | CCAGAGGTCTCCTGTAACCCA | (SEQ ID NO: 046) | |
| rs1799742 | | *TNFa* | 31,60 | TCCAGGGCTATGAAAGTCGA | (SEQ ID NO: 047) | Basic-screen |
| | | | | TGCTGGTTTCAGTCTTGGCT | (SEQ ID NO: 048) | |
| rs1800629 | | *TNFa* | 31,60 | CCTGCATCCTGTCTGGAAGTTAGAAG | (SEQ ID NO: 049) | Basic-screen |
| | | | | TGGGCCACTGACTGATTTGTGTGT | (SEQ ID NO: 050) | |
| rs361525 | | *TNFa* | 31,60 | CAGTGGCCCAGAAGACCC | (SEQ ID NO: 051) | Basic-screen |
| | | | | AGCATCAAGGATACCCCTCAC | (SEQ ID NO: 052) | |
| rs707916 | | *DDAH2* | 31,76 | CTGGAGCCGGGAGTAGTGC | (SEQ ID NO: 053) | Basic-screen |
| | | | | CGAGCCTAAGGAGTTAAGCATCC | (SEQ ID NO: 054) | |
| rs707926 | | *VARS2* | 31,81 | GGTGGAGTGGGAGCAGTCAG | (SEQ ID NO: 055) | Basic-screen |
| | | | | TCACGGCCGGAAGATGAG | (SEQ ID NO: 056) | |
| rs2075800 | | *HSP70-* | 31,84 | AACGAGCTCCTTTCGTGGCT | (SEQ ID NO: 057) | Basic-screen |
| | | *HOM* | | CACATACCCTGTTCCGCAGG | (SEQ ID NO: 058) | |
| rs539689 | | *HSPA1B* | 31,86 | CAAAGTCCTTGAGTCCCAACAGT | (SEQ ID NO: 059) | Basic-screen |
| | | | | AACCCCATCATCAGCGGA | (SEQ ID NO: 060) | |
| AF019413_C 89532G | | *C2* | 31,96 | TGAGCGTTGCCATTATCACC | (SEQ ID NO: 061) | Basic-screen |
| | | | | TCCTCTCTCATCACCATCACGT | (SEQ ID NO: 062) | |
| rs1035798 | | *AGER* | 32,18 | CCAGGGTCTTCTCCAAGGC | (SEQ ID NO: 063) | Basic-screen |
| | | | | GCCGTGAGTTCAGAGGCAG | (SEQ ID NO: 064) | |
| rs2070600 | | *AGER* | 32,18 | AGGCCCCTGGGACAGTGT | (SEQ ID NO: 065) | Basic/extension |
| | | | | CCGACAGCCGGAAGGAA | (SEQ ID NO: 066) | |
| rs1800624 | hCV3293837 | *AGER* | 32,18 | | | Basic/extension |
| rs2071287 | hCV3293818 | *NOTCH* 4 | 32,20 | | | Basic/extension |
| rs384247 | hCV2412441 | *NOTCH* 4 | 32,21 | | | Basic/extension |
| rs915894 | hCV3293780 | *NOTCH* 4 | 32,22 | | | Basic/extension |
| rs436845 | hCV2412414 | | 32,22 | | | Basic/extension |
| rs560505 | hCV2495657 | *TSBP* | 32,29 | | | Basic/extension |
| rs485774 | hCV2495577 | *TSBP* | 32,32 | | | Basic/extension |
| rs552339 | hCV2495574 | *TSBP* | 32,32 | | | Basic/extension |
| rs761187 | hCV2234065 | *TSBP* | 32,34 | | | Basic/extension |
| rs2073044 | hCV2488525 | *TSBP* | 32,37 | | | Basic/extension |
| rs2050190 | hCV2488524 | *TSBP* | 32,37 | | | Basic/extension |
| rs2073045 | hCV2488523 | *TSBP* | 32,37 | | | Basic/extension |
| AL034394_T 26355G | | | 32,37 | AGAACTTCTTAGATGAGAGTGCAACTTC | (SEQ ID NO: 067) | Basic/extension |
| | | | | GGGAAAGAAAATGTCTATGGCAAAGG | (SEQ ID NO: 068) | |
| AL034394_A 26270C | | | 32.37 | CCATAGACATTTTCTTTCCCTGATGATTTTTT | (SEQ ID NO: 069) | Basic/extension |
| | | | | CTTGAGACACAGATTCTGGAACCT | (SEQ ID NO: 070) | |
| AL034394_C 20283T | | | 32.38 | GTAGGAGCTTAAGACACTGTGTACTG | (SEQ ID NO: 071) | Basic/extension |
| | | | | GCCAAGGCATCTGAACTAAGAGA | (SEQ ID NO: 072) | |
| AL034394_C 16965T | | | 32,38 | GCAGCTCATCCCAACCTCATC | (SEQ ID NO: 073) | Basic/extension |
| | | | | GCAGCTCATCCCAACCTCATC | (SEQ ID NO: 074) | |
| | hCV2488476 | | 32,38 | | | Basic/extension |
| AL034394_C 12339T | | | 32,39 | GTTCAGAGTGCTGTTCATGAGTGAT | (SEQ ID NO: 075) | Basic/extension |
| | | | | CAGGATGGCAGACGTCAGA | (SEQ ID NO: 076) | |
| AL034394_T 10552C | | | 32,39 | ACTGTAAAGAAAGAGAATCCATTCTGATAATTAATCAATAT | (SEQ ID NO: 077) | Basic/extension |
| | | | | GCTGTTTTGTGTGCTCTAGAAAGTG | (SEQ ID NO: 078) | |
| BTNL2_E36 0Amb | | *BTNL2* | 32,39 | GCGAGGAGAAAATCGCAACTT | (SEQ ID NO: 079) | Basic/extension |
| | | | | TTTCCAAACCTGAAGGAACATAAGGAA | (SEQ ID NO: 080) | |
| BTNL2_P29 9Q | | *BTNL2* | 32,39 | GAGTCAGGGCCTGGGAAGA | (SEQ ID NO: 081) | Basic/extension/replication |
| | | | | GGAGGGGCAAGAAGATGGA | (SEQ ID NO: 082) | |
| BTNL2_M28 6I | | *BTNL2* | 32,39 | ACCCTTAGCAATGTCTGCACGT | (SEQ ID NO: 083) | Basic/extension/replication |
| | | | | GGTTCTTCCCCACTGATCACTG | (SEQ ID NO: 084) | |
| BTNL2_P285L | | *BTNL2* | 32,39 | GTTCTTCCCCACTGATCACTGT | (SEQ ID NO: 085) | Basic/extension/replication |
| | | | | TGAGTCAGGGCCTGGGA | (SEQ ID NO: 086) | |
| rs2076530 | | *BTNL2* | 32,39 | AGTACCGCTGCCTTTTTGAAAA | (SEQ ID NO: 087) | Basic/extension/replication |
| | | | | GTGGCAGGAGCAGGTATTGAATA | (SEQ ID NO: 088) | |
| BTNL2_S24 0L | | *BTNL2* | 32,39 | AGACTGACCCTGCAGATACTCA | (SEQ ID NO: 089) | Basic/extension |
| | | | | TTTTCAAAAAGGCAGCGGTACTG | (SEQ ID NO: 090) | |
| rs2076529 | | *BTNL2* | 32,39 | GGAGAGCAGATGGCAGAGTACAG | (SEQ ID NO: 091) | Basic/extension |
| | | | | CCTCGTCAATGGCGTCACT | (SEQ ID NO: 092) | |
| rs2294878 | hCV2488463 | *BTNL2* | 32,39 | | | Basic/extension |
| BTNL2_i4_A 239T | | *BTNL2* | 32,40 | TTGCAGCTGTGTTACCTGACA | (SEQ ID NO: 093) | Basic/extension |
| | | | | CAGAGATGATGCCATGCTTCCT | (SEQ ID NO: 094) | |
| rs2076524 | hCV2488453 | *BTNL2* | 32,40 | | | Basic/extension |
| BTNL2_i3_C 1900T | | *BTNL2* | 32,40 | TCTGAGCATCGCATCCAAGAT | (SEQ ID NO: 095) | Basic/extension |
| | | | | AGACTCTGCAGAGGCGTTCCT | (SEQ ID NO: 096) | |
| rs2076523 | | *BTNL2* | 32,40 | CCAGAGCCCCAGGTGTATTG | (SEQ ID NO: 097) | Basic-screen/extension |
| | | | | TGGACCAAGCAGGACACAGA | (SEQ ID NO: 098) | |
| BTNL2_i3_A 1837G | | *BTNL2* | 32,40 | CAGAGCCCCAGGTGTATTGG | (SEQ ID NO: 099) | Basic/extension |
| | | | | ACACGGCCAGCAGCTTCT | (SEQ ID NO: 100) | |
| BTNL2_W94R | | *BTNL2* | 32,40 | GACTGAGATGCAGATGGAGGAGTAC | (SEQ ID NO: 101) | Basic/extension |
| | | | | CTTTGCAATGCCATTCTCTATCC | (SEQ ID NO: 102) | |
| BTNL2_H60 H | | *BTNL2* | 32,40 | TGCCAGCTACTCCCCAAGAG | (SEQ ID NO: 103) | Basic/extension |
| | | | | GGGCTCTGAGCGGTACCA | (SEQ ID NO: 104) | |
| BTNL2_i1_G 1717T | | *BTNL2* | 32,40 | GGAGCCAAGCCCATTTCAC | (SEQ ID NO: 105) | Basic/extension |
| | | | | CAGTCTTAGCAGAGAATCCACATCA | (SEQ ID NO: 106) | |
| | hCV2455668 | | 32,43 | | | Basic/extension |
| 06DRAp_23 1 | | *HLA-DRA* | 32,43 | AATGTGCTTCAGGTATATCCCTGTCT | (SEQ ID NO: 107) | Basic/extension |
| | | | | AGTAAAGTTCTTAAACAAACAGGACAACAA | (SEQ ID NO: 108) | |
| 06DRAp_224 | | *HLA-DRA* | 32,43 | AATGTGCTTCAGGTATATCCCTGTCT | (SEQ ID NO: 109) | Basic/extension |
| | | | | AGTAAAGTTCTTAAACAAACAGGACAACAA | (SEQ ID NO: 110) | |
| 06DRAp_19 6 | | *HLA-DRA* | 32,43 | AATGTGCTTCAGGTATATCCCTGTCT | (SEQ ID NO: 111) | Basic/extension |
| | | | | CCGTTCATTGGATAAAGAAGTAAAGTT | (SEQ ID NO: 112) | |
| 06DRAp_12 7 | | *HLA-DRA* | 32,43 | TTATCCAATGAACGGAGTATCTTGTG | (SEQ ID NO: 113) | Basic/extension |
| | | | | ATGACGCATCTGTTGCTAGGG | (SEQ ID NO: 114) | |
| rs14004 | | *HLA-DRA* | 32,43 | CCTCACTCCCGAGCTCTACTGA | (SEQ ID NO: 115) | Basic/extension |
| | | | | CCACTTATGGCCATTTTCTTCTTG | (SEQ ID NO: 116) | |
| | hCV2455646 | *HLA-DRA* | 32,44 | | | Basic/extension |
| rs8084 | hCV2455637 | *HLA-DRA* | 32,44 | | | Basic/extension |
| rs7192 | | *HLA-DRA* | 32,44 | TGGGCCTGACTGTGGGTC | (SEQ ID NO: 117) | Basic-screen/extension |
| | | | | ACCACACCTAACTCACCTCCATG | (SEQ ID NO: 118) | |
| rs2213584 | | *HLA-DRA* | 32,44 | AATGGGTAAGGCCAGTCTTCAG | (SEQ ID NO: 119) | Basic/extension |
| | | | | TGCAGGATTTACATATCAACATCGT | (SEQ ID NO: 120) | |
| rs2395182 | | *HLA-DRA* | 32,44 | GGTGGTTTCAAGAATCAGTCAGACT | (SEQ ID NO: 121) | Basic/extension |
| | | | | GGCCTTACCCATTCTGTTAGACATA | (SEQ ID NO: 122) | |
| rs707952 | | *HLA-DQA1* | 32,62 | CATAGAGGTTCCTGAGGTCACA | (SEQ ID NO: 123) | Basic-screen/extension |
| | | | | TGGTCTCAGAAACACCTTCTGT | (SEQ ID NO: 124) | |
| X66401_G48 | | *TAP2* | 32,80 | GGTGGTTTCAGTTGGGCAG | (SEQ ID NO: 125) | Basic-screen |
| 629A | | | | CTCCATTTCCTGGATGAAGTCA | (SEQ ID NO: 126) | |
| rs1042337 | | *HLA-DMB* | 32,91 | GCCCAGCCCAATGGAGAC | (SEQ ID NO: 127) | Basic-screen |
| | | | | CCTAATTCCATCCATCTGCCATAC | (SEQ ID NO: 128) | |
| rs1063478 | | *HLA-DMA* | 32,92 | AAAAGGCCTGGAAGCTGAGTC | (SEQ ID NO: 129) | Basic-screen |
| | | | | GAGTTTGGCAAGCCCAACAC | (SEQ ID NO: 130) | |
| rs3918143 | | *RING3* | 32,95 | GATGCCAGATGAACCACTAGAACC | (SEQ ID NO: 131) | Basic-screen |
| | | | | CCTCAGAGGAGCTCTCACTGCTAC | (SEQ ID NO: 132) | |
| rs2581 | | *HLA-DNA* | 32,98 | TGTGCTGACTTTGAGTGGGATC | (SEQ ID NO: 133) | Basic-screen |
| | | | | AACACCACCAAAGCATTTAGTGC | (SEQ ID NO: 134) | |
| rs926424 | | *RXRB* | 33,16 | CTCCTTCTCTTCCACTGATGTGC | (SEQ ID NO: 135) | Basic-screen |
| | | | | GCACGTGAAGACCTGTGGC | (SEQ ID NO: 136) | |
| rs1547387 | | *HKE4* | 33,17 | AGTGCGGGAGGTGAGGAATAG | (SEQ ID NO: 137) | Basic-screen |
| | | | | CCCCAAATACACACTCATTGTGTC | (SEQ ID NO: 138) | |
| rs466384 | | *BING4* | 33,26 | CCTCAGGCTTAGGTGTGTATTAGCA | (SEQ ID NO: 139) | Basic-screen |
| | | | | TCGTGCCTGTAGAATCTACTGCC | (SEQ ID NO: 140) | |
| rs2071888 | | *TAPBP* | 33,27 | GCATTTGCTGCTTGGGATG | (SEQ ID NO: 141) | Basic-screen |
| | | | | AGCAAGCTCCAGGGTGACC | (SEQ ID NO: 142) | |
| rs999942 | | *ITPR3* | 33,63 | CCCTCCATCAGACTGGCACTC | (SEQ ID NO: 143) | Basic-screen |
| | | | | CGGTCACACAGCCACATGC | (SEQ ID NO: 144) | |
| rs943461 | | *MLN* | 33,77 | CCCCAAGACCCAGAGAGACC | (SEQ ID NO: 145) | Basic-screen |
| | | | | AGGGATACAGCTCCATGCTGAA | (SEQ ID NO: 146) | |
| rs755658 | | *FKBP5* | 35,55 | TTGAAGTCCTGTATCACAGGCATG | (SEQ ID NO: 147) | Basic-screen |
| | | | | ACCAACAGTGTTAAGACCATGTTACTAGG | (SEQ ID NO: 148) | |
| rs992105 | | *FKBP5* | 35,56 | TTCCATCATGACCAAGCTGTAGC | (SEQ ID NO: 149) | Basic-screen |
| | | | | CAGAGGGAGATAAGGCATGGC | (SEQ ID NO: 150) | |
| rs1320991 | | *FKBP5* | 35,56 | TGTTGGTATGTTGGAGAATTATGTGAA | (SEQ ID NO: 151) | Basic-screen |
| | | | | AAACAAGTAAATTCACTGCCTGCAC | (SEQ ID NO: 152) | |
| rs1326752 | | *SRPK1* | 35,83 | AAAAAAGCCTGTTGTCTTAGGTATGC | (SEQ ID NO: 153) | Basic-screen |
| | | | | AGAGGTTAATGCTAGGTTGCGC | (SEQ ID NO: 154) | |
| Z95152_G57 723A | | | 36,03 | CAGGTGATTGGTCTGTTGGACG | (SEQ ID NO: 155) | Basic-screen |
| | | | | CCCAAAAAGAGGTGCATGATAGG | (SEQ ID NO: 156) | |
| Z95152_T58 | | | 36,08 | GGAGAAGAGGGCTAATATATCCTAGAGTAG | (SEQ ID NO: 157) | Basic-screen |
| 784G | | | | GGCCGCTGTAATTCTCTTATCTG | (SEQ ID NO: 158) | |
| Z95152_T10 6861C | | | 36,08 | GAAGTCATCAGCTTTGTGCCAC | (SEQ ID NO: 159) | Basic-screen |
| | | | | ACCATGGAGGAAATCTCTGCA | (SEQ ID NO: 160) | |
| rs763096 | | *MAPK13* | 36,10 | CTGTGAGGACTGGCAGATGTG | (SEQ ID NO: 161) | Basic-screen |
| | | | | GCCTTTGCCTGTCAAGTGTG | (SEQ ID NO: 162) | |
| rs2007683 | | *MAPK13* | 36,10 | CAGCTTCTTCCATCTGCTCA | (SEQ ID NO: 163) | Basic-screen |
| | | | | ACAGTCGATATTGGGTAGCCA | (SEQ ID NO: 164) | |
| rs881648 | | *TEL2* | 36,35 | CTAGGCCTCCAACCCAGGAC | (SEQ ID NO: 165) | Basic-screen |
| | | | | CCCATGGCTTATTTACAGCTAGGA | (SEQ ID NO: 166) | |
| rs881647 | | *TEL2* | 36,35 | TTCAGAACTGGGCTGATATCACAG | (SEQ ID NO: 167) | Basic-screen |
| | | | | AATGAGGCCATGGTGGTGAC | (SEQ ID NO: 168) | |
| rs916287 | | *TEL2* | 36,35 | AGAAGAAGAGACGCAGGACCG | (SEQ ID NO: 169) | Basic-screen |
| | | | | CCATTTTCAGAACTGGGATGATA | (SEQ ID NO: 170) | |
| rs1885205 | | *TEL2* | 36,35 | CTCCTGACTCACTCTGGCCTTG | (SEQ ID NO:171) | Basic-screen |
| | | | | TCCACTCTGAGGACAACCGTG | (SEQ ID NO: 172) | |
| rs2055272 | | *FLJ2033 7* | 37,29 | CATGGTGGTAATGAATTGTGATTATG | (SEQ ID NO: 173) | Basic-screen |
| | | | | ATGCTTAAGATTTATGTCATGAGTCCA | (SEQ ID NO: 174) | |
| rs1004279 | | *GLP1R* | 39,03 | AGGAGGCTGAGGTCAAGGC | (SEQ ID NO: 175) | Basic-screen |
| | | | | GCCATCATGAAACTTATCACTACTGAC | (SEQ ID NO: 176) | |
| rs1541816 | | *KCNK5* | 39,16 | AGGCCTCTAGGAATTGGACCA | (SEQ ID NO: 177) | Basic-screen |
| | | | | GAGCAGGAGGCCAAGTCAGA | (SEQ ID NO: 178) | |
| rs1014737 | | *APOBE C2* | 41,02 | AACACAGAGGGAGGGTAGGTCA | (SEQ ID NO: 179) | Basic-screen |
| | | | | GTTGTGATTATAAAGCCCTGGACA | (SEQ ID NO: 180) | |
| rs2234246 | | *TREM1* | 41,25 | AAAAGGCAGGGAGTTAATAACATGA | (SEQ ID NO: 181) | Basic-screen |
| | | | | TAGAGGAACGAGGGCAGTGG | (SEQ ID NO: 182) | |
| rs1817538 | | *TREM1* | 41,25 | CTGCTTGCATGTCTGCTGATG | (SEQ ID NO: 183) | Basic-screen |
| | | | | CGAGAAGAATCCACCCGTAGTG | (SEQ ID NO: 184) | |
| rs1351835 | | *TREM1* | 41,25 | ATGTTCCTGCATAGCACTGGC | (SEQ ID NO: 185) | Basic-screen |
| | | | | AGGAGTGAGGGCACTTGGC | (SEQ ID NO: 186) | |
| rs2234237 | | *TREM1* | 41,25 | GAACCCATTCTCTTTCCCTGCT | (SEQ ID NO: 187) | Basic-screen |
| | | | | AGCGTGTAGTCACATTTCACATCC | (SEQ ID NO: 188) | |
| AL391903_C 88694T | | *TREM1* | 41,26 | ATTTACATTCAAGTATCATTCTCCCTTAGAT | (SEQ ID NO: 189) | Basic-screen |
| | | | | GACATCAACACAATAATACAGAGACCAA | (SEQ ID NO: 190) | |
| rs742735 | | *MDFI* | 41,62 | TCAATTGATGGCTGATGAGGAG | (SEQ ID NO: 191) | Basic-screen |
| | | | | AGCAGAATAAAATGGTGCCAGG | (SEQ ID NO: 192) | |
| rs1410492 | | *CCND3* | 41,91 | GGGCATTGTGGTGTGATGG | (SEQ ID NO: 193) | Basic-screen |
| | | | | GCAAAGTACTAGTGAGTGTAGACATGCA | (SEQ ID NO: 194) | |
| rs1160867 | | *TNRC5* | 42,90 | CCCTAACGGAGGCTCTGG | (SEQ ID NO: 195) | Basic-screen |
| | | | | TTCCCCACATCTTCCAGCAC | (SEQ ID NO: 196) | |
| rs9395 | | *SRF* | 43,15 | CCCAGTGAGTGAATCAGGCA | (SEQ ID NO: 197) | Basic-screen |
| | | | | ACCCCAGAGCCACCGAG | (SEQ ID NO: 198) | |
| rs1418488 | | *CAPN11* | 44,14 | GAAGGACAGTGTGAGAGCCACTG | (SEQ ID NO: 199) | Basic-screen |
| | | | | AAAGAGCCACAAATTGCTAACAACTA | (SEQ ID NO: 200) | |

**Table 4b: Probes for all Taqman SNP assays used. "Assay on Demand" assays (starting with hCV, https://store.appliedbiosystems.com).**

| Marker | hCV number | Gene | Position | Probes | | Cohort used |
|---|---|---|---|---|---|---|
| | | | (NCBI) MB | | | |
| rs2073528 | hCV11255445 | *BTN3A2* | 26,44 | | | Basic/extension |
| rs2072803 | hCV2474923 | *BTN2A2* | 26,46 | | | BasiGextension |
| | hCV2474907 | *BTN3A1* | 26,48 | | | Basic/extension |
| | hCV2474883 | | 26,50 | | | Basic/extension |
| | hCV9804528 | | 26,54 | | | Basic/extension |
| rs3736781 | hCV9804725 | *BTN1A1* | 26,57 | | | Basic/extension |
| rs126007 | | | 27,80 | FAM-AGAAGAGCAGCAACCAAAGGCTAACGTGA | (SEQ ID NO: 201) | Basic-screen |
| | | | | TET-AGAAGAGCAGCAAACAAAGGCTAACGTGAGA | (SEQ ID NO: 202) | |
| rs929042 | | *RFP* | 28,95 | FAM-CTGTAGCTGGCGAGGAAGGCAGGAGT | (SEQ ID NO: 203) | Basic-screen |
| | | | | TET-CTGTAGCTGGCGAGGAAGCCAGGAGT | (SEQ ID NO: 304) | |
| rs53161 | | | 29,56 | FAM-AAGGGGCAACATACACAATGTGTTTACCAAAATT | (SEQ ID NO: 205) | Basic-screen |
| | | | | TET-AAGGGGCAACATACACATTGTGTTTACCAAAATT | (SEQ ID NO: 206) | |
| rs404240 | | *UBD* | 29,59 | FAM-TCTTGGAGCCCAGCGAAAGAACCTG | (SEQ ID NO: 207) | Basic-screen |
| | | | | TET-ATCTTGGAGCCCAGCAAAAGAACCTGG | (SEQ ID NO: 208) | |
| rs362536 | | *UBD* | 29,59 | FAM-ATTGGGAGAATATTTCTGAATGGAGTATGAGGGAG | (SEQ ID NO: 209) | Basic-screen |
| | | | | TET-TATCTAGATTGGGAAAATATTTCTGAATGGAGTATGAGGG | (SEQ ID NO: 210) | |
| rs1805057 | | *GABBR1* | 29,64 | FAM-AGGAGGCGGCCGTTCTGGTGTG | (SEQ ID NO: 211) | Basic-screen |
| | | | | TET-TGGAGGAGGCAGCCGTTCTGGTGT | (SEQ ID NO: 212) | |
| rs3130253 | | *MOG* | 29,73 | FAM-CACTCTTGGCCTCGTCTTCCTCTGCC | (SEQ ID NO: 213) | Basic-screen |
| | | | | TET-TCACTCTTGGCCTCATCTTCCTCTGCCT | (SEQ ID NO: 214) | |
| rs1569315 | | *HLA-G* | 29,85 | FAM-AGGAGGCGTCCGTCGGACCC | (SEQ ID NO: 215) | Basic-screen |
| | | | | TET-AGGAGGCGACCGTCGGACCC | (SEQ ID NO: 216) | |
| rs8347 | | *HCG-V* | 30,10 | FAM-AAATGGAATAAGAAATAATCATATCCTTTCTTCCCACCC | (SEQ ID NO: 217) | Basic-screen |
| | | | | TET-AAATGGAACAAGAAATAATCATATCCTTTCTTCCCACC | (SEQ ID NO: 218) | |
| rs1264457 | | *HLA-E* | 30,52 | FAM-CCCGACAGGCGCTTCCTCCG | (SEQ ID NO: 219) | Basic-screen |
| | | | | TET-CCCGACGGGCGCTTCCTCC | (SEQ ID NO: 220) | |
| rs2074510 | | *GTF2H4* | 30,94 | FAM-CATTCTGGGCTCTTGCCTGTGAAATCTTTC | (SEQ ID NO: 221) | Basic-screen/extension |
| | | | | TET-CCATTCTGGGCTCTTTCCTGTGAAATCTTTC | (SEQ ID NO: 222) | |
| rs3095318 | | *CDSN* | 31,15 | FAM-CACGGGATGATGGCACTGCTGCT | (SEQ ID NO: 223) | Basic-screen |
| | | | | TET-CACGGGATGTTGGCACTGCTGCT | (SEQ ID NO: 224) | |
| rs2073721 | | *SC1* | 31,19 | FAM-ACCTGGGGAAGTGGGGACCACG | (SEQ ID NO: 225) | Basic-screen |
| | | | | TET-ACCTGGGGAAATGGGGACCACG | (SEQ ID NO: 226) | |
| rs1050393 | | *HLA-C* | 31,30 | FAM-CCAGGCACAGGCTGACCGAGTG | (SEQ ID NO: 227) | Basic-screen |
| | | | | TET-CGCCAGGCACAGACTGACCGAGT | (SEQ ID NO: 228) | |
| rs1051488 | | *HLA-B* | 31,38 | FAM-AGCTGTGGTCGCTGCTGTGATGTGA | (SEQ ID NO: 229) | Basic-screen |
| | | | | TET-AGCTGTGGTCGCTACTGTGATGTGAGGAG | (SEQ ID NO: 230) | |
| rs1063630 | | *MICA* | 31,44 | FAM-ATCCCCGGACAGCA | (SEQ ID NO: 231) | Basic/extension |
| | | | | VIC-CATCCCAGGACAGCA | (SEQ ID NO: 232) | |
| rs1063635 | | *MICA* | 31,44 | FAM-ACCAGGATTTGCCAAGGAGAGGAGCA | (SEQ ID NO: 233) | Basic-screen/extension |
| | | | | VIC-ACCAGGATTTGCCGAGGAGAGGAGC | (SEQ ID NO: 234) | |
| rs3828916 | hCV3273671 | | 31,53 | | | Basic/extension |
| rs3131639 | | *MICB* | 31,53 | FAM-CAGGACATTTTCTG | (SEQ ID NO: 235) | Basic/extension |
| | | | | TET-AGGACATCTTCTGCC | (SEQ ID NO: 236) | |
| rs3134900 | | *MICB* | 31,53 | FAM-TCTGTGAGATCCATGAAGACAGCAGCACC | (SEQ ID NO: 237) | Basic-screen/extension |
| | | | | TET-CTGTGAGATGCATGAAGACAGCAGCACC | (SEQ ID NO: 238) | |
| AC006046_ G30156A | | *MICB* | 31,53 | FAM-TGCAGATGACATTCA | (SEQ ID NO: 239) | Basic/extension |
| | | | | TET-TGCAGGTGACATTC | (SEQ ID NO: 240) | |
| rs3130062 | | *IkBL* | 31,59 | FAM-TCGACAGGATCCCTCTGCTTCTCG | (SEQ ID NO: 241) | Basic-screen/extension |
| | | | | TET-TCGACGGGATCCCTCTGCTTCTC | (SEQ ID NO: 242) | |
| rs1800683 | hCV7514865 | *LTA* | 31,60 | | | Basic/extension |
| rs1041981 | | *LTA* | 31,60 | FAM-TGCCCACAGCAACCTCAAACCTGC | (SEQ ID NO: 243) | Basic/extension |
| | | | | TET-TTGCCCACAGCACCCTCAAACCTG | (SEQ ID NO: 244) | |
| rs1799964 | | *TNFa* | 31,60 | FAM-AGCAAAGGAGAAGCTGAGAAGATGAAGGAAAAGTC | (SEQ ID NO: 245) | Basic-screen/extension |
| | | | | TET-AGCAAAGGAGAAGCTGAGAAGACGAAGGAAAAG | (SEQ ID NO: 246) | |
| rs1799742 | | *TNFa* | 31,60 | FAM-AGTATGGGGACCCCCCCTTAATGAAGA | (SEQ ID NO: 247) | Basic-screen |
| | | | | TET-TATGGGGACCCCCCCTTAACGAAG | (SEQ ID NO: 248) | |
| rs1800629 | | *TNFa* | 31,60 | FAM-AACCCCGTCCTCATGCCCCTC | (SEQ ID NO: 249) | Basic-screen |
| | | | | TET-AACCCCGTCCCCATGCCCCTC | (SEQ ID NO: 250) | |
| rs361525 | | *TNFa* | 31,60 | FAM-AATCAGAGCAGGGAGGATGGGGA | (SEQ ID NO: 251) | Basic-screen |
| | | | | TET-AATCGGAGCAGGGAGGATGGG | (SEQ ID NO: 252) | |
| rs707916 | | *DDAH2* | 31,76 | FAM-TTTATACTAGGTCGTGCTAGGGGCAGCGT | (SEQ ID NO: 253) | Basic-screen |
| | | | | TET-TTTATACTAGGTCGCGCTAGGGGCAGC | (SEQ ID NO: 254) | |
| rs707926 | | *VARS2* | 31,81 | FAM-CCATAGATGACGTCCAGGGGATCGAT | (SEQ ID NO: 255) | Basic-screen |
| | | | | TET-CCATAGATGACATCCAGGGGATCGATG | (SEQ ID NO: 256) | |
| rs2075800 | | *HSP70-HOM* | 31,84 | FAM-CATAAGAGAAAGGAATTGGAGCAGATGTGTAACCC | (SEQ ID NO: 257) | Basic-screen |
| | | | | TET-TCATAAGAGAAAGGAATTGAAGCAGATGTGTAACCC | (SEQ ID NO: 258) | |
| rs539689 | | *HSPA1B* | 31,86 | FAM-CCCGAAGCCGCCAGGCC | (SEQ ID NO: 259) | Basic-screen |
| | | | | TET-CCCCGAAGCCCCCAGGCC | (SEQ ID NO: 260) | |
| AF019413_C 89532G | | C2 | 31,96 | FAM-TCCCGGGATATGACTGAGGTGATCAGC | (SEQ ID NO: 261) | Basic-screen |
| | | | | TET-CCCGGGATATGACTGACGTGATCAGC | (SEQ ID NO: 262) | |
| rs1035798 | | *AGER* | 32,18 | FAM-TGGCCTCATTTCCACATACAGAGTTTGAGAAC | (SEQ ID NO: 263) | Basic-screen |
| | | | | TET-TGGCCTCATTTCCACACACAGAGTTTGA | (SEQ ID NO: 264) | |
| rs2070600 | | *AGER* | 32,18 | FAM-CCAACGGCTCCCT | (SEQ ID NO: 265) | Basic/extension |
| | | | | TET-CCAACAGCTCCCTC | (SEQ ID NO: 266) | |
| rs1800624 | hCV3293837 | *AGER* | 32,18 | | | Basic/extension |
| rs2071287 | hCV3293818 | NOTCH 4 | 32,20 | | | Basic/extension |
| rs384247 | hCV2412441 | *NOTCH* 4 | 32,21 | | | Basic/extension |
| rs915894 | hCV3293780 | *NOTCH* 4 | 32,22 | | | Basic/extension |
| rs436845 | hCV2412414 | | 32,22 | | | Basic/extension |
| rs560505 | hCV2495657 | *TSBP* | 32,29 | | | Basic/extension |
| rs485774 | hCV2495577 | *TSBP* | 32,32 | | | Basic/extension |
| rs552339 | hCV2495574 | *TSBP* | 32,32 | | | Basic/extension |
| rs761187 | hCV2234065 | *TSBP* | 32,34 | | | Basic/extension |
| rs2073044 | hCV2488525 | *TSBP* | 32,37 | | | Basic/extension |
| rs2050190 | hCV2488524 | *TSBP* | 32,37 | | | Basic/extension |
| rs2073045 | hCV2488523 | *TSBP* | 32,37 | | | Basic/extension |
| AL034394_T 26355G | | | 32,37 | FAM-TGAGCGTAGTCGATTC | (SEQ ID NO: 267) | Basic/extension |
| | | | | TET-TGAGCGTAGTAGATTC | (SEQ ID NO: 268) | |
| AL034394_A 26270C | | | 32,37 | FAM-CAAACACATTTTTCTTTC | (SEQ ID NO: 269) | Basic/extension |
| | | | | TET-CAAACACATGTTTCTTTC | (SEQ ID NO: 270) | |
| AL034394_C20283T | | | 32,38 | FAM-AGGGCTATTGCTTATT | (SEQ ID NO: 271) | Basic/extension |
| | | | | TET-CAGGGCTATTACTTATT | (SEQ ID NO: 272) | |
| AL034394_C 16965T | | | 32,38 | FAM-TTCTTCAACGGAGAATG | (SEQ ID NO: 273) | Basic/extension |
| | | | | TET-TTCTTCAACAGAGAATG | (SEQ ID NO: 274) | |
| | hCV2488476 | | 32,38 | | | Basic/extension |
| AL034394_C 12339T | | | 32,39 | FAM-CGCATGGGACCGC | (SEQ ID NO: 275) | Basic/extension |
| | | | | TET-CCGCATAGGACCGC | (SEQ ID NO: 276) | |
| AL034394_T 10552C | | | 32,39 | FAM-TTCCACTTTAAATTTCTTACGTC | (SEQ ID NO: 277) | Basic/extension |
| | | | | TET-CTTCCACTTTAAATTTCTTATGTC | (SEQ ID NO: 278) | |
| BTNL2_E36 0Amb | | *BTNL2* | 32,39 | FAM-TTTCTCTCTCATGTTGGTG | (SEQ ID NO: 279) | Basic/extension |
| | | | | TET-TTTCTCTCTCAGGTTGGTG | (SEQ ID NO: 280) | |
| BTNL2_P29 9Q | | *BTNL2* | 32,39 | FAM-TGTCCCTCCACTGCA | (SEQ ID NO: 281) | Basic/extension/replication |
| | | | | TET-ATGTCCCTCCATGGC | (SEQ ID NO: 282) | |
| BTNL2_M28 6I | | *BTNL2* | 32,39 | FAM-ATCCGAAGAGCATATC | (SEQ ID NO: 283) | Basic/extension/replication |
| | | | | FAM-CATCTGAAGAGCATATC | (SEQ ID NO: 284) | |
| | | | | VIC-CATCTGAAGAGCACATC | (SEQ ID NO: 285) | |
| | | | | VIC-ATCCGAAGAGCACATC | (SEQ ID NO: 286) | |
| BTNL2_P28 5L | | *BTNL2* | 32,39 | FAM-TGGAGAAATGCAGCTGA | (SEQ ID NO: 287) | Basic/extension/replication |
| | | | | TET-ATGGAGAAATGCAGCCGA | (SEQ ID NO: 288) | |
| rs2076530 | | *BTNL2* | 32,39 | FAM-AAGGTGGTAGGTAAGAA | (SEQ ID NO: 289) | Basic/extension/replication |
| | | | | TET-AGGTGGTAAGTAAGAAT | (SEQ ID NO: 290) | |
| BTNL2_S24 0L | | *BTNL2* | 32,39 | FAM-TCGTCCAAAGGTCT | (SEQ ID NO: 291) | Basic/extension |
| | | | | TET-TCGTCCGAAGGTCT | (SEQ ID NO: 292) | |
| rs2076529 | | *BTNL2* | 32,39 | FAM-ACCAGCACAGTCCT | (SEQ ID NO: 293) | Basic/extension |
| | | | | TET-ACCAGTACAGTCCTC | (SEQ ID NO: 294) | |
| rs2294878 | hCV2488463 | *BTNL2* | 32,39 | | | Basic/extension |
| BTNL2_i4_A 239T | | *BTNL2* | 32,40 | FAM-ATCAGATGGTCACCTAT | (SEQ ID NO: 295) | Basic/extension |
| | | | | TET-CATCAGATGGACACCTAT | (SEQ ID NO: 296) | |
| rs2076524 | hCV2488453 | *BTNL2* | 32,40 | | | Basic/extension |
| BTNL2_i3_C 1900T | | *BTNL2* | 32,40 | FAM-TGGCTTCCACATAGA | (SEQ ID NO: 297) | Basic/extension |
| | | | | TET-CTTCCGCATAGAAC | (SEQ ID NO: 298) | |
| rs2076523 | | *BTNL2* | 32,40 | FAM-CATCGCATCCAAGATAAAGATGGCCTGTT | (SEQ ID NO: 299) | Basic-screen/extension |
| | | | | TET-CATCGCATCCAAGATGAAGATGGCCT | (SEQ ID NO: 300) | |
| BTNL2_i3_A 1837G | | *BTNL2* | 32,40 | FAM-CTCCCCGGATGTC | (SEQ ID NO: 301) | Basic/extension |
| | | | | TET-CTCCCTGGATGTCT | (SEQ ID NO: 302) | |
| BTNL2_W94 R | | *BTNL2* | 32,40 | FAM-AGAGGCTGGGTAGAGT | (SEQ ID NO: 303) | Basic/extension |
| | | | | TET-AGAGGCAGGGTAGAGT | (SEQ ID NO: 304) | |
| BTNL2_H60 H | | *BTNL2* | 32,40 | FAM-AATGCATGTGGAGGTG | (SEQ ID NO: 305) | Basic/exterision |
| | | | | TET-AATGCACGTGGAGGT | (SEQ ID NO: 306) | |
| BTNL2_i1_G 1717T | | *BTNL2* | 32,40 | FAM-ACCCCTGAACAGTC | (SEQ ID NO: 307) | Basic/extension |
| | | | | TET-CCCCTGCACAGTC | (SEQ ID NO: 308) | |
| | hCV2455668 | | 32,43 | | | Basic/extension |
| 06DRAp_23 1 | | *HLA-DRA* | 32,43 | FAM-TGTCCGTGATTGAC | (SEQ ID NO: 309) | Basic/extension |
| | | | | TET-TGTCCGTCATTGAC | (SEQ ID NO: 310) | |
| 06DRAp_22 4 | | *HLA-DRA* | 32,43 | FAM-ATTTAAGACTGTTAGTCAAT | (SEQ ID NO: 311) | Basic/extension |
| | | | | TET-TTAAGACTGTTGGTCAAT | (SEQ ID NO: 312) | |
| 06DRAp_19 6 | | *HLA-DRA* | 32,43 | FAM-CAACAACAACAACAAA | (SEQ ID NO: 313) | Basic/extension |
| | | | | TET-ACAACGACAACAAAT | (SEQ ID NO: 314) | |
| 06DRAp_12 7 | | *HLA-DRA* | 32,43 | FAM-CCTGGACTCTTTG | (SEQ ID NO: 315) | Basic/extension |
| | | | | TET-CTGGACCCTTTGCA | (SEQ ID NO: 316) | |
| rs14004 | | *HLA-DRA* | 32,43 | FAM-CTCCCAACAGAGCG | (SEQ ID NO: 317) | Basic/extension |
| | | | | TET-TCCCAAAAGAGCGC | (SEQ ID NO: 318) | |
| | hCV2455646 | *HLA-DRA* | 32,44 | | | Basic/extension |
| rs8084 | hCV2455637 | *HLA-DRA* | 32,44 | | | Basic/extension |
| rs7192 | | *HLA-DRA* | 32,44 | FAM-TCATCATCAAGGGATTGCGCAAAAGC | (SEQ ID NO: 319) | Basic-screen/extension |
| | | | | TET-TCATCATCAAGGGAGTGCGCAAAAGC | (SEQ ID NO: 320) | |
| rs2213584 | | *HLA-DRA* | 32,44 | FAM-TGTCCAATCTCTTTGC | (SEQ ID NO: 321) | Basic/extension |
| | | | | TET-TGTCCAACCTCTTTG | (SEQ ID NO: 322) | |
| rs2395182 | | *HLA-DRA* | 32,44 | FAM-CGGTAATACAATAGGC | (SEQ ID NO: 323) | Basic/extension |
| | | | | TET-CTCGGTAATAAAATAGG | (SEQ ID NO: 324) | |
| rs707952 | | *HLA-DQA1* | 32,62 | FAM-CTGGGTCAGCCCAACACCCTC | (SEQ ID NO: 325) | Basic-screen/extension |
| | | | | TET-CTGGGTCAGCCCAACATCCTCA | (SEQ ID NO: 326) | |
| X66401_G48 629A | | *TAP2* | 32,80 | FAM-TGTGAGGAACAACATTACTTATGGGCTGCAG | (SEQ ID NO: 327) | Basic-screen |
| | | | | TET-TGAGGAACAACATTGCTTATGGGCTGC | (SEQ ID NO: 328) | |
| rs1042337 | | *HLA-DMB* | 32,91 | FAM-TGTGTGGTAGAGCACATTGGGGCTCC | (SEQ ID NO: 329) | Basic-screen |
| | | | | TET-TGTGTGGTAGAGCACACTGGGGCTCC | (SEQ ID NO: 330) | |
| rs1063478 | | *HLA-DMA* | 32,92 | FAM-CTTCCACAGGGATGGAATGATGCTG | (SEQ ID NO: 331) | Basic-screen |
| | | | | TET-CTTCCACAGGGACGGAATGATGCT | (SEQ ID NO: 332) | |
| rs3918143 | | *RING3* | 32,95 | FAM-CCTGGCTTGGCCAAATCGTCTTCAG | (SEQ ID NO: 333) | Basic-screen |
| | | | | TET-CCCTGGCTTGGTCAAATCGTCTTCAGA | (SEQ ID NO: 334) | |
| rs2581 | | *HLA-DNA* | 32,98 | FAM-CCTTCCCAT7CAACACACACACACATTC | (SEQ ID NO: 335) | Basic-screen |
| | | | | TET-CCTTCCCATTCAACACAAACACACATTGTT | (SEQ ID NO: 336) | |
| rs926424 | | *RXRB* | 33,16 | FAM-CATTGCCTCCTTTTCACACCGATCC | (SEQ ID NO: 337) | Basic-screen |
| | | | | TET-CATTGCCTCCTTCTCACACCGATCC | (SEQ ID NO: 338) | |
| rs1547387 | | *HKE4* | 33,17 | FAM-CCTCACCACTGTGGGAGTGTCCATGT | (SEQ ID NO: 339) | Basic-screen |
| | | | | TET-CTCACCACTGTGCGAGTGTCCATGTC | (SEQ ID NO: 340) | |
| rs466384 | | *BING4* | 33,26 | FAM-CTCTTCCAGGTACTGTGTCTTTATGGAGTCCAG | (SEQ ID NO: 341) | Basic-screen |
| | | | | TET-TCCAGGTACTGCGTCTTTATGGAGTCCA | (SEQ ID NO: 342) | |
| rs2071888 | | *TAPBP* | 33,27 | FAM-CTTCTGGCTGCCTAGAGTTCAACCCTTTCA | (SEQ ID NO: 343) | Basic-screen |
| | | | | TET-TCTGGCTGCCTACAGTTCAACCCTTTCA | (SEQ ID NO: 344) | |
| rs999942 | | *ITPR3* | 33,63 | FAM-CAGCAGGAGAGGCTTGCCATGG | (SEQ ID NO: 345) | Basic-screen |
| | | | | TET-CAGCAGGAGAGCCTTGCCATGG | (SEQ ID NO: 346) | |
| rs943461 | | *MLN* | 33,77 | FAM-CAAGCCCAGATGACGAGGGCTCTG | (SEQ ID NO: 347) | Basic-screen |
| | | | | TET-CAAGCCCAGATGACCAGGGCTCTG | (SEQ ID NO: 348) | |
| rs755658 | | *FKBP5* | 35,55 | FAM-ACTCGACAGTGAGATGTACGCGCTGC | (SEQ ID NO: 349) | Basic-screen |
| | | | | TET-TCGGCAGTGAGATGTACGCGCTG | (SEQ ID NO: 350) | |
| rs992105 | | *FKBP5* | 35,56 | FAM-ACTCTGGTAAGCACTGCTACAAAGTGCATCTTTAGAA | (SEQ ID NO: 351) | Basic-screen |
| | | | | TET-CGTACTCTGGTAAGCACTGCTACAAAGTGAATCTTTAGA | (SEQ ID NO: 352) | |
| rs1320991 | | *FKBP5* | 35,56 | FAM-TAGAAAACCCCTACGTGCTATATATGTGTGTGCGT | (SEQ ID NO: 353) | Basic-screen |
| | | | | TET-TAGAAAACCCCTACATGCTATATATGTGTGTGCGTATG | (SEQ ID NO: 354) | |
| rs1326752 | | *SRPK1* | 35,83 | FAM-CTGCAGCCAGGTCAGCCCGAA | (SEQ ID NO: 355) | Basic-screen |
| | | | | TET-CTGCAGCCACGTCAGCCCGA | (SEQ ID NO: 356) | |
| Z95152_G57 723A | | | 36,03 | FAM-TGAGTAAATTTTTTGCATTTGCCTTCCTGGTCTA | (SEQ ID NO: 357) | Basic-screen |
| | | | | TET-TGAGTAAATTTTTTGCGTTTGCCTTCCTGG | (SEQ ID NO: 358) | |
| Z95152 T58784G | | | 36,08 | FAM-TTCCTTCCTGTCTATGGTACTGATAGCTGTCGACTT | (SEQ ID NO: 359) | Basic-screen |
| | | | | TET-TCCTTCCTGTCTCTGGTACTGATAGCTGTCGAC | (SEQ ID NO: 360) | |
| Z95152_T10 6861C | | | 36,08 | FAM-AAGGCCTTTTCACGGGAACTCTCCAAA | (SEQ ID NO: 361) | Basic-screen |
| | | | | TET-AAGGCCTTTTCATGGGAACTCTCCAAATATTC | (SEQ ID NO: 362) | |
| rs763096 | | *MAPK13* | 36,10 | FAM-CCAGGCAAGCACTCCACAAAGTCCTT | (SEQ ID NO: 363) | Basic-screen |
| | | | | TET-CAGGCAAGCACCCCACAAAGTCCT | (SEQ ID NO: 364) | |
| rs2007683 | | *MAPK13* | 36,10 | FAM-ATGTTGATATCTGAATCAATGCTATCTTACCACATT | (SEQ ID NO: 365) | Basic-screen |
| | | | | TET-TGTTGATATCTGAATCAATACTATCTTACCACATTCTACA | (SEQ ID NO: 366) | |
| rs881648 | | *TEL2* | 36,35 | FAM-CTCACTTTGGATGCAGAGGAAATGAGACTTC | (SEQ ID NO: 367) | Basic-screen |
| | | | | TET-CTCACTTTGGATGCAAAGGAAATGAGACTTC | (SEQ ID NO: 368) | |
| rs881647 | | *TEL2* | 36,35 | FAM-CAAGGGACAACTTGGGAGAATTTTGAGAGG | (SEQ ID NO: 369) | Basic-screen |
| | | | | TET-AAGGGACAACTTGGGAGAATTCTGAGAGGA | (SEQ ID NO: 370) | |
| rs916287 | | *TEL2* | 36,35 | FAM-CTCTGAACTGCCTCCTCTCAAAATTCTCCCAA | (SEQ ID NO: 371) | Basic-screen |
| | | | | TET-CTCTGAACTGCCTCCTCTCAGAATTCTCCCA | (SEQ ID NO: 372) | |
| rs1885205 | | *TEL2* | 36,35 | FAM-ACCCTCATGGCCATGGACCGG | (SEQ ID NO: 373) | Basic-screen |
| | | | | TET-CATGGCCATGGACCAGGGATGG | (SEQ ID NO: 374) | |
| rs2055272 | | *FLJ2033* | 37,29 | FAM-AGGTAGGTTCGGGCGCTCTTACATTGTCT | (SEQ ID NO: 375) | Basic-screen |
| | | 7 | | TET-AGGTTCGAGCGCTCTTACATTGTCTCCAC | (SEQ ID NO: 376) | |
| rs1004279 | | *GLP1R* | 39.03 | FAM-TGAGTGGTCCGCCTGCCTTTCC | (SEQ ID NO: 377) | Basic-screen |
| | | | | TET-AGTGAGTGGTCCACCTGCCTTTCCTG | (SEQ ID NO: 378) | |
| rs1541816 | | *KCNK5* | 39,16 | FAM-CCCTCCCGGGTTAGGCTGGGTAC | (SEQ ID NO: 379) | Basic-screen |
| | | | | TET-CCTCCCGGGTCAGGCTGGG | (SEQ ID NO: 380) | |
| rs1014737 | | *APOBE* | 41,02 | FAM-AGCAATGGATCCTGAAGGGTTAAGTCCACT | (SEQ ID NO: 381) | Basic-screen |
| | | *C2* | | TET-AGCAATGGATCCTGAAGGGATAAGTCCACT | (SEQ ID NO: 382) | |
| rs2234246 | | *TREM1* | 41,25 | FAM-ATCTGTAATCACCGGCTATTTCTAAAGTCAGCGTCT | (SEQ ID NO: 383) | Basic-screen |
| | | | | TET-ATCTGTAATCACCAGCTATTTCTAAAGTCAGCGTCTCA | (SEQ ID NO: 384) | |
| rs1817538 | | *TREM1* | 41,25 | FAM-CCCAGAAACCTTAGAATCATTTTGCCAAAATCT | (SEQ ID NO: 385) | Basic-screen |
| | | | | TET-CCCAGAAACCTTAGAATCATTTCGCCAAAAT | (SEQ ID NO: 386) | |
| rs1351835 | | *TREM1* | 41,25 | FAM-CTCTCATCTGCTTGCTTGTCTGCTGATGAA | (SEQ ID NO: 387) | Basic-screen |
| | | | | TET-TCTCATCTGCTTGCATGTCTGCTGATGA | (SEQ ID NO: 388) | |
| rs2234237 | | *TREM1* | 41,25 | FAM-CTCCGAGCTGCAACTAAATTATCTGAGGAAAAGTATGA | (SEQ ID NO: 389) | Basic-screen |
| | | | | TET-CTCCGAGCTGCAACTAAATTAACTGAGGAAAAGTATGA | (SEQ ID NO: 390) | |
| AL391903_C | | *TREM1* | 41,26 | FAM-AGGTAATTGTCATTATTACCACAAAAAGGAAAACTGGAG | (SEQ ID NO: 391) | Basic-screen |
| 88694T | | | | TET-AGGTAATTGTCATTATTACCACAGAAAGGAAAACTGGA | (SEQ ID NO: 392) | |
| rs742735 | | *MDFI* | 41,62 | FAM-CACATCGGCTGGTTGGGTGTCTG | (SEQ ID NO: 393) | Basic-screen |
| | | | | TET-CACATCGGCTGGCTGGGTGTCT | (SEQ ID NO: 394) | |
| rs1410492 | | *CCND3* | 41,91 | FAM-AGGGGATGCCACTCCTTGCTGGTCT | (SEQ ID NO: 395) | Basic-screen |
| | | | | TET-AGGGGATGCCACTCCTTCCTGGTCTT | (SEQ ID NO: 396) | |
| rs1160867 | | *TNRC5* | 42,90 | FAM-ACCAAGACCCGAGGAGAAGAAGCCT | (SEQ ID NO: 397) | Basic-screen |
| | | | | TET-CAAGACCCGAGAAGAAGCCTGCG | (SEQ ID NO: 398) | |
| rs9395 | | *SRF* | 43,15 | FAM-ATGCGTGTGTCAGGGATGAGTTGAGGT | (SEQ ID NO: 399) | Basic-screen |
| | | | | TET-CGTGTGTCAGCGATGAGTTGAGGTGA | (SEQ ID NO: 400) | |
| rs1418488 | | *CAPN11* | 44,14 | FAM-CCCACCAAAGAAATCATGTAAGGTAATTTTTTAAGTCCC | (SEQ ID NO: 401) | Basic-screen |
| | | | | TET-CCCACCAAAGAAATCATGTAAGCTAATTTTTTAAGTCCC | (SEQ ID NO: 402) | |

**Table 5: Primers for mutation detection in the BTNL2 gene and extra-genic regions of disease association. For: forward primer; Rev: reverse primer.**

| Primer | Sequence | | Amplicon length | SNP marker* |
|---|---|---|---|---|
| 1_For | 5'-CCTCACACATGCCAATTCCC-3' | (SEQ ID NO: 403) | 463bp | 06orf_001 |
| 1_Rev | 5'-TTTTGCCTCCCCATCGAC-3' | (SEQ ID NO: 404) | | 06orf_002 |
| 2_For | 5'-CCTCACACATGCCAATTCCC-3' | (SEQ ID NO: 405) | 359bp. | 06orf_004 |
| 2_Rev | 5'-TTTTGCCTCCCCATCGAC-3' | (SEQ ID NO: 406) | | |
| 3_For | 5'-CAAACCACCACTCTCTCTTCATTG-3' | (SEQ ID NO: 407) | 384bp. | 06orf_005 |
| 3_Rev | 5'-CTCCCTCTTGTTTTTCTCCATTCT-3' | (SEQ ID NO: 408) | | |
| 4_For | 5'-TAAACATTTGTGGAAGAATAATGTGTAGTT-3' | (SEQ ID NO: 409) | 271bp. | 06orf_007 |
| 4_Rev | 5'-TCAAGAAGATAAACTATATGTGGAAGACAG-3' | (SEQ ID NO: 410) | | |
| 5_For | 5'-GAGCTGTGTTTGAAGCCAATGTC-3' | (SEQ ID NO: 411) | 540bp. | 06orf_009 |
| 5_Rev | 5'-GAGCCTCCCTTTTCACAAGTCA-3' | (SEQ ID NO: 412) | | |
| *BTNL2*_e8_For | 5'-CTTGTCTTCCTGTTTGGCTGTTAA-3' | (SEQ ID NO: 413) | 497bp. | |
| *BTNL2*_e8_Rev | 5'-GGGAAGGTGTAGATAGGGCACTT-3' | (SEQ ID NO: 414) | | |
| *BTNL2_*e7_For | 5'-AGCAGGCCAACTGCTTCCT-3' | (SEQ ID NO: 415) | 402bp. | |
| *BTNL2*_e7_Rev | 5'-ACTGAGCCTGGATTGCATGAT-3' | (SEQ ID NO: 416) | | |
| *BTNL2*_e6_For | 5'-CCTGGCCGAAGTTATTTTGT-3' | (SEQ ID NO: 417) | 738bp. | *BTNL2*_e6_E360Amb |
| *BTNL2*_e6_Rev | 5'-TAGAATCCCTGGGTGTCCTG-3' | (SEQ ID NO: 418) | | *BTNL2*_e6_P299Q |
| | | | | *BTNL2*_e6_M2861 |
| *BTNL2_*e5_For | 5'-GGTTTCTAAACTCCAATGGAGCTGTT-3' | (SEQ ID NO: 419) | 528bp. | *BTNL2_*e5_S240L |
| *BTNL2*_e5_Rev | 5'-CAAATGTCAGAGAAATTGTCCAGGA-3' | (SEQ ID NO: 420) | | |
| *BTNL2_*i4_For | 5'-GGTAAGTACGAGGTGCTGGCA-3' | (SEQ ID NO: 421) | 541 bp. | *BTNL2*_i4_A239T |
| *BTNL2*_i4_Rev | 5'-TTGCTACACTCACAGTATCCCAGG-3' | (SEQ ID NO: 422) | | |
| *BTNL2_*e4_For | 5'-GTGACAGCATTTTTGTTGTGCAG-3' | (SEQ ID NO: 423) | 474bp. | |
| *BTNL2*_e4_Rev | 5'-GGTGTCCATCTGATGCTCACTG-3' | (SEQ ID NO: 424) | | |
| *BTNL2*_i3_For | 5'-CCTCAGACTCCTGAACTCCG-3' | (SEQ ID NO: 425) | 469bp. | *BTNL2*_i3_C1900T |
| *BTNL2_*i3_Rev | 5'-GGTGCTATGGTGCAGTCCC-3' | (SEQ ID NO: 426) | | *BTNL2_*i3_A1837G |
| *BTNL2*_e3_For | 5'-CATTTTGACAGGCTGGACACC-3' | (SEQ ID NO: 427) | 511bp. | *BTNL2*_e3_W94R |
| *BTNL2*_e3_Rev | 5'-CCTCAACTGTCACAAAGCTTACCA-3' | (SEQ ID NO: 428) | | *BTNL2*_e3_H60H |
| *BTNL2*_e2_For | 5'-CAATGGAAGCCATGGAGTGTG-3' | (SEQ ID NO: 429) | 489bp. | |
| *BTNL2*_e2_Rev | 5'-AACGTGGTGCAACCACTGATT-3' | (SEQ ID NO: 430) | | |
| *BTNL2*_i1_For | 5'-AAGCCGAACTACATCAACGTTTG-3' | (SEQ ID NO: 431) | 528bp. | *BTNL2*_i1_G1717T |
| *BTNL2*_i1_Rev | 5'-CTGCACCAGACAGATTGTAGCC-3' | (SEQ ID NO: 432) | | |
| *BTNL2*_e1_For | 5'-TGTCCTCTAAGGGCAAAACAGC-3' | (SEQ ID NO: 433) | 693bp. | |
| *BTNL2*_e1_Rev | 5'-GTTTCCCCCTTTCCAAGAAACTA-3' | (SEQ ID NO: 434) | | |
| DRAp_For | 5'-TAGGTGTTTCCATTGATTCTATTCTCAC-3' | (SEQ ID NO: 435) | 462bp. | 06DRAp_231 |
| DRAp_Rev | 5'-TGATAGCCCATGATTCCTGAGC-3' | (SEQ ID NO: 436) | | 06DRAp_224 |
| | | | | 06DRAp_196 |
| | | | | 060RAp_127 |

**Table 6: Primers for rt-PCR and cDNA cloning. The position of the primer sequences in the revised BNTL2 transcript refers to the 5' end for forward primers and to the 3' end for reverse primers.**

| Designation | Sequence | | Position in cDNA |
|---|---|---|---|
| BTNL2f1 | ACCCTTCAGTCTAAGGGGAG | (SEQ ID NO: 437) | 1 |
| BTNL2f2 | TGGTGGATTTTCCAGGCTAC | (SEQ ID NO: 438) | 45 |
| BTNL2f3 | TTAGAGTCATTGGCCCTGCT | (SEQ ID NO: 439) | 126 |
| BTNL2r1 | CTTCAGTGCCACATTTCCCT | (SEQ ID NO: 440) | 370 |
| BTNL2r2 | CTTCTCCCACTCTGACGAGG | (SEQ ID NO: 441) | 542 |
| BTNL2f4 | CCTGCAGATACTCAGTGCCA | (SEQ ID NO: 442) | 733 |
| BTNL2r3 | CGAAGGTCTGGCACTGAGTA | (SEQ ID NO: 443) | 760 |
| BTNL2f5 | AGTACCGCTGCCTTTTTGAA | (SEQ ID NO: 444) | 771 |
| BTNL2r4 | TTCAAAAAGGCAGCGGTACT | (SEQ ID NO: 445) | 790 |
| BTNL2f6 | TCGTCTTTCCTTCCATGTCC | (SEQ ID NO: 446) | 958 |
| BTNL2f7 | CGGAGATGTTTGTGACCCTT | (SEQ ID NO: 447) | 1042 |
| BTNL2f8 | ACTTGTTCCATCAGCATCCC | (SEQ ID NO: 448) | 1073 |
| BTNL2r5 | TGGATCACACGCTTGTTCTG | (SEQ ID NO: 449) | 1282 |
| BTNL2r6 | GCCCATAGAGCAGATAGTCAG | (SEQ ID NO: 450) | 1337 |

### REFERENCES

Abdallah, A. et al. Inhibitor kappa B-alpha (IkappaB-alpha) promoter polymorphisms in UK and Dutch sarcoidosis. *Genes and Immunity* **4**, 450-454 (2003).
Agadjanyan, M.G. et al. Costimulatory molecule immune enhancement in a plasmid vaccine model is regulated in part through the Ig constant-like domain of CD80/86. *J Immunol* **171**, 4311-9 (2003).
Albrecht, M., Domingues, F.S., Schreiber, S. & Lengauer, T. Identification of mammalian orthologs associates PYPAF5 with distinct functional roles. *FEBS Lett* **538,** 173-7 (2003).
Bajorath, J. Structural biology of T-cell costimulatory proteins: New insights, more surprises. *J Mol Graph Model* **19**, 619-623 (2001).
Borriello, F. et al. B7-1 and B7-2 have overlapping, critical roles in immunoglobulin class switching and germinal center formation. *Immunity. 1997 Mar; 6(3): 303-13.* **6**, 303-13 (1997).
Clayton, D. & Jones, H. Transmission/disequilibrium tests for extended marker haplotypes. *Am J Hum Genet* **65,** 1161-9. (1999).
Clayton, D. A generalization of the transmission/disequilibrium test for uncertain- haplotype transmission. Am J Hum Genet 65, 1170-7. (1999).
Collins, A.V. et al. The interaction properties of costimulatory molecules revisited. *Immunity* **17**, 201-10. (2002).
Costabel, U. & Hunninghake, G.W. ATS/ERS/WASOG statement on sarcoidosis, sarcoidosis Statement Committee. American Thoracic Society. European Respiratory Society. World Association for sarcoidosis and Other Granulomatous Disorders. *Eur Respir J* **14**, 735-7. (1999).
Foley, P.J. et al. Human leukocyte antigen-DRB1 position 11 residues are a common protective marker for sarcoidosis. *Am J Respir Cell Mol Biol* **25,** 272-7. (2001).
Grutters, J.C. et al. Increased frequency of the uncommon tumor necrosis factor -857T allele in British and Dutch patients with sarcoidosis. *Am J Respir Crit Care Med* **165**, 1119-24. (2002).
Hampe, J. et al. A non-electrophoretic method for high-throughput HLA-DRB1 group genotyping. *Biotechniques,* in press (2004).
Hampe, J. et al. An integrated system for high throughput TaqMan based SNP genotyping. *Bioinformatics* **17**, 654-5. (2001).
Hampe, J. et al. Evidence for a NOD2-independent susceptibility locus for inflammatory bowel disease on chromosome 16p. *Proc Natl Acad Sci USA* **99**, 321-6. (2002).
Hunninghake, G.W., Gadek, J.E., Kawanami, O., Ferrans, V.J. & Crystal, R.G. Inflammatory and immune processes in the human lung in health and disease: evaluation by bronchoalveolar lavage. *Am J Pathol* **97,** 149-206. (1979).
Ikemizu, S. et al. Structure and dimerization of a soluble form of B7-1. *Immunity* **12**, 51-60 (2000).
Krawczak, M. et al. Allelic association of the cystic fibrosis locus and two DNA markers, XV2c and KM19, in 55 German families. *Hum Genet* **80**, 78-80. (1988).
Krawczak, M., Reiss, J. & Cooper, D.N. The mutational spectrum of single base-pair substitutions in mRNA splice junctions of human genes: causes and consequences. *Hum Genet* **90**, 41-54. (1992).
Kruglyak, L., Daly, M.J., Reeve Daly, M.P. & Lander, E.S. Parametric and nonparametric linkage analysis: a unified multipoint approach. *Am J Hum Genet* **58**, 1347-63 (1996).
Long, M. & Deutsch, M. Association of intron phases with conservation at splice site sequences and evolution of spliceosomal introns. *Mol Biol Evol* **16,** 1528-34. (1999).
Newman, L.S., Rose, C.S. & Maier, L.A. sarcoidosis. *N Engl J Med* **336,** 1224-34. (1997).
Rhodes, D.A., Stammers, M., Malcherek, G., Beck, S. & Trowsdale, J. The cluster of BTN genes in the extended major histocompatibility complex. *Genomics* **71**, 351-62 (2001).
Rossman, M.D. et al. HLA-DRB 1*11 01: A Significant Risk Factor for sarcoidosis in Blacks and Whites. *Am J Hum Genet* **73**, in press (2003).
Rybicki, B.A. et al. Familial aggregation of sarcoidosis. A case-control etiologic study of sarcoidosis (ACCESS). *Am J Respir Crit Care Med* **164**, 2085-91. (2001).
Rybicki, B.A. et al. The major histocompatibility complex gene region and sarcoidosis susceptibility in african americans. *Am J Respir Crit Care Med* **167**, 444-9. (2003).
Sato, H. et al. HLA-DQB1*0201: a marker for good prognosis in British and Dutch patients with sarcoidosis. *Am J Respir Cell Mol Biol* **27**, 406-12. (2002).
Schurmann, M. et al. Results from a genome-wide search for predisposing genes in sarcoidosis. *Am J Respir Crit Care Med* **164,** 840-6 (2001).
Schwartz, J.C., Zhang, X., Fedorov, A.A., Nathenson, S.G. & Almo, S.C. Structural basis for co-stimulation by the human CTLA-4B7-2 complex. *Nature* **410,** 604-8 (2001).
Sharpe, A.H. & Freeman, G.J. The B7-CD28 superfamily. *Nat Rev Immunol* **2,** 116-26 (2002).
Stammers, M., Rowen, L., Rhodes, D., Trowsdale, J. & Beck, S. BTL-II: a polymorphic locus with homology to the butyrophilin gene family, located at the border of the major histocompatibility complex class II and class III regions in human and mouse. *Immunogenetics* **51,** 373-82 (2000).
Stamper, C.C. et al. Crystal structure of the B7-1/CTLA-4 complex that inhibits human immune responses. *Nature* **410,** 608-11 (2001).
Statement on sarcoidosis. Joint Statement of the American Thoracic Society (ATS), the European Respiratory Society (ERS) and the World Association of sarcoidosis and Other Granulomatous Disorders (WASOG) adopted by the ATS Board of Directors and by the ERS Executive Committee. *Am J Respir Crit Care Med* **160,** 736-55 (1999).
Ueda, H. ct al. Association of the T-cell regulatory gene CTLA4 with susceptibility to autoimmune disease. *Nature* **423,** 506-11. (2003).
Walunas, T.C., Bakker, C.Y. & Bluestone, J.A. CTLA-4 ligation blocks CD28-dependent T cell activation. *J Exp Med* **183** (1996).
Zhang, X., Schwartz, J.C., Almo, S.C. & Nathenson, S.G. Crystal structure of the receptor-binding domain of human B7-2: insights into organization and signaling. *Proc Natl Acad Sci U S A* **100,** 2586-91 (2003).
Ziegenhagen, M. & Müller-Quemheim, J. The cytokine network in sarcoidosis and its clinical relevance. *J Internal Med* **253**, 18-30 (2003).
Zissel, G. et al. Human alveolar epithelial cells type II are capable of regulating T-cell activity. *J Investig Med* **48**, 66-75 (2000).

## Claims

1. A method of identifying individuals having a nucleic acid molecule not associated with sarcoidosis, comprising:
(a) amplifying a DNA fragment comprising an individual's nucleotide sequence selected from the group of:
(i) nucleic acid molecules comprising the nucleotide sequence as depicted in SEQ ID NO: 455,
(ii) nucleic acid molecules encoding a polypeptide comprising the amino acid sequence as depicted in SEQ ID NO: 456,
(iii) nucleic acid molecules the complementary strand of which hybridizes to a nucleic acid molecule of (i) or (ii) and which encode a polypeptide of the butyrophilin-like portein-type and
(iv) nucleic acid molecules the sequence of which differs from the sequence of a nucleic acid molecule of (iii) due to the degeneracy of the genetic code,
using an oligonucleotide primer which specifically hybridizes to sequences within the individual's nucleotide sequence;
(b) sequencing said amplified DNA fragment with dideoxy sequencing;
(c) repeating steps (a) and (b) until the individual's nucleotide sequence is completely sequenced;
(d) comparing the individual's nucleotide sequence with the nucleotide sequence as depicted in SEQ ID NO: 455;
(e) determining the absence of a polymorphic variation in the individual's nucleotide sequence at position 16071 of the nucleotide sequence as depicted in SEQ ID NO: 455.

2. A method of detecting an increased susceptibility to sarcoidosis resulting from the presence of a polymorphism at position 16071 in exon 5 of the gene as depicted in SEQ ID NO: 455, comprising:
(a) amplifying a DNA fragment comprising an individual's nucleotide sequence selected from the group of:
(i) nucleic acid molecules comprising the nucleotide sequence as depicted in SEQ ID NO: 455,
(ii) nucleic acid molecules encoding a polypeptide comprising the amino acid sequence as depicted in SEQ ID NO: 456,
(iii) nucleic acid molecules the complementary strand of which hybridizes to a nucleic acid molecule of (i) or (ii) and which encode a polypeptide of the butyrophilin-like portein-type and
(iv) nucleic acid molecules the sequence of which differs from the sequence of a nucleic acid molecule of (iii) due to the degeneracy of the genetic code,
using an oligonucleotide primer which specifically hybridizes to sequences within the individual's nucleotide sequence;
(b) sequencing said amplified DNA fragment with dideoxy sequencing;
(c) repeating steps (a) and (b) until the individual's nucleotide sequence is completely sequenced;
(d) comparing the individual's nucleotide sequence with the nucleotide sequence as depicted in SEQ ID NO: 455;
(e) determining the presence of a polymorphic variation in the individual's nucleotide sequence at position 16071 of the nucleotide sequence as depicted in SEQ ID NO: 455, wherein said polymorphic variation is a single nucleotide polymorphism, in which A is substituted by G.

3. A method according to claim 1 or 2, wherein said oligonucleotide primer is labelled with a radiolabel, a fluorescent label, a bioluminescent label, a chemiluminescent label, or an enzyme label.

4. Use of a vector comprising a nucleotide sequence of SEQ ID NO: 455 for preparing a pharmaceutical composition for the treatment of sarcoidosis.

5. Use of a protein selected from the group of proteins as depicted in SEQ ID NO: 456, SEQ ID NO: 457 and SEQ ID NO: 458 for preparing a pharmaceutical composition for the treatment of sarcoidosis.

6. Use of a peptide sharing at least a common sequence with a protein, wherein said protein is capable of binding to T-cells, selected from the group of proteins as depicted in SEQ ID NO: 456, SEQ ID NO: 457 and SEQ ID NO: 458 for preparing a pharmaceutical composition for the treatment of sarcoidosis.

7. Use of a molecule resembling the binding motif of a protein, wherein said binding motif mediates the binding of said protein to T-cells, selected from the group of proteins as depicted in SEQ ID NO: 456, SEQ ID NO: 457 and SEQ ID NO: 458 for preparing a pharmaceutical composition for the treatment of sarcoidosis.

## Patentansprüche

1. Verfahren zum Identifizieren von Individuen mit einem Nukleinsäuremolekül, das nicht mit Sarkoidose im Zusammenhang steht, mit den Schritten:
(a) Amplifizieren eines DNA-Fragments mit einer Nukleotidsequenz eines Individuums ausgewählt aus der Gruppe von:
(i) Nukleinsäuremolekülen mit der in der SEQ ID NO: 455 dargestellten Nukleotidsequenz,
(ii) Nukleinsäuremolekülen, die für ein Polypepeptid mit der in der SEQ ID NO: 456 dargestellten Aminosäuresequenz kodieren,
(iii) Nukleinsäuremolekülen, deren Komplementärstrang mit einem Nukleinsäuremolekül von (i) oder (ii) hybridisiert und die für ein Polypeptid des Butyrophilin-ähnlichen Proteintyps kodieren und
(iv) Nukleinsäuremolekülen, deren Sequenz von der Sequenz eines Nukleinsäuremoleküls von (iii) aufgrund des degenerierten genetischen Codes abweicht,
unter Verwendung eines Oligonukleotid Primers, der spezifisch an Sequenzen innerhalb der Nukleotidsequenz des Individuums hybridisiert;
(b) Sequenzieren des amplifizierten DNA-Fragments mit dem Dideoxy-Verfahren;
(c) Wiederholen der Schritte (a) und (b) bis die Nukleotidsequenz des Individuums vollständig sequenziert ist;
(d) Vergleichen der Nukleotidsequenz des Individuums mit der in der SEQ ID NO: 455 dargestellten Nukleotid-Sequenz;
(e) Bestimmen der Abwesenheit einer polymorphen Abweichung in der Nukleotidsequenz des Individuums an der Position 16071 von der in der SEQ ID NO: 455 dargestellten Nukleotidsequenz.

2. Ein Verfahren zum Aufdecken einer erhöhten Anfälligkeit gegenüber Sarkoidose, die sich aus der Anwesenheit eines Polymorphismus an der Position 16071 im Exon 5 des in der SEQ ID NO: 455 dargestellten Gens ergibt, mit den Schritten
(a) Amplifizieren eines DNA-Fragments mit einer Nukleotidsequenz eines Individuums ausgewählt aus der Gruppe von:
(i) Nukleinsäuremolekülen mit der in der SEQ ID NO: 455 dargestellten Nukleotidsequenz,
(ii) Nukleinsäuremolekülen, die für ein Polypepeptid mit der in der SEQ ID NO: 456 dargestellten Aminosäuresequenz kodieren,
(iii) Nukleinsäuremolekülen, deren Komplementärstrang mit einem Nukleinsäuremolekül von (i) oder (ii) hybridisiert und die für ein Polypeptid des Butyrophilin-ähnlichen Proteintyps kodieren und
(iv) Nukleinsäuremolekülen, deren Sequenz von der Sequenz eines Nukleinsäuremoleküls von (iii) aufgrund des degenerierten genetischen Codes abweicht,
unter Verwendung eines Oligonukleotid Primers, der spezifisch an Sequenzen innerhalb der Nukleotidsequenz des Individuums hybridisiert;
(b) Sequenzieren des amplifizierten DNA-Fragments mit dem Dideoxy-Verfahren;
(c) Wiederholen der Schritte (a) und (b) bis die Nukleotidsequenz des Individuums vollständig sequenziert ist;
(d) Vergleichen der Nukleotidsequenz des Individuums mit der in der SEQ ID NO: 455 dargestellten Nukleotid-Sequenz;
(e) Bestimmen einer polymorphen Abweichung in der Nukleotidsequenz des Individuums an Position 16071 der in der SEQ ID NO: 455 dargestellten Nukleotidsequenz, wobei die polymorphe Abweichung ein Einzelnukleotidpolymorphismus ist, bei dem A durch G substituiert ist.

3. Verfahren nach einem der Ansprüche 1 oder 2, **dadurch gekennzeichnet, dass** der Oligonukleotidprimer mit einem radioaktiven Marker, einem fluoreszierenden Marker, einem biolumineszenten Marken, einem chemilumineszenten Marker oder einem enzymatischen Marker markiert ist.

4. Verwendung eines Vektors mit der SEQ ID No. 455 dargestellten Nukleotidsequenz zur Zubereitung einer pharmazeutischen Zusammensetzung zur Behandlung von Sarkoidose.

5. Verwendung eines Proteins, ausgewählt aus der in der SEQ ID No: 456, SEQ ID NO: 457 und SEQ ID NO: 458 dargestellten Gruppe von Proteinen zur Zubereitung einer pharmazeutischen Zusammensetzung zur Behandlung von Sarkoidose.

6. Verwendung eines Peptids, das wenigstens eine gemeinsame Sequenz mit einem Protein, das an T-Zellen zu binden fähig ist, gemeinsam hat, ausgewählt aus der in der SEQ ID NO: 456, SEQ ID NO: 457 und SEQ ID NO: 458 dargestellten Gruppe von Proteinen zur Zubereitung einer pharmazeutischen Zusammensetzung zur Behandlung von Sarkoidose.

7. Verwendung eines Moleküls, das dem Bindungsmotiv eines Proteins ähnlich sieht, wobei das Bindungsmotiv die Bindung des Proteins an T-Zellen vermittelt, ausgewählt aus der in der SEQ ID NO: 456, SEQ ID NO: 457 und SEQ ID NO: 458 dargestellten Gruppe von Proteinen zur Zubereitung einer pharmazeutischen Zusammensetzung zur Behandlung von Sarkoidose.

## Revendications

1. Procédé d'identification d'individus avec une molécule d'acide nucléique qui n'est pas en rapport avec la sarcoïdose, comportant les étapes suivantes :
(a) amplification d'un fragment d'ADN avec une séquence de nucléotide d'un individu sélectionnée dans le groupe composé :
(i) de molécules d'acide nucléique ayant la séquence de nucléotides représentée dans l'ID SEQ. n° 455,
(ii) de molécules d'acide nucléique qui codent pour un polypeptide ayant la séquence d'acides aminés représentée dans l'ID SEQ. n° 456,
(iii) de molécules d'acide nucléique dont le brin complémentaire est hybridé avec une molécule d'acide nucléique de (i) ou (ii) et qui codent pour un polypeptide du type de protéine similaire à la butyrophiline, et
(iv) de molécules d'acide nucléique dont la séquence diffère de la séquence d'une molécule d'acide nucléique de (iii) du fait du code génétique dégénéré,
en utilisant une amorce d'oligonucléotide qui hybride de façon spécifique aux séquences au sein de la séquence nucléotidique de l'individu ;
(b) séquençage du fragment d'ADN amplifié avec un procédé didésoxy ;
(c) répétition des étapes (a) et (b) jusqu'à ce que la séquence nucléotidique de l'individu soit entièrement séquencée ;
(d) comparaison de la séquence nucléotidique de l'individu avec la séquence nucléotidique représentée dans l'ID SEQ. n° 455 ;
(e) détermination de l'absence d'une différenciation polymorphe dans la séquence nucléotidique de l'individu en position 16071 par rapport à la séquence nucléotidique représentée dans l'ID SEQ. n° 455.

2. Procédé de découverte d'une prédisposition accrûe vis-à-vis de la sarcoïdose qui découle de la présence d'un polymorphisme en position 16071 de l'exon 5 du gène représenté dans l'ID SEQ. n° 455, comportant les étapes suivantes :
(a) amplification d'un fragment d'ADN avec une séquence nucléotidique d'un individu sélectionnée dans le groupe composé :
(i) de molécules d'acide nucléique ayant la séquence de nucléotides représentée dans l'ID SEQ. n° 455,
(ii) de molécules d'acide nucléique qui codent pour un polypeptide ayant la séquence d'acides aminés représentée dans l'ID SEQ. n° 456,
(iii) de molécules d'acide nucléique dont le brin complémentaire est hybridé avec une molécule d'acide nucléique de (i) ou (ii) et qui codent pour un polypeptide du type de protéine similaire à la butyrophiline, et
(iv) de molécules d'acide nucléique dont la séquence diffère de la séquence d'une molécule d'acide nucléique de (iii) du fait du code génétique dégénéré,
en utilisant une amorce d'oligonucléotide qui hybride de façon spécifique aux séquences au sein de la séquence nucléotidique de l'individu ;
(b) séquençage du fragment d'ADN amplifié avec le procédé didésoxy ;
(c) répétition des étapes (a) et (b) jusqu'à ce que la séquence nucléotidique de l'individu soit entièrement séquencée ;
(d) comparaison de la séquence nucléotidique de l'individu avec la séquence nucléotidique représentée dans l'ID SEQ. n° 455 ;
(e) détermination d'une différenciation polymorphe dans la séquence nucléotidique de l'individu en position 16071 de la séquence nucléotidique représentée dans l'ID SEQ. n° 455, la différenciation polymorphe étant un polymorphisme d'un seul nucléotide dans lequel A est substitué par G.

3. Procédé selon l'une des revendications 1 ou 2, **caractérisé en ce que** l'amorce d'oligonucléotide est marquée avec un marqueur radioactif, un marqueur fluorescent, un marqueur bioluminescent, un marqueur chimioluminescent ou un marqueur enzymatique.

4. Utilisation d'un vecteur comportant la séquence nucléotidique représentée dans l'ID SEQ. n° 455 pour préparer une composition pharmaceutique destinée au traitement de la sarcoïdose.

5. Utilisation d'une protéine sélectionnée dans le groupe des protéines représenté par l'ID SEQ. n° 456, ID SEQ. n° 457 et ID SEQ. n° 458 pour préparer une composition pharmaceutique destinée au traitement de la sarcoïdose.

6. Utilisation d'un peptide qui a en commun au moins une séquence commune avec une protéine qui est apte à se lier aux cellules T, sélectionnée parmi le groupe des protéines représenté par l'ID SEQ. n° 456, ID SEQ. n° 457 et ID SEQ. n° 458, pour préparer une composition pharmaceutique destinée au traitement de la sarcoïdose.

7. Utilisation d'une molécule qui est similaire au motif de liaison d'une protéine, dans laquelle le motif de liaison confère la liaison de la protéine aux cellules T, sélectionnée parmi le groupe des protéines représentées par l'ID SEQ. n° 456, ID SEQ. n° 457 et ID SEQ. n° 458 pour préparer une composition pharmaceutique destinée au traitement de la sarcoïdose.
